# EUROPÄISCHE PATENTSCHRIFT

(11) **EP 0 507 170 B1**
(45) Veröffentlichungstag und Bekanntmachung des Hinweises auf die Patenterteilung: **15.01.1997**
(21) Anmeldenummer: 92104970.6
(22) Anmeldetag: 23.03.1992
(51) Int. Cl.: C12N 15/12, C12Q 1/00

(54) **Gewebe-spezifische humane neuronale Calcium-Kanal-Subtypen und deren Verwendung**
Calcium channel subtype specific of human neuronal tissue and its use
Sous-type du canal calcique spécifique des tissues neuronaux humains et son utilisation

(30) Priorität: 04.04.1991 DE 4110785
(43) Veröffentlichungstag der Anmeldung: 07.10.1992
(73) Patentinhaber: BAYER AG, 51368 Leverkusen (DE)
(72) Erfinder: Franz, Jürgen, Dr., W-5657 Haan (DE); Weingärtner, Bernhard, Dr., W-5603 Wülfrath (DE); Unterbeck, Axel, Dr., W-5060 Bergisch Gladbach 2 (DE); Rae, Peter, Prof., West Haven CT 06516 (US)

(56) Entgegenhaltungen:
- WO-A-89/07608
- WO-A-89/09834
- JOURNAL OF BIOLOGICAL CHEMISTRY Bd. 265, Nr. 33, 25. November 1990, BALTIMORE, US Seiten 20430 - 20436; E. PEREZ-REYES ET AL.: 'Molecular diversity of L-type calcium channels. Evidence for alternative splicing of the transcripts of three non-allelic genes'
- FEBS LETTERS Bd. 274, Nr. 1-2, 12. November 1990, AMSTERDAM, NL Seiten 207 - 213; P. HUANG ET AL.: 'Polymerase chain reaction cloning of L-type calcium channel sequences from the heart and the brain'
- FEBS LETTERS Bd. 250, Nr. 2, Juli 1989, AMSTERDAM, NL Seiten 386 - 388; W.J. KOCH ET AL.: 'Characterization of cDNA clones encoding two putative isoforms of the alpha1-subunit of the dihydropyridine-sensitive voltage-dependent calcium channel isolated from rat brain and rat aorta'
- SOCIETY FOR NEUROSCIENCE ABSTRACTS Bd. 17, Nr. 1-2, 1991, US Seite 772; D.H. FELDMAN ET AL.: 'Cloning and expression of a novel human neuronal calcium channel'
- FEBS LETTERS Bd. 291, Nr. 2, Oktober 1991, AMSTERDAM, NL Seiten 253 - 258; M. PRAGNELL ET AL.: 'Cloning and tissue-specific expression of the brain calcium channel beta-subunit'
- NATURE Bd. 350, 4. April 1991, LONDON GB Seiten 398 - 402; Y. MORI ET AL.: 'Primary structure and functional expression from complementary DNA of a brain calcium channel'
- Ellis et al. (1988), Science, 241, p.1661-1664.

## Beschreibung

Calcium-Ionen haben in jedem biologischen System vielfältige Funktionen. Die zelluläre Calciumhomöostase spielt speziell für die Physiologie von Nervenzellen eine wesentliche Rolle. Die intrazelluläre Calcium-Konzentration beträgt etwa 0,1 µM gegenüber 1 mM außerhalb der Nervenzelle. Die Regulation dieses starken Konzentrationsgefälles (x 10.000) erfolgt primär durch spannungsabhängige Calcium-Kanäle (VOCC), die von bestimmten Calcium-Antagonisten blockier werden können. Während einer cerebralen Ischämie (Hirnschlag) wird die Calciumhomöostase in Neuronen des betroffenen Infarktgebietes erheblich verändert Die spannungsabhängigen Calcium-Kanäle werden durch anhaltende Membrandepolarisationen im geöffneten Zustand gehalten, welches einen massiven Einstrom von Calcium-Ionen zur Folge hat. Die intrazelluläre Calcium-Konzentration steigt dabei um das 1000fache an. Der hohe Überschuß an Calcium aktiviert durch die Bindung an Calmodulin verschiedene Calcium/Calmodulin anhängige zelluläre Enzymsysteme, wie z.B. Kinasen, Proteasen und Phospholipasen, die zusammen, bei anhaltender Aktivierung, zu irrreversiblen Nervenzellschädigungen führen.

Ein therapeutischer Ansatz zur Neuroprotektion bei cerebraler Ischämie ist die reversible Blockierung des massiven Calcium-Einstroms in die Nervenzelle. Die spannungsabhängigen neuronalen Calcium-Kanäle sind hierbei ein geeigneter pharmakologischer Angriffspunkt. VOCCs existieren in verschiedenen Muskelzellen (Gefäß-, Herz- und Skelettmuskel), Neuronen und sekretorischen Zellen mit gewebespezifischen physiologischen Eigenschaften.

Elektrophysiologische Untersuchungen (Tsien et al., 1988, Trends in Neurol. Sci.11: 431-438) deuten auf mindestens drei verschiedene Typen von VOCCs hin (L-, N-und T-Kanäle). Die 1,4-Dihydropyridine (DHPs) sind potente Blocker der L-Typ Calcium-Kanäle, die sowohl in Muskelzellen als auch in Nervenzellen zu finden sind. Der Kaninchen-Skelettmuskel Dihydropyridin Rezeptor ist biochemisch charakterisiert und kloniert worden (Tanabe et al., 1987, Nature 328:313-318). Die Primärsequenz dieser α1 UE des VOCC konnte von den cDNA Daten abgeleitet werden und ist konsistent mit einem 212 kD Transmembranprotein mit fünf N-Glycosylierungsstellen und sieben möglichen Phosphorylierungsstellen. Das Protein enthält vier untereinander ähnliche Transmembrandomänen mit jeweils sechs - vermutlich α-helikalen - membrangängigen Segmenten (S1-S6). Jeweils das vierte Transmembransegment (S4) jeder Domäne enthält ein geordnetes Muster an positiven Ladungen (Lys, Arg), welche den Spannungssensor des Calcium-Kanals bilden können. Die Struktur dieser klonierten α1 UE ist konsistent mit einer Ionen leitenden, spannungsgesteuerten Einheit des DHP-sensitiven Calcium-Kanals.

Der klonierte Skelettmuskel DHP-R cDNA Klon des Karpfens (Grabner et al., 1991, Proc. Natl. Acad. Sci. (USA), 88:727-731) wurde als Hybridisierungsprobe eingesetzt, um verwandte Gene des humanen Calcium-Kanals aus Neuronen zu isolieren und zu charakterisieren. Mit dieser Klonierungsstrategie konnte eine Anzahl von verschiedenen homologen cDNA Klonen aus neuronalen cDNA Banken des Menschen isoliert und charakterisiert werden, die deutlich anzeigen, daß verschiedene Calcium-Kanal Subtypen im ZNS des Menschen existieren. Neuronale Subtypen besitzen neue Rezeptorstellen, für die bislang keine Liganden (Agonisten, Antagonisten) bekannt sind. Die klonierten Calcium-Kanal Subtypen sollen in transformierten Tierzellen (z.B. cos Zellen, Maus L Zellen, CHO Zellen etc.) exprimiert werden (Gluzman, 1981, Cell 23:175, und Chen, et al., 1987, Mol. Cell. Biol. 7:2745-2752) und in Bindungstests und/oder funktionellen Testsystemen zur Auffindung (Screening) neuer, Subtyp-spezifischer Liganden eingesetzt werden. Hierbei werden komplette oder partielle cDNA Gene von verschiedenen Calcium-Kanal Subtypen (inkl. Herz-, Blutgefäß- und Skelettmuskel-Kanäle) in geeignete eukaryontische Expressionsvektoren (Sambrook et al., 1989, in: Molecular Cloning, A laboratory manual, ed. Chris Nolan, Cold Spring Harbor Laboratory Press, New York, N.Y.) kloniert. Die Proteinexpression wird dabei entweder durch homologe Regulatorelemente (Promotoren und Enhancer) oder heterologe Promotoren (virale, z.B. SV40, BPV, CMV, etc. oder induzierbare, z.B. Metallothionein, cAMP, Calcium, Temperatur, etc.) in Kombination mit bekannten Enhancern und RNA Prozessierungssignalen (z.B. Capping, Poly A) gesteuert.

Weiterhin sollen mit diesen rekombinanten Zellsystemen funktionale Calcium-Flux Assays entwickelt werden, mit deren Hilfe spezifische Liganden auf ihre agonistische bzw. antagonistische Wirkung überprüft werden können. Der Unterschied und Hauptvorteil dieser rekombinanten Assays, im Vergleich zu herkömmlichen Assays (Hirnmembranpräparationen, Zellinien), liegt in der Reinheit des Rezeptor/Kanal Präparates, da ausschließlich der rekombinant exprimierte neuronale Calcium-Kanal Subtyp in beliebiger Anzahl auf einer Tierzell-Oberfläche präsent ist. Dies ist eine essentielle Voraussetzung für die Selektion spezifischer neuronaler Calcium-Kanal Liganden, die möglichst keine Wirkung auf Calcium-Kanäle nicht-neuronaler Gewebetypen zeigen sollen.

Nachfolgend sind einige Beispiele für die Anwendung der oben beschriebenen rekombinanten Screeningassays aufgeführt.

### 1. Rezeptor-Bindungs-Test

Die mit humanen Calcium-Kanal Subtypen transformierten Tierzellen (Beispiel: siehe oben) können kultiviert und zur Präparation von Membranen eingesetzt werden. Diese Membranpräparationen können für Bindungsstudien mit verschiedenen radioaktiv markierten Substanzklassen (Beisp. 1-5) zum Screening neuer Liganden (Kompetitionstest) eingesetzt werden. Beispiele für bekannte Calcium-Kanal bindende Substanzen sind:
1. Phenylalkylamine,
2. Benzothiazepine,
3. Dihydropyridine,
4. Bisphenylbutylpiperidine,
5. Omega Conotoxine.

### 2. 45 Calcium-Flux-Test

Die Zellmembranen von humanen Calcium-Kanal Subtypen transformierten Kulturzellen (s.o.) können mit Kalium-Ionen oder mit Alkaloiden wie z.B. Veratridine depolarisiert werden. Membrandepolarisation führt zur Öffnung von Calcium-Kanälen, was einen Einstrom (Flux) von Calcium-Ionen in die Zellen zur Folge hat. Dieser spannunngsabhängige Calcium-Einstrom kann mit radioaktiv markiertem Calcium (45Ca) gemessen werden (Beisp.: Messing et al., 1985, J. Pharmacology and Exp. Therapeutics 235:407-411) und zum funktionellen Testen/Screening von Calcium-Kanal Antagonisten oder Agonisten eingesetzt werden.

### 3. Fura-2 Test

Humane Calcium-Kanal exprimierende Tierzellen (s.o.) können in Gegenwart von Calcium sensitiven, fluoreszierenden Farbstoffen (z.B. Fura-2 oder Fluoro-3) für Messungen der intrazellulären Calcium-Konzentration nach Öffnung und Blockierung der Calcium-Kanäle eingesetzt werden (Beisp.: Rosario et al.1989 , Neurosci. 29,735-747). Die Änderung der intrazellulären Calcium-Konzentration kann dabei fluorimetrisch (spektrophotometrisch) gemessen werden. Dieses rekombinante Zellsystem kann als funktioneller Test für das Auffinden Subtyp-spezifischer Calcium-Kanal Liganden(Agonisten und Antagonisten) eingesetzt werden.

### 4. Elektrophysiologie

Die durch Membrandepolarisation erzeugten Calcium-Ströme können elektrophysiologisch gemessen werden (Beisp.: Carbone et al. 1990, Pflügers Arch., 416: 170-179). Die Wirkung von potentiellen Calcium-Kanal Antagonisten oder Agonisten kann direkt an humanen Calcium-Kanälen mit Hilfe der rekombinanten Tierzellinien (s.o.) physikalisch gemessen und pharmakologisch charakterisiert werden.

### 5. Indirekte Meßmethoden

Viele zelluläre Prozesse werden von der intrazellulären Calcium-Ionen Konzentration reguliert (z.B. Rezeptor mediierte Signalübertragung, verschiedene Enzymreaktionen, wie z.B. Phosphorylierungen, Dephosphorylierungen, Neurotransmitter-Freisetzung, Ca-abhängige Genregulation, etc.). Einige dieser biochemischen Reaktionen sind mit einem spezifischen Assay meßbar. In einem rekombinanten Calcium-Kanal exprimierenden Zellsystem kann somit indirekt (physiologisch) die Wirkung von Calcium-Kanal Modulatoren auf Calcium abhängige Zellvorgänge erfaßt werden(Beisp.: Zernig et al. 1986, Eur.J.Pharmacol. 128,221-229).

Zusätzlich können durch gezielte Mutagenesen eingeführte Veränderungen, wie z.B. Punktmutationen, Insertionen, Deletionen, Austausch von DNA-Segmenten verschiedener Calciumkanal-Subtypen, direkte Auswirkungen auf physiologische Vorgänge erfaßt werden (Bsp.: Yool and Schwarz, 1991, Nature 349:700-704).

### 1. cDNA Methoden/Banken

Zur Isolierung humaner neuronaler Ca-Kanäle mittels Homologiescreening wurden folgende käuflich erworbenen cDNA-Banken eingesetzt:
a) cDNA Bank aus einer humanen Neuroblastomazellinie
   - Vektor:: Lambda gt10
   - Quelle:: Fa. Clontech Laboratories, Inc. Palo Alto, CA. USA: (Kat. Nr. HL 1007a)
b) cDNA Bank aus humanem Hippocampus;
   - Vektor:: Lambda ZAPII
   - Quelle:: Fa. Stratagene Inc., La Jolla,CA, CA, USA (Kat. Nr. 936205)
c) cDNA Bank aus humanem temporalem Cortex
   - Vektor:: Lambda ZAPII
   - Quelle:: Fa. Stratagene Inc., La Jolla, CA, USA (Kat. Nr. 935205)
d) cDNA Bank aus humanem visuellem Cortex
   - Vektor:: Lambda gt10
   - Quelle:: Fa. Clontech Laboratories, Palo Alto, CA, USA (Kat. Nr. HL 1081a)
e) cDNA Bank aus humanem frontalem Cortex
   - Vektor:: Lambda ZAPII
   - Quelle:: Fa. Stratagene Inc., La Jolla, CA, USA (Kat. Nr. 935205)

### 2. Screenen der cDNA Banken

### 2.1 Ausplattieren der cDNA Banken und Prozessieren der Nitrozellulosefilter

Das Ausplattieren der cDNA Banken und das Prozessieren der Nitrozellulosefilter erfolgte gemäß den Angaben der Hersteller oder nach Sambrook et al., 1989, (Molecular Cloning, A laboratory manual, ed. Chris Nolan, Cold Spring Harbor Laboratory Press,New York,N.Y.).

### 2.2 Hybridisierungsproben

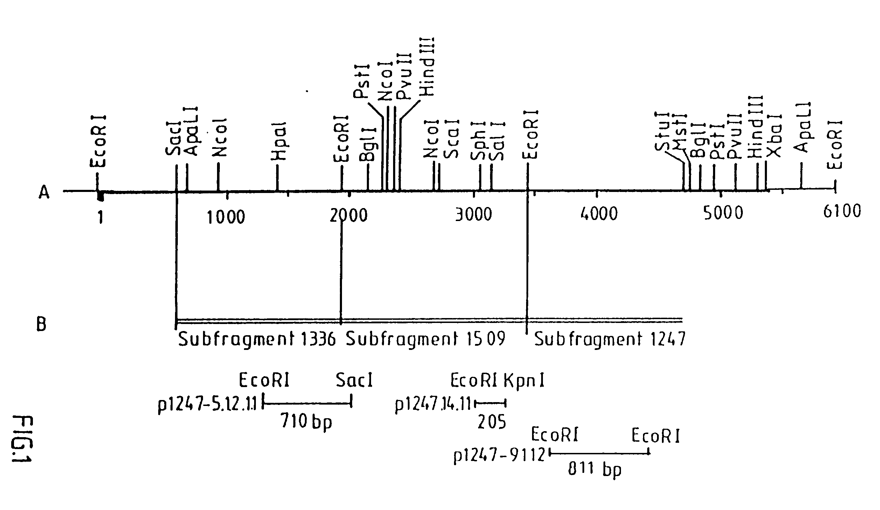

Als Hybridisierungsprobe diente ein 6.1 kb langer cDNA Klon (Abb. 1), der die gesamte kodierende Region, einschließlich 5'- und 3'- nicht translatierter Bereiche der α₁-Untereinheit (UE) des Ca-Kanals der Skelettmuskulatur des Karpfens (Cyprinus carpio) enthält (Grabner et al., 1990, Proc. Natl. Acad. Sci. (USA), 88: 727-731). Für das Homologiescreening wurden folgende Abschnitte dieses Klons eingesetzt (Abb.1):
- der komplette cDNA Klon (6.1 kb)
- Subfragment 1 336
- Subfragment 1 509
- Subfragment 1 247.

Zum weiteren Screenen von cDNA Banken wurden folgende Fragmente humaner Ca-Kanal cDNA Klone eingesetzt:
- Insert des Klons p 1247-9.1.1.2(811 bp)
- Subfragment des Klons p 1247-14.1.1.1 (EcoRI-KpnI; 205 bp)
- Subfragment des Klons p 1247-5.1.2.1.1 (EcoRI-SacI; 710bp)
- Insert des Klons pCA 33 (684 bp)
- Subfragmente des Klons pCA 3
   (EcoRI-EcoRI, 640pb; PstI-PstI, 198 bp; PstI-PstI,600bp)

### 2.3 Markieren von DNA Fragmenten mit radioaktiven DNA Vorläufern

Zur Markierung von doppelsträngigen DNA Fragmenten wurden Standardprotokolle (Sambrook et al., 1989 in: Molecular Cloning, A laboratory manual, ed. Chris Nolan, Cold Spring Harbor Laboratory Press, New York, N.Y.) in Verbindung mit einem käuflichen "Random Primed Labeling Kit" (Boehringer Mannheim GmbH, Postfach 310120,D-6800 Mannheim; Ka. Nr. 1004 760) angewendet.

### 2.4 Hybridisier- und Waschbedingungen

Die Nitrozellulosefilter wurden über Nacht mit radioaktiv markierten cDNA Fragmenten in 30 % Formamid, 5 x Denhardt's Lösung, 5 x SSC bei 42°C hybridisiert und anschließend wie folgt gewaschen:
- 2 x 20 Minuten in 2 x SSC, 0,05 % SDS bei Raumtemperatur
- 2 x 20 Minuten in 0,2 x SSC, 0,2 % SDS bei 45°C und
- 1 x 20 Minuten in 0,2 x SSC bei Raumtemperatur.

Danach wurde ein Kodak X-OMAT AR Röntgenfilm für unterschiedliche Zeiten bei -80°C mit Verstärkerfolien durch die Filter belichtet.

### 3. Isolieren der Lambda Phagen, Subklonieren und Sequenzieren der cDNA Inserts

### 3.1 cDNA Inserts aus Lambda gt 10

Die Lambda Phagen DNA wurde isoliert, mit EcoRI geschnitten, die cDNA Inserts in ein pUC-Derivat (pT7T3 18U; Fa. Pharmacia) subkloniert und anschließend mit Sequenase (Fa. USB, Cleveland, Ohio, USA) mittels der Dideoxy-Terminationsmethode nach Sanger (Sanger et al., 1977, Proc. Natl. Acad. Sci. USA, 74: 5463-5467.) die Nukleotidsequenz bestimmt.

### 3.2 cDNA Inserts aus Lambda ZAPII

### 3.2.1 Ausschneiden der kompletten cDNA Inserts aus Lambda-Phagen und Überführen der Inserts in Plasmide

Die cDNA Inserts aus positiven Lambda-ZAPII Phagen wurden nach einem Protokoll des Herstellers (Stratagene) mittels eines fl-abgeleiteten Helferphagens vorgenommen und in die Plasmidform überführt.

### 3.2.2 Bestimmung der Größe und Sequenzanalyse der isolierten cDNA Inserts

Aus XL1-Blue Zellen, die ein rekombinates pBluescript Plasmid trugen, wurde Plasmid DNA präpariert (Sambrook, J., et al., (1989) in: Molecular cloning, A laboratory manual, ed. Chris Nolan, Cold Spring Harbor Laboratory Press, New York, N.Y.) und jeweils 0,5 µg dieser DNA mit dem Restriktionsenzym EcoRI behandelt. Aus Anzahl und Größe der entstandenen DNA Fragmente konnte die Gesamtlänge der insertierten cDNA abgeleitet werden. Die Nukleotidsequenz der vorhandenen cDNA wurde mit SEQUENASE (USB, Cleveland, Ohio, USA) nach der Methode von Sanger an doppelsträngiger DNA ermittelt.

### Beschreibung der bisher isolierten cDNA Klone für humane neuronale Ca-Kanäle

Folgende cDNA Klone wurden bisher isoliert und konnten durch DNA- und Aminosäuresequenzvergleiche mit anderen bekannten Ca-Kanalsequenzen als Ca-Kanäle identifiziert werden (als Referenz dient die Nukleotidsequenz des Ca-Kanals der Skelettmuskulatur des Kaninchens (Tanabe et al., Nature 328, 313-318), die Numerierung der Nukleotide bzw. Aminosäuren ist analog zur Numerierung der EMBL-Datenbank):

### Kombination überlappender und in ihrer Nukleotidsequenz identischer Calciumkanal cDNA Subklone.

In ihren überlappenden Sequenzen übereinstimmende cDNA-Subklone werden über geeignete Restriktionsschnittstellen zu einem kompletten oder partiellen cDNA-Klon zusammengesetzt, der für einen bestimmten Calciumkanal-Subtyp kodiert. Dieses cDNA-Gen wird mittels eines eukaryontischen Expressionsvektor in Sängerzellen exprimiert und in den unter den Beispielen Punkt 1 bis 5 beschriebenen Test eingesetzt.

### 1. pCA33:

Länge: 683 Nukleotide, Position AS 1.000-1.230 (3' zusätzliche 3 AS); umfaßt Sequenzen der dritten Domäne ab IIIs6 bis vierte Domäne IVs3.

### 2. p1247-5.1.2.1.1:

Länge von ca 4.919 bp; Position ab AS 343 bis zum Ende der kodierenden Region; enthält damit nach Domäne I das komplette Gen.

### 3. p1247-9.1.1.2:

Länge 811 bp; Position AS 1.115-1.390 und umfaßt damit die gesamte Domäne IV (s1 bis s6)und flankierende Sequenzen auf beiden Seiten.

### 4. p1247-10.1.1.1:

Länge 1.354 bp; Position AS 1.050-1.512 und umfaßt damit das Ende der dritten Domäne (IIIs6) und die gesamte Domäne IV und etwa 130 c-terminal flankierende AS, die dem letzten zytoplasmatischen Teil des Proteins zuzuordnen sind.

### 5. p1247-14.1.1.1:

Länge: 5.438 bp; Position AS 967-1.327. Dieser Klon überlappt über einen großen Bereich (Position 1-3.238) mit Klon pR14-5.3.3.1. (Position 2.988-4.232). Im überlappenden Teil sind beide Klone nahezu identisch. Es bestehen folgende Unterschiede zu pR14-5.3.3.1 (Nukleotid und Position bei pR14-5.3.3.1 sind jeweils in Klammern angegeben):
(1) Cytosin an Position 520 (T; 3.507); keine Änderung der abgeleiteten Proteinsequenz.
(2) Cytosin an Position 775 (G; 3.768); keine Änderung der abgeleiteten Proteinsequenz.
(3) Cytosin an Position 1.617 (T; 4.611).
(4) Adenosin an Position 2.360 (G; 5.353).
(5) Deletion von sechs Nukleotiden an Position 708 (CGGAAA;3.695-3.700).
(6) Deletion eines Adenosinrestes an Position 1.013; dies führt im Vergleich zu pR14-5.3.3.1 zu einer Verschiebung im Leseraster, so daß an Position 1.028-1.030 ein Stop-Codon das abgeleitete Protein terminiert.
(7) Ab Position 3.240 sind weitere 2.199 Nukleotide der 3'-nichttranslatierten Region vorhanden, die bei pR14-5.3.3.1 fehlen. Es folgt dann ein Teil eines Polyadenylat-Schwanzes.

Aufgrund der hohen Ähnlichkeit der Klone p1247-14.1.1.1 und pR14-5.3.3.1 ist davon auszugehen, daß es sich bei der Deletion von einem Nukleotid an Position 1.013 um einen Artefakt während der cDNA Synthese handelt.

### 6. pR9112-4.1.1.1:

Länge ca. 1.722 bp; Position AS 1.223-1.870; dieser Klon enthält somit ab s4 den c-terminalen Teil der vierten Domäne, einschließlich der kompletten kodierenden Sequenz bis zum tatsächlichen C-Terminus des Proteins. Dieser Klon ist fast identisch mit überlappenden Sequenzen des Klons 1247-9.1.1.2. Die Sequenzen von pR9112-4.1.1.1 und pR9112-2.1.1.1 sind im wesentlichen identisch (1 bp Unterschied von bisher 1.464 sequenzierten Nukleotiden des Überlapps); die Klone sind wahrscheinlich überlappende cDNA Klone der selben mRNA.

### 7. pR9112-10.1.1.1:

Länge 2.049 bp; Position von AS 991-1.650. Dieser Klon enthält cDNA Sequenzen, die für einen Teil der Domäne III (s6), die gesamte vierte Domäne und einen Teil des c-terminalen zytoplasmatischen Teils des Proteins kodieren. Der Klon pR9112-10.1.1.1 unterscheidet sich von pR9112-2.1.1.1 und pR9112-4.1.1.1 durch einen 57 bp Insert (1.4541-1.510). Dieses Insert besitzt an beiden Enden Splicing-Konsensussequenzen und bietet damit die Mögiichkeit für alternatives Splicing.

### 8. pR9112-12.1.1.1:

Länge 997 bp; Position bis AS 1.509. Die Seqzenz dieses cDNA-Klons ist fast identisch mit Klon p1247-14.1.1.1. Das 5' Ende des Klons enthält dazu als Kloningsartefakt etwa 250 bp mitochondrialer DNA, sowie ein poly(A)-Anteil von 39 bp.

### 9. pR9112-2.1.1.1:

Länge 1.471 bp; wie cDNA Klon pR9112-4.1.1.1, enthält dieser Klon ab IVs3-4 die komplette kodierende Region bis zum tatsächlichen C-Terminus des Proteins, sowie etwa 500 bp der 3' nicht translatierten Region der mRNA.

### 10. pRR5-8:

Länge 2.655 bp; am 5'-Ende enthält dieser Klon 235 bp nichttranslatierte Sequenz gefolgt von einem ATG Startcodon (Pos. 236-238). Von diesem Startcodon erstreckt sich ein offenes Leseraster bis zum 3'-Ende des Klons. Das daraus abgeleitete Protein beginnt mit dem tatsächlichen N-Terminus eines Ca-Kanal cDNA-Gens und enthält die Domänen I und II, sowie einen Teil des intrazellulären Loops zwischen Domäne II und III. Ab Position 1.318 bis zum 3'-Ende überlappt der Klon mit dem Klon p1247-5.1.2.1.1. Beide Klone können z.B. an einer gemeinsamen Xhol-Schnittstelle (Pos. 1.506-1.511 bei pRR5-8) miteinander kombiniert werden zu einer cDNA, die neben dem zytoplasmatischen N-Terminus für die Domänen I-IV und angrenzende zytoplasmatische c-terminale Bereiche des Ca-Kanals kodiert.

### 11. pR14-5.3.3.1:

Länge: 6.232 bp; Position AS 358 bis zum C-Terminus eines Ca-Kanals. Am 5'-Ende enthält dieser Klon 252 bp, die 85 % Homologie zu humanen Alu-repeat Sequenzen aufweisen und möglicherweise artifiziell während der cDNA Klonierung an die restlichen 5.980 bp der Ca-Kanal cDNA ligiert wurden. Das offene Leseraster des für einen Ca-Kanal kodierenden cDNA Stranges kodiert für 1.931 AS und umspannt die Domänen II bis IV, sowie den C-terminalen cytoplasmatischen Anteil des Ca-Kanals. Es folgen 187 bp 3'-nichttranslatierte Region, einschließlich eines Polyadenylierungssignal an Position 6.215-6.220, und läuft in ein poly A-Ende aus (44 Adenosinreste).

### 12. pCA3

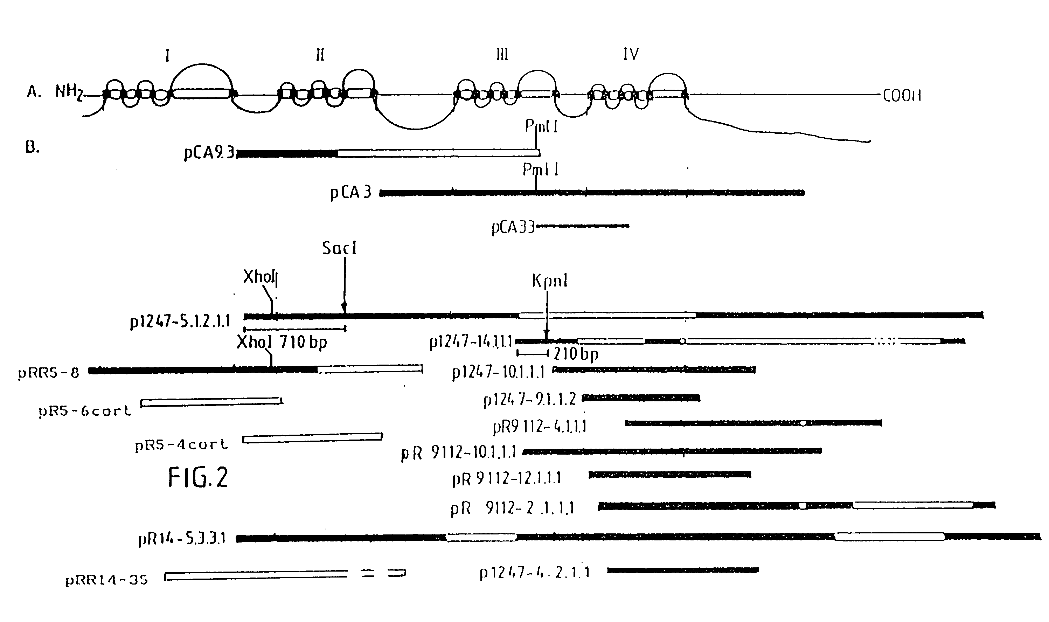

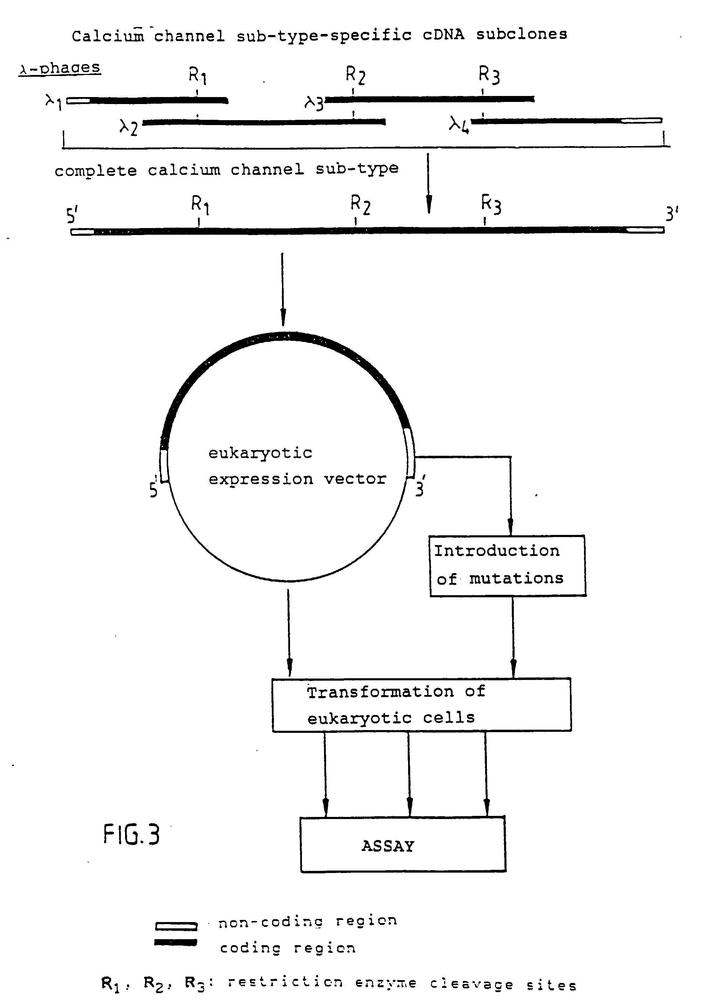

Länge 2.837 bp; besitzt eine interne EcoRI-Schnittstelle (2 Subfragmente: 2.197 bp, 640 bp). Das 5'-Ende des cDNA Klons liegt zwischen Domäne II und III, das 3'-Ende bei AS 1.622; der Klon umfaßt die vollständigen Domänen III und IV und einen Teil der für den C-Terminus kodierenden Sequenz. Das 5'-Ende des cDNA-Klons überlappt mit dem 3'-Ende des Klons pCA9.3 auf einer Länge von 830 bp. Beide cDNA-Klone sind über 671 bp identisch, lediglich die ersten 159 bp des 5'-Endes von Klon pCA3 zeigen zu dem entsprechenden Abschnitt des Klons pCA9.3 keinerlei Homologien (Abb. 2, schraffierter Bereich). Kon pCA 3 und Klon pCA9.3 über eine gemeinsamePmII Restriktionsschnittstelle im überlappenden Bereich kombiniert werden.

### 13. pCA9.3

Länge 1.857 bp; das 5'-Ende des cDNA Klons beginnt unmittelbar nach Domäne I (AS 337); das 3'-Ende liegt bei AS 922; der cDNA-Klon umfaßt Sequenzen des zweiten zytoplasmatischen Abschnittes (zwischen Domäne I und II) bis einschließlich der 4. Transmembranregion der Domäne III(IIIS4).

### 14. p1247-4.2.1.1:

Länge 920 bp; Position AS 1.178-1.496 der Kaninchenskelettmuskel α₁-Untereinheit (Domäne IVs3-IVs6). Die Sequenz des Klons p1247-4.2.1.1 ist vollständig in der Sequenz des Klons p1247-10.1.1.1 enthalten. Beide Klone sind bis auf eine 6 Basenpaare umfassende Insertion in Klon 1247-4 (Position 88-93) und 2 weitere Basenaustausche identisch.

### 15. pR5-6cort

Länge: 1,424 bp; Position AS 25-458. Am 5'-Ende enthält dieser Klon an Position 60 eine putative Splice-Donor-Stelle, so daß die ersten 60 Nukleotide möglicherweise Intronsequenzen darstellen. Der für einen Ca-Kanal kodierende Bereich (ab Position 61) umfaßt einen Teil der N-terminalen cytoplasmatischen Region, sowie die gesamte Domäne I und die erste membrandurchbrechende Region (S1) der Domäne II.

### 16. pR5-4cort

Länge: 910 bp; Position AS 409-713. Dieser Klon überlappt am 5'-Ende 151 bp mit dem 3'-Ende des Klons pR5-6cort (100 % Identität über 151 bp). Diese beiden Klone stellen daher unabhängig klonierte cDNA Abschnitte ein und derselben mRNA dar und können z.B. über die gemeinsame Stu I Restruktionsabschnittstelle miteinander kombiniert werden.

### 17. pRR14-35(5'-Ende des Klons)

Länge: ca 3.400bp, hiervon sind bisher 1.100 bp sequenziert; Position: das 5'-Ende des Klons liegt bei AS 257 und somit zwischen den Membrandurchgängen S5 und S6 der Domäne I. Im bisher sequenzierten Bereich überlappt der Klon Sequenzidentisch mit pR14-5.3.3.1 (Position 253-964). Somit können die beiden Klone pRR14-35 und pR14-5.3.3.1 als 2 unabhängig klonierte cDNA Abschnitte einer mRNA angesehen werden. Klon pRR14-35 ergänzt die in pR14-5.3.3.1 enthaltene Ca-Kanal cDNA zum 5'-Ende um 129 AS und eliminiert hierbei die artiflziell vorhandene Alu-repeat Sequenz am 5'-Ende dieses Klons. Beide Klone können über eine gemeinsame Bgl II Restriktionsschnittstelle miteinander kombiniert werden.

### Beschreibung zu den Abbildungen:

Im oberen Teil (A) von Abbildung 1 ist die cDNA des Skelettmuskel Ca-Kanals des Karpfens mit den Schnittstellen für einige Restriktionsendonukleasen und den Subfragmenten dargestellt, die als Screeningproben verwendet wurden. Im unteren Teil (B) sind DNA Fragmente der humanen Klone p1247-5.1.2.1.1 und p1247-14.1.1, die mit den Restriktionsenzymen EcoRI/SacI bzw. EcoRI/KpnI erzeugt wurden, sowie das gesamte cDNA Insert des Klons p1247-9.1.1.2, die ebenfalls als Screeningproben verwendet wurden.

Abbildung 2 zeigt im oberen Teil (A) eine schematische Darstellung der α₁-Untereinheit des Skelettmuskel Ca-Kanals vom Kaninchen. Die 4 Domänen sind als Kästen gekennzeichnet und mit römischen Ziffern beschriftet. Innerhalb dieser Domänen sind die Transmembranbereiche als schwarze Blöcke hervorgehoben Im unteren Teil (B) sind die Teilstücke der bisher isolierten humanen neuronalen Ca-Kanäle entsprechend ihrer Größe und ihrer Homologie zu dem Kaninchen Skelettmuskel Ca-Kanal Protein angeordnet.

### Abbildung 3:

Schematische Darstellung der Klonierung und Expression gewebespezifischer humaner neuronaler Calcium-Kanal-Subtypen und deren Verwendung in einem Testsystem. Die vollständige, für einen Calcium-Kanal-Subtyp kodierende cDNA wird mittels geeigneter Restriktionsschnittstellen aus überlappenden Lambda-Phagen zusammengesetzt und in einen eukaryontischen Expressionsvektor kloniert.

Mit diesem Vektor werden geeignete eukaryontische Zellen transformiert und stabile Zellinien hergestellt, die das Protein eines Calcium-Kanal-Subtyps exprimieren. Diese stabilen Zellinien werden dann in den beschriebenen Rezeptor-Bindungstest eingesetzt.

Zusätzlich in die für den Calcium-Kanal kodierende cDNA eingeführte Mutationen sollen zur Identifizierung von Struktur-Funktionsdomänen führen.

### Sequenzprotokoll:

- Sequenz Nr.:: 27980/1
- Int. Code:: pCA33
- Länge:: 683 bp
- Typ:: DNA + Aminosäure

10                    30                    50

GTCCTCTCTGCTATGATGGCGCTCTTCACAGTCTCCACGTTTGAGGGCTGGCCTGCGTTG
ValLeuSerAlaMetMetAlaLeuPheThrValSerThrPheGluGlyTrpProAlaLeu

70                    90                    110

CTGTATAAAGCCATCGACTCGAATGGAGAGAACATCGGCCCAATCTACAACCACCGCGTG
LeuTyrLysAlaIleAspSerAsnGlyGluAsnIleGlyProIleTyrAsnHisArgVal

130                   150                   170

GAGATCTCCATCTTCTTCATCATCTACATCATCATTGTAGCTTTCTTCATGATGAACATC
GluIleSerIlePhePheIleIleTyrIleIleIleValAlaPhePheMetMetAsnIle

190                   210                   230

TTTGTGGGCTTTGTCATCGTTACATTTCAGGAACAAGGAGAAAAAGAGTATAAGAACTGT
PheValGlyPheValIleValThrPheGlnGluGlnGlyGluLysGluTyrLysAsnCys

250                   270                   290

GAGCTGGACAAAAATCAGCGTCAGTGTGTTGAATACGCCTTGAAAGCACGTCCCTTGCGG
GluLeuAspLysAsnGlnArgGlnCysValGluTyrAlaLeuLysAlaArgProLeuArg

310                   330                   350

AGATACATCCCCAAAAACCCCTACCAGTACAAGTTCTGGTACGTGGTGAACTCTTCGCCT
ArgTyrIleProLysAsnProTyrGlnTyrLysPheTrpTyrValValAsnSerSerPro

370                   390                   410

TTCGAATACATGATGTTTGTCCTCATCATGCTCAACACACTCTGCTTGGCCATGCAGCAC
PheGluTyrMetMetPheValLeuIleMetLeuAsnThrLeuCysLeuAlaMetGlnHis

430                   450                   470

TACGAGCAGTCCAAGATGTTCAATGATGCCATGGACATTCTGAACATGGTCTTCACCGGG
TyrGluGlnSerLysMetPheAsnAspAlaMetAspIleLeuAsnMetValPheThrGly

490                   510                   530

GTGTTCACCGTCGAGATGGTTTTGAAAGTCATCGCATTTAAGCCTAAGGGGTATTTTAGT
ValPheThrValGluMetValLeuLysValIleAlaPheLysProLysGlyTyrPheSer

550                   570                   590

GACGCCTGGAACACGTTTGACTCCCTCATCGTAATCGGCAGCATTATAGACGTGGCCCTC
AspAlaTrpAsnThrPheAspSerLeuIleValIleGlySerIleIleAspValAlaLeu

610                   630                   650

AGCGAAGCAGACCCAACTGAAAGTGAAAATGTCCCTGTCCCAACTGCTACACCTGGGAAC
SerGluAlaAspProThrGluSerGluAsnValProValProThrAlaThrProGlyAsn

670

TCTGAAGAGAGCAATAGAATC
SerGluGluSerAsnArgIle

### Sequenzprotokoll:

- Sequenz Nr.:: 27980/2
- Int. Code:: p1247-5.1.2.1.1 (entire clone)
- Länge:: 4919 bp
- Typ:: DNA + Aminosäure

10 30 50
GGAGAACCGAAGGGCTTTCATGAAGCTGCGGCGCCACGAGCAGATTGAGCGTGACGTGAAT
GluAsnArgArgAlaPheMetLysLeuArgArgHisGluGlnIleGluArgAspValAsn

70 90 110
GGCTACCGTGCCTGGATAGACAAAGCAGAGGAAGTCATGCTCGCTGAAGAAAATAAAAAT
GlyTyrArgAlaTrpIleAspLysAlaGluGluValMetLeuAlaGluGluAsnLysAsn

130 150 170
GCTGGAACATCCGCCTTAGAAGTGCTTCGAAGGGCAACCATGAAGAGGAGCCGGACAGAG
AlaGlyThrSerAlaLeuGluValLeuArgArgAlaThrMetLysArgSerArgThrGlu

190 210 230
GCCATGACTCGAGACTCCAGTGATGAGCACTGTGTTGATATCTCCTCTGTGGGCACACCT
AlaMetThrArgAspSerSerAspGluHisCysValAspIleSerSerValGlyThrPro

250 270 290
CTGGCCCGAGCCAGTATCAAAAGTGCAAAGGTAGACGGGGTCTCTTATTTCCGGCACAAG
LeuAlaArgAlaSerIleLysSerAlaLysValAspGlyValSerTyrPheArgHisLys

310 330 350
GAAAGGCTTCTGCGCATCTCCATTCGCCACATGGTTAAATCCCAGGTGTTTTACTGGATT
GluArgLeuLeuArgIleSerIleArgHisMetValLysSerGlnValPheTyrTrpIle

p1247-5.1.2.1.1, cont.

370 390 410
GTGCTGAGCCTTGTGGCACTCAACACTGCCTGTGTGGCCATTGTCCATCACAACCAGCCC
ValLeuSerLeuValAlaLeuAsnThrAlaCysValAlaIleValHisHisAsnGlnPro

430 450 470
CAGTGGCTCACCCACCTCCTCTACTATGCAGAATTTCTGTTTCTGGGACTCTTCCTCTTG
GlnTrpLeuThrHisLeuLeuTyrTyrAlaGluPheLeuPheLeuGlyLeuPheLeuLeu

490 510 530
GAGATGTCCCTGAAGATGTATGGCATGGGGCCTGCCCTTTATTTTCACTCTTCATTCAAC
GluMetSerLeuLysMetTyrGlyMetGlyProAlaLeuTyrPheHisSerSerPheAsn

550 570 590
TGCTTTGATTTTGGGGTCACAGTGGGCAGTATCTTTGAAGTGGTCTGGGCAATCTTCAGA
CysPheAspPheGlyValThrValGlySerIlePheGluValValTrpAlaIlePheArg

610 630 650
CCTGGTACGTCTTTTGGAATCAGTGTCTTGCGAGCCCTCCGGCTTCTAAGAATATTTAAA
ProGlyThrSerPheGlyIleSerValLeuArgAlaLeuArgLeuLeuArgIlePheLys

670 690 710
ATAACCAAGTATTGGGCTTCCCTACGGAATTTGGTGGTCTCCTTGATGAGCTCAATGAAG
IleThrLysTyrTrpAlaSerLeuArgAsnLeuValValSerLeuMetSerSerMetLys

p1247-5.1.2.1.1, cont.

730 750 770
TCTATCATCAGTTTGCTTTTCCTCCTCTTCCTCTTCATCGTTGTCTTTGCTCTCCTAGGA
SerIleIleSerLeuLeuPheLeuLeuPheLeuPheIleValValPheAlaLeuLeuGly

790 810 830
ATGCAGTTATTTGGAGGCAGGTTTAACTTTAATGATGGGACTCCTTCGGCAAATTTTGAT
MetGlnLeuPheGlyGlyArgPheAsnPheAsnAspGlyThrProSerAlaAsnPheAsp

850 870 890
ACCTTCCCTGCAGCCATCATGACTGTGTTCCAGATCCTGACGGGTGAGGACTGGAATGAG
ThrPheProAlaAlaIleMetThrValPheGlnIleLeuThrGlyGluAspTrpAsnGlu

910 930 950
GTGATGTACAATGGGATCCGCTCCCAGGGTGGGGTCAGCTCAGGCATGTGGTCTGCCATC
ValMetTyrAsnGlyIleArgSerGlnGlyGlyValSerSerGlyMetTrpSerAlaIle

970 990 1010
TACTTCATTGTGCTCACCTTGTTTGGCAACTACACGCTACTGAATGTGTTCTTGGCTATC
TyrPheIleValLeuThrLeuPheGlyAsnTyrThrLeuLeuAsnValPheLeuAlaIle

1030 1050 1070
GCTGTGGATAATCTCGCCAACGCCCAGGAACTGACCAAGGATGAACAGGAGGAAGAAGAG
AlaValAspAsnLeuAlaAsnAlaGlnGluLeuThrLysAspGluGlnGluGluGluGlu

1090 1110 1130
GCCTTCAACCAGAAACATGCACTGCAGAAGGCCAAGGAGGTCAGCCCGATGTCTGCACCC
AlaPheAsnGlnLysHisAlaLeuGlnLysAlaLysGluValSerProMetSerAlaPro

p1247-5.1.2.1.1, cont.

1150 1170 1190
AACATGCCTTCGATCGAAAGAGACAGAAGGAGAAGACACCACATGTCGATGTGGAGCCA
AsnMetProSerIleGluArgAspArgArgArgArgHisHisMetSerMetTrpGluPro

1210 1230 1250
CGCAGCAGCCACCTGAGGGAGCGGaggcgccGGCACCACATGTCCGTGTGGGAGCAGCGT
ArgSerSerHisLeuArgGluArgArgArgArgHisHisMetSerValTrpGluGlnArg

1270 1290 1310
ACCAGCCAGCTGAGGAAGCACATGCAGATGTCCAGCCAGGAGGCCCTCAACAGAGAGGAG
ThrSerGlnLeuArgLysHisMetGlnMetSerSerGlnGluAlaLeuAsnArgGluGlu

1330 1350 1370
GCGCCGACCATGAACCCGCTCAACCCCCTCAACCCGCTCAGCTCCCTCAACCCGCTCAAT
AlaProThrMetAsnProLeuAsnProLeuAsnProLeuSerSerLeuAsnProLeuAsn

1390 1410 1430
GCCCACCCCAGCCTTTATCGGCGACCCAGGGCCATTGAGGGCTGGCCTGGGCTGGCCCTG
AlaHisProSerLeuTyrArgArgProArgAlaIleGluGlyTrpProGlyLeuAlaLeu

1450 1470 1490
GAGAAGTTCGAGGAGGAGCGCATCAGCCGTGGGGGGTCCCTCAAGGGGGATGGAGGGGAC
GluLysPheGluGluGluArgIleSerArgGlyGlySerLeuLysGlyAspGlyGlyAsp

1510 1530 1550
CGATCCAGTGCCCTGGACAACCAGAGGACCCCTTTGTCCCTGGGCCAGCGGGAGCCACCA
ArgSerSerAlaLeuAspAsnGlnArgThrProLeuSerLeuGlyGlnArgGluProPro

p1247-5.1.2.1.1, cont.

1570 1590 1610
TGGCTGGCCAGGCCCTGTCATGGAAACTGTGACCCGACTCAGCAGGAGGCAGGGGGAGGA
TrpLeuAlaArgProCysHisGlyAsnCysAspProThrGlnGlnGluAlaGlyGlyGly

1630 1650 1670
GAGGCTGTGGTGACCTTTGAGGACCGGGCCAGGCACAGGCAGAGCCAACGGCGCAGCGCG
GluAlaValValThrPheGluAspArgAlaArgHisArgGlnSerGlnArgArgSerAla

1690 1710 1730
CATCGCCGCGTCAGGACAGAAGGCAAGGAGTCCTCTTCAGCCTCCCGGAGCAGGTCTGCC
HisArgArgValArgThrGluGlyLysGluSerSerSerAlaSerArgSerArgSerAla

1750 1770 1790
AGCCAGGAACGCAGTCTGGATGAAGCCATGCCCACTGAAGGGGAGAAGGACCATGAGCTC
SerGlnGluArgSerLeuAspGluAlaMetProThrGluGlyGluLysAspHisGluLeu

1810 1830 1850
AGGGGCAACCATGGTGCCAAGGAGCCAACGATCCAAGAAGAGAGAGCCCAGGATTTAAGG
ArgGlyAsnHisGlyAlaLysGluProThrIleGlnGluGluArgAlaGlnAspLeuArg

1870 1890 1910
AGGACCAACAGTCTGATGGTGTCCAGAGGCTCCGGGCTGGCAGGAGGCCTTGATGAGGCT
ArgThrAsnSerLeuMetValSerArgGlySerGlyLeuAlaGlyGlyLeuAspGluAla

1930 1950 1970
GACACCCCCCTAGTCCTGCCCCATCCTGAGCTGGAAGTGGGGAACGACGTGGTGTCGACG
AspThrProLeuValLeuProHisProGluLeuGluValGlyAsnAspValValSerThr

p1247-5.1.2.1.1, cont.

1990 2010 2030
GAGCAGGAGCCAGAAGGCAGCAGTGAGCAGGCCCTGCTGGGGAATGTGCAGCTAGACATG
GluGlnGluProGluGlySerSerGluGlnAlaLeuLeuGlyAsnValGlnLeuAspMet

2050 2070 2090
GGCCGGGTCATCAGCCAGAGCGAGCCTGACCTCTCCTGCATCACGGCCAACACGGACAAG
GlyArgValIleSerGlnSerGluProAspLeuSerCysIleThrAlaAsnThrAspLys

2110 2130 2150
GCCACCACCGAGAGCACCAGCGTCACCGTCGCCATCCCCGACGTGGACCCCTTGGTGGAC
AlaThrThrGluSerThrSerValThrValAlaIleProAspValAspProLeuValAsp

2170 2190 2210
TCAACCGTGGTGCACATTAGCAACAAGaCGGATGGGGAAGCCAGTCCCTTGAAGGAGGCA
SerThrValValHisIleSerAsnLysThrAspGlyGluAlaSerProLeuLysGluAla

2230 2250 2270
GAGATCAGAGAGGATGAGGAGGAGGTGGAGAAGAAGAAGCAGAAGAAGGAGAAGCGTGAG
GluIleArgGluAspGluGluGluValGluLysLysLysGlnLysLysGluLysArgGlu

2290 2310 2330
ACAGGCAAAGcCATGGTGCCCCACAGCTCAATGTTCATCTTCAGCACCACCAACCCGATC
ThrGlyLysAlaMetValProHisSerSerMetPheIlePheSerThrThrAsnProIle

2350 2370 2390
CGGAGGGCCTGCCACTACATCGTGAACCTGCGCTACTTTGAGATGTGCATCCTCCTGGTG
ArgArgAlaCysHisTyrIleValAsnLeuArgTyrPheGluMetCysIleLeuLeuVal

p1247-5.1.2.1.1, cont.

2410 2430 2450
ATTGCAGCCAGCAGCATCGCCCTGGCgGCAGAGGACCCCGtCcTGACCAACTCGGAGCGC
IleAlaAlaSerSerIleAlaLeuAlaAlaGluAspProValLeuThrAsnSerGluArg

2470 2490 2510
AACAAAGTcCTGAGGTATTTTGACTATGTGTTCACGgGCGAGTTCACCTTTGAGATGGTT
AsnLysValLeuArgTyrPheAspTyrValPheThrGlyGluPheThrPheGluMetVal

2530 2550 2570
ATAaAGATGATAGACCAAGGCTTGATCCTGCAGGATGGGTcCTACTTCCGAGACTTGTGG
IleLysMetIleAspGlnGlyLeuIleLeuGlnAspGlySerTyrPheArgAspLeuTrp

2590 2610 2630
AACATCCTGGACTTTGTGGTGGTCGTTGGCGCATTGGTGGCCTTTGCTCTGGCGAACGCT
AsnIleLeuAspPheValValValValGlyAlaLeuValAlaPheAlaLeuAlaAsnAla

2650 2670 2690
TTGGGAACCAACAAAGGACGGGACATCAAGACCATCAAGTCTCTGCGGGTGCTCCGAGTT
LeuGlyThrAsnLysGlyArgAspIleLysThrIleLysSerLeuArgValLeuArgVal

2710 2730 2750
CTAAGGCCACTGAAAACCATCAAGCGCTTGCCCAAGCTCAAGGCCGTCTTCGACTGCGTA
LeuArgProLeuLysThrIleLysArgLeuProLysLeuLysAlaValPheAspCysVal

2770 2790 2810
GTGACCTCCTTGAAGAATGTCTTCAACATACTCATTGTGTACAAGCTCTTCATGTTCATC
ValThrSerLeuLysAsnValPheAsnIleLeuIleValTyrLysLeuPheMetPheIle

p1247-5.1.2.1.1, cont.

2830 2850 2870
TTTGCTGTCATCGCAGTTCAGCTCTTCAAGGGAAAGTTCTTTTATTGCACGGACAGTTCC
PheAlaValIleAlaValGlnLeuPheLysGlyLysPhePheTyrCysThrAspSerSer

2890 2910 2930
AAGGACACAGAGAAGGAGTGCATAGGCAACTATGTAGATCATGAGAAAAACAAGATGGAG
LysAspThrGluLysGluCysIleGlyAsnTyrValAspHisGluLysAsnLysMetGlu

2950 2970 2990
GTGAAGGGCCGGGAATGGAAGCGCCATGAATTCCACTACGACAACATTATCTGGGCCCTG
ValLysGlyArgGluTrpLysArgHisGluPheHisTyrAspAsnIleIleTrpAlaLeu

3010 3030 3050
CTGACCCTCTTCACCGTCTCCACAGGGGAAGGATGGCCTCAAGTTCTGCAGCACTCTGTA
LeuThrLeuPheThrValSerThrGlyGluGlyTrpProGlnValLeuGlnHisSerVal

3070 3090 3110
GATGTGACAGAGGAAGACCGAGGCCCAAGCCGCAGCAACCGCATGGAGATGTCTATCTTT
AspValThrGluGluAspArgGlyProSerArgSerAsnArgMetGluMetSerIlePhe

3130 3150 3170
TATGTAGTCTACTTTGTGGTCTTCCCCTTCTTCTTTGTCAATATCTTTGTGGCTCTCATC
TyrValValTyrPheValValPheProPhePhePheValAsnIlePheValAlaLeuIle

3190 3210 3230
ATCATCACCTTCCAGGAGCAAGGGGATAAGATGATGGAGGAGTGCAGCCTGGAGAAGAAT
IleIleThrPheGlnGluGlnGlyAspLysMetMetGluGluCysSerLeuGluLysAsn

p1247-5.1.2.1.1, cont.

3250 3270 3290
GAGAGGGCGTGCATCGACTTCGCCATCAGCGCCAAACCTCTCACCCGCTACATGTTGCAG
GluArgAlaCysIleAspPheAlaIleSerAlaLysProLeuThrArgTyrMetLeuGln

3310 3330 3350
AACAGACACACCTTCCAGTACCGCGTGTGGCACTTTGTGGTGTCTCCGTCCTTTGAGTAC
AsnArgHisThrPheGlnTyrArgValTrpHisPheValValSerProSerPheGluTyr

3370 3390 3410
ACCATTATGGCCATGATCGCCTTGAATACTGTTGTGCTGATGATGAAGTATTATTCTGCT
ThrIleMetAlaMetIleAlaLeuAsnThrValValLeuMetMetLysTyrTyrSerAla

3430 3450 3470
CCCTGTACCTATGAGCTGGCCCTGAAGTACCTGAATATCGCCTTCACCATGGTGTTTTCC
ProCysThrTyrGluLeuAlaLeuLysTyrLeuAsnIleAlaPheThrMetValPheSer

3490 3510 3530
CTGGAATGTGTCCTGAAGGTCATCGCTTTTGGCTTTTTGAACTATTTCCGAGACACCTGG
LeuGluCysValLeuLysValIleAlaPheGlyPheLeuAsnTyrPheArgAspThrTrp

3550 3570 3590
AATATCTTTGACTTCATCACCGTGATTGGCAGTATCACAGAAATTATCCTGACAGACAGC
AsnIlePheAspPheIleThrValIleGlySerIleThrGluIleIleLeuThrAspSer

3610 3630 3650
AAGCTGGTGAACACCAGTGGCTTCAATATGAGCTTTCTGAAGCTCTTCCGAGCTGCCCGC
LysLeuValAsnThrSerGlyPheAsnMetSerPheLeuLysLeuPheArgAlaAlaArg

p1247-5.1.2.1.1, cont.

3670 3690 3710
CTCATAAAGCTCCTGCGTCAGGGCTATACCATACGCATTTTGCTGTGGACCTTTGTGCAG
LeuIleLysLeuLeuArgGlnGlyTyrThrIleArgIleLeuLeuTrpThrPheValGln

3730 3750 3770
TCCTTTAAGGCCCTCCCTTATGTCTGCCTTTTAATTGCCATGCTTTTCTTCATTTATGCC
SerPheLysAlaLeuProTyrValCysLeuLeuIleAlaMetLeuPhePheIleTyrAla

3790 3810 3830
ATCATTGGGATGCAGGTATTTGGAAACATAAAATTAGACGAGGAGAGTCACATCAACCGG
IleIleGlyMetGlnValPheGlyAsnIleLysLeuAspGluGluSerHisIleAsnArg

3850 3870 3890
CACAACAACTTCCGGAGTTTCTTTGGGTCCCTAATGCTACTCTTCAGGAGTGCCACAGGT
HisAsnAsnPheArgSerPhePheGlySerLeuMetLeuLeuPheArgSerAlaThrGly

3910 3930 3950
GAGGCCTGGCAGGAGATTATGCTGTCATGCCTTGGGGAGAAGGGCTGTGAGCCTGACACC
GluAlaTrpGlnGluIleMetLeuSerCysLeuGlyGluLysGlyCysGluProAspThr

3970 3990 4010
ACCGCACCATCAGGGCAGAACGAGAACGAACGCTGCGGCACCGATCTGGCCTACGTGTAC
ThrAlaProSerGlyGlnAsnGluAsnGluArgCysGlyThrAspLeuAlaTyrValTyr

4030 4050 4070
TTTGTCTCCTTCATCTTCTTCTGCTCCTTCTTGATGCTCAACCTGTTTGTGGCCGTCATC
PheValSerPheIlePhePheCysSerPheLeuMetLeuAsnLeuPheValAlaValIle

1247-5.1.2.1.1, cont.

4090 4110 4130
. . . . . .
ATGGACAACTTTGAGTACCTGACTCGGGACTCCTCCATCCTGGGGCCTCACCACTTGGAC
MetAspAsnPheGluTyrLeuThrArgAspSerSerIleLeuGlyProHisHisLeuAsp

4150 4170 4190
. . . . . .
GAGTTTGTCCGCGTCTGGGCAGAATATGACCGAGCAGCATGGTGCGTAGGCCCCTCGGCC
GluPheValArgValTrpAlaGluTyrAspArgAlaAlaTrpCysValGlyProSerAla

4210 4230 4250
. . . . . .
CCGCCAGCGAGCGGGGCCCAGAGCAAAGGTCTCTGGAGTTCCCAGGGAAGAGGCTGGAAT
ProProAlaSerGlyAlaGlnSerLysGlyLeuTrpSerSerGlnGlyArgGlyTrpAsn

4270 4290 4310
. . . . . .
TGGAGCCACCCAAATGCCTGCCTGTTACAGAAGGAAAGGAGATTCCTCTTGATTGTGGCC
TrpSerHisProAsnAlaCysLeuLeuGlnLysGluArgArgPheLeuLeuIleValAla

4330 4350 4370
. . . . . .
CATAGAAGAGGCTCTGGTATCAAGCCAGTCACCAAGGACTTCTGTATCCTCCTTTCCCCT
HisArgArgGlySerGlyIleLysProValThrLysAspPheCysIleLeuLeuSerPro

4390 4410 4430
. . . . . .
CTCTTTCCTTCTGTGACAGGGTTTTCCCTTTGTGGCTGGTCTCATCATCCAGTCCTCAC
LeuPheProSerValThrGlyPheSerLeuCysGlyLeuValSerSerSerSerProHis

4450 4470 4490
. . . . . .
CTCAGATTATTTGGGCTCAGTCCCAAGAGAACTTTCCAATAATCTTTTTCTGCCAATGGT
LeuArgLeuPheGlyLeuSerProLysArgThrPheGlnEnd

p1247-5.1.2.1.1, cont.

4510 4530 4550
TAGTGGACTCCTTTGAGTTATCAGGGGGTTTGACAAGGGTTTTTGGCCAGCTGGACATCT

4570 4590 4610
GGCCACCATGATCAGTGACTGTGTTTTCTCTTTCTCGTTCCTCTCAACAGATACAGTTGG

4630 4650 4670
ATATAACTCTGAAATTATTTTTTAAAAACCAAACAAGTTGTTCTTCCAACTGTTCCCTTA

4690 4710 4730
AATATTTGTGGATTTTTATTACATGGAACTAAATTAAATGGTAGGAATTGGTGAAGATAA

4750 4770 4790
CTTCTTTACTGTCTCTTTTGCTGTTTAGTAGTTCTGAAGTGATAGCGTGCCTTCTAGGCT

4810 4830 4850
CTAGAGCAGGGATTGGCAAACTACATCCCATGGGCAAAATTCAGCCCATTGTCTGTTTTT

4870 4890 4910
GCAAATAAAGTTTGGTCcGTCTTGCGCCGGTCCAAGAATTTCACCTCTAGCGGCGCAA

### Sequenzprotokoll:

- Sequenz Nr.:: 2798013
- Int. Code:: p1247-9.1.1.2 entire clone
- Länge:: 811 bp
- Typ:: DNA + Aminosäure

10                                      30                                      50

GGTCAACTCCACCTACTTCGAGTACCTGATGTTCGTCCTCATCCTGCTCAACACCATCTGC

ValAsnSerThrTyrPheGluTyrLeuMetPheValLeuIleLeuLeuAsnThrIleCys

70                                      90                                      110

CTGGCCATGCAGCACTACGGCCAGAGCTGCCTGTTCAAAATCGCCATGAACATCCTCAAC

LeuAlaMetGlnHisTyrGlyGlnSerCysLeuPheLysIleAlaMetAsnIleLeuAsn

130                                     150                                     170

ATGCTCTTCACTGGCCTCTTCACCGTGGAGATGATCCTGAAGCTCATTGCCTTCAAACCC

MetLeuPheThrGlyLeuPheThrValGluMetIleLeuLysLeuIleAlaPheLysPro

190                                     210                                     230

AAGCACTATTTCTGTGATGCATGGAATACATTTGACnCCTTGATTGTTGTGGGTAGCATT

LysHisTyrPheCysAspAlaTrpAsnThrPheAspXxxLeuIleValValGlySerIle

250                                     270                                     290

GTTGATATAGCAATCACCGAGGTAAACCCAGCTGAACATACCCAATGCTCTCCCTCTATG

ValAspIleAlaIleThrGluValAsnProAlaGluHisThrGlnCysSerProSerMet

310                                     330                                     350

AAcgcAGAGGAAAACTCCCGCATCTCCATCACCTTCTTCCGCCTGTTCCGGGTCATGCGT

AsnAlaGluGluAsnSerArgIleSerIleThrPhePheArgLeuPheArgValMetArg

370                                     390                                     410

CTGGTGAAGCTGCTGAGCCGTGGGGAGGGCATCCGGACGCTGCTGTGGACCTTCATCAAG

LeuValLysLeuLeuSerArgGlyGluGlyIleArgThrLeuLeuTrpThrPheIleLys

### Sequenzprotokoll:

- Sequenz Nr.:: 27980/3
- Int. Code:: p1247-9.1.1.2 entire clone
- Länge:: 811 bp
- Typ:: DNA + Aminosäure

430 450 470
TCCTTCCAGGCCCTGCCCTATGTGGCCCTCCTGATCGTGATGCTGTTCTTCATCTACGCG
SerPheGlnAlaLeuProTyrValAlaLeuLeuIleValMetLeuPhePheIleTyrAla

490 510 530
GtGATCGGGATGCAGGTGTTTGGGAAAATTGCCCTGAATGATACCACAGAGATCAACCGG
ValIleGlyMetGlnValPheGlyLysIleAlaLeuAsnAspThrThrGluIleAsnArg

550 570 590
AACAACAACTTTCAGACCTTCCCCCAGGCCGTGCTGCTCCTCTTCAGGTGTGCCACCGGG
AsnAsnAsnPheGlnThrPheProGlnAlaValLeuLeuLeuPheArgCysAlaThrGly

610 630 650
GAGGCCTGGCAGGACATCATGCTGGCCTGCATGCCAGGCAAGAAGTGTGCCCCAGAGTCC
GluAlaTrpGlnAspIleMetLeuAlaCysMetProGlyLysLysCysAlaProGluSer

670 690 710
GAGCCCAGCAACAGCACGGAGGGTGAAACACCCTGTGGTAGCAGCTTTGCTGTCTTCTAC
GluProSerAsnSerThrGluGlyGluThrProCysGlySerSerPheAlaValPheTyr

730 750 770
TTCATCAGCTTCTACATGCTCTGTGCCTTCCTGATCATCAACCTCTTTGTAGCTGTCATC
PheIleSerPheTyrMetLeuCysAlaPheLeuIleIleAsnLeuPheValAlaValIle

790 810
ATGGACAACTTTGACTACCTGACAAGGGAC
MetAspAsnPheAspTyrLeuThrArgAsp

### Sequenzprotokoll:

- Sequenz Nr.:: 27980/4
- Int. Code:: p1247.10.1.1.1, entire clone
- Länge:: 1.354 bp
- Typ:: DNA + Aminosäure

10 30 50
GTGGTGTTCCCCTTCTTCTTTGTCAATATCTTTGTGGCCTTGATCATCATCACCTTCCAG
ValValPheProPhePhePheValAsnIlePheValAlaLeuIleIleIleThrPheGln

70 90 110
GAGCAAGGGGACAAGATGATGGAGGAATACAGCCTGGAGAAAAATGAGAGGGCCTGCATT
GluGlnGlyAspLysMetMetGluGluTyrSerLeuGluLysAsnGluArgAlaCysIle

130 150 170
GATTTCGCCATCAGCGCCAAGCCGCTGACCCGACACATGCCGCAGAACAAGCAGAGCTTC
AspPheAlaIleSerAlaLysProLeuThrArgHisMetProGlnAsnLysGlnSerPhe

190 210 230
CAGTACCGCATGTGGCAGTTCGTGGTGTCTCCGCCTTTCGAGTACACGATCATGGCCATG
GlnTyrArgMetTrpGlnPheValValSerProProPheGluTyrThrIleMetAlaMet

250 270 290
ATCGCCCTCAACACCATCGTGCTTATGATGAAGTTCTATGGGGCTTCTGTTGCTTATGAA
IleAlaLeuAsnThrIleValLeuMetMetLysPheTyrGlyAlaSerValAlaTyrGlu

310 330 350
AATGCCCTGCGGGTGTTCAACATCGTCTTCACCTCCCTCTTCTCTCTGGAATGTGTGCTG
AsnAlaLeuArgValPheAsnIleValPheThrSerLeuPheSerLeuGluCysValLeu

370 390 410
AAAGTCATGGCTTTTGGGATTCTGAATTATTTCCGCGATGCCTGGAACATCTTCGACTTT
LysValMetAlaPheGlyIleLeuAsnTyrPheArgAspAlaTrpAsnIlePheAspPhe

### Sequenzprotokoll:

- Sequenz Nr.:: 27980/4
- Int. Code:: p1247-10.1.1.1, entire clone
- Länge:: 1.354 bp
- Typ:: DNA + Aminosäure

430 450 470
GTGACTGTTCTGGGCAGCATCACCGATATCCTCGTGACTGAGTTTGGGAATAACTTCATC
ValThrValLeuGlySerIleThrAspIleLeuValThrGluPheGlyAsnAsnPheIle

490 510 530
AACCTGAGCTTTCTCCGCCTCTTCCGAGCTGCCCGGCTCATCAAACTTCTCCGTCAGGGT
AsnLeuSerPheLeuArgLeuPheArgAlaAlaArgLeuIleLysLeuLeuArgGlnGly

550 570 590
TACACCATCCGcATTCTTCTCTGGACCTTTGTGCAGTCCTTCAAGGCCCTGCCTTATGTC
TyrThrIleArgIleLeuLeuTrpThrPheValGlnSerPheLysAlaLeuProTyrVal

610 630 650
TGTCTGCTGATCGCCATGCTCTTCTTCATCTATGCCATCATTGGGATACAGGTGTTTGGT
CysLeuLeuIleAlaMetLeuPhePheIleTyrAlaIleIleGlyIleGlnValPheGly

670 690 710
AACATTGGCATCGACGTGGAGGACGAGGACAGTGATGAAGATGAGTTCCAAATCACTGAG
AsnIleGlyIleAspValGluAspGluAspSerAspGluAspGluPheGlnIleThrGlu

730 750 770
CACAATAACTTCCGGACCTTCTTCCAGGCCCTCATGCTTCTCTTCCGGAGTGCCACCGGG
HisAsnAsnPheArgThrPhePheGlnAlaLeuMetLeuLeuPheArgSerAlaThrGly

790 810 830
GAAGCTTGGCACAACATCATGCTTTCCTGCCTCAGCGGGAAACCGTGTGATAAGAACTCT
GluAlaTrpHisAsnIleMetLeuSerCysLeuSerGlyLysProCysAspLysAsnSer

### Sequenzprotokoll:

- Sequenz Nr.:: 27980/4
- Int. Code:: p1247-10.1.1.1, entire clone
- Länge:: 1.354 bp
- Typ:: DNA + Aminosäure

850 870 890
. . . . . .
GGCATCCTGACTCGAGAGTGTGGCAATGAATTTGCTTATTTTACTTTGTTTCCTTCATC
GlyIleLeuThrArgGluCysGlyAsnGluPheAlaTyrPheTyrPheValSerPheIle

910 930 950
. . . . . .
TTCCTCTGCTCGTTTCTGATGCTGAATCTCTTTGTCGCCGTCATCATGGACAACTTTGAG
PheLeuCysSerPheLeuMetLeuAsnLeuPheValAlaValIleMetAspAsnPheGlu

970 990 1010
. . . . . .
TACCTCACCCGAGACTCCTCCATCCTGGGCCCCCACCACCTGGATGAGTACGTGCGTGTC
TyrLeuThrArgAspSerSerIleLeuGlyProHisHisLeuAspGluTyrValArgVal

1030 1050 1070
. . . . . .
TGGGCCGAGTATGACCCCGCAGCTTGGGGCCGCATGCCTTACCTGGACATGTATCAGATG
TrpAlaGluTyrAspProAlaAlaTrpGlyArgMetProTyrLeuAspMetTyrGlnMet

1090 1110 1130
. . . . . .
CTGAGACACATGTCTCCGCCCCTGGGTCTGGGGAAGAAGTGTCCGGCCAGAGTGGCTTAC
LeuArgHisMetSerProProLeuGlyLeuGlyLysLysCysProAlaArgValAlaTyr

1150 1170 1190
. . . . . .
AAGCTTCTTCTGCGGATGGACCTGCCCGTCGCAGATGACAACACCGTCCACTTCAATTCC
LysLeuLeuLeuArgMetAspLeuProValAlaAspAspAsnThrValHisPheAsnSer

1210 1230 1250
. . . . . .
ACCCTCATGGCTCTGATCCGCACAGCCCTGgACATCAAGATTGCCAAGGNNGGAGCCGAC
ThrLeuMetAlaLeuIleArgThrAlaLeuAspIleLysIleAlaLysXxxGlyAlaAsp

### Sequenzprotokoll:

- Sequenz Nr.:: 27980/4
- Int. Code:: p1247-10.1.1.1, entire clone
- Länge:: 1.354 bp
- Typ:: DNA + Aminosäure

1270                    1290                    1310
AAACAGCAGATGGACGCTGAGCTGCGGAAGGAGATGATGGCGATTTGGCCCAATCTGTCC
LysGlnGlnMetAspAlaGluLeuArgLysGluMetMetAlaIleTrpProAsnLeuSer

1330                    1350
CAGAAGACGCTAGACCTGCTGGTCACACCTCACA
GlnLysThrLeuAspLeuLeuValThrProHis

### Sequenzprotokoll:

- Sequenz Nr.:: 27980/5
- Int. Code:: p1247-14.1.1.1, complete sequence
- Länge:: 5438 bp
- Typ:: DNA + Aminosäure

10 30 50
GGACTGCAGGGGTCAGTATTTGGATTATGAGAAGGAGGAAGTGGAAGCTCAGCCCAGGCAG
AspCysArgGlyGlnTyrLeuAspTyrGluLysGluGluValGluAlaGlnProArgGln

70 90 110
TGGAAGAAATACGACTTTCACTACGACAATGTGCTCTGGGCTCTGCTGACGCTGTTCACA
TrpLysLysTyrAspPheHisTyrAspAsnValLeuTrpAlaLeuLeuThrLeuPheThr

130 150 170
GTGTCCACGGGAGAAGGCTGGCCCATGGTGCTGAAACACTCCGTGGATGCCACCTATGAG
ValSerThrGlyGluGlyTrpProMetValLeuLysHisSerValAspAlaThrTyrGlu

190 210 230
GAGCAGGGTCCAAGCCCTGGGTACCGCATGGAGCTGTCCATCTTCTACGTGGTCTACTTT
GluGlnGlyProSerProGlyTyrArgMetGluLeuSerIlePheTyrValValTyrPhe

250 270 290
GTGGTCTTTCCCTTCTTCTTCGTCAACATCTTTGTGGCTTTGATCATCATCACCTTCCAG
ValValPheProPhePhePheValAsnIlePheValAlaLeuIleIleIleThrPheGln

310 330 350
GAGCAGGGGGACAAGGTGATGTCTGAATGCAGCCTGGAGAAGAACGAGAGGGCTTGCATT
GluGlnGlyAspLysValMetSerGluCysSerLeuGluLysAsnGluArgAlaCysIle

### Sequenzprotokoll:

- Sequenz Nr.:: 27980/5
- Int Code:: p1247-14.1.1.1, complete sequence
- Länge:: 5438 bp
- Typ:: DNA + Aminosäure

p1247-14.1.1.1, cont.

370 390 410
GACTTCGCCATCAGCGCCAAACCCCTGACACGGTACATGCCCCAAAACCGGCAGTCGTTC
AspPheAlaIleSerAlaLysProLeuThrArgTyrMetProGlnAsnArgGlnSerPhe

430 450 470
CAGTATAAGACGTGGACATTTGTGGTCtcCCCGCCCTTTGAATACTTCATCATGGCCATG
GlnTyrLysThrTrpThrPheValValSerProProPheGluTyrPheIleMetAlaMet

490 510 530
ATAGCCCTCAACACTGTGGTGCTGATGATGAAGTTCTACGATGCACCCTATGAGTACGAG
IleAlaLeuAsnThrValValLeuMetMetLysPheTyrAspAlaProTyrGluTyrGlu

550 570 590
CTGATGCTGAAATGCCTGAACATCGTGTTCACATCCATGTTCTCCATGGAATGCGTGCTG
LeuMetLeuLysCysLeuAsnIleValPheThrSerMetPheSerMetGluCysValLeu

610 630 650
AAGATCATCGCCTTTGGGGTGCTGAACTATTTCAGAGATGCCTGGAATGTCTTTGACTTT
LysIleIleAlaPheGlyValLeuAsnTyrPheArgAspAlaTrpAsnValPheAspPhe

670 690 710
GTCACTGTGTTGGGAAGTATTACTGATATTTTAGTAACAGAGATTGCGAACAATTTCATC
ValThrValLeuGlySerIleThrAspIleLeuValThrGluIleAlaAsnAsnPheIle

730 750 770
AACCTCAGCTTCCTCCGCCTCTTTCGAGCTGCGCGGCTGATCAAGCTGCTCCGCCAGGGC
AsnLeuSerPheLeuArgLeuPheArgAlaAlaArgLeuIleLysLeuLeuArgGlnGly

### Sequenzprotokoll:

- Sequenz Nr.:: 27980/5
- Int. Code:: p1247-14.1.1.1, complete sequence
- Länge:: 5438 bp
- Typ:: DNA + Aminosäure

p1247-14.1.1.1, cont.

790 810 830
. . . . . .
TACACCATCCGCATCCTGCTGTGGACCTTTGTCCAGTCCTTCAAGGCCCTGCCCTACGTG
TyrThrIleArgIleLeuLeuTrpThrPheValGlnSerPheLysAlaLeuProTyrVal

850 870 890
. . . . . .
TGTCTGCTCATTGCCATGCTGTTCTTCATCTACGCCATCATCGGCATGCAGGTGTTTGGG
CysLeuLeuIleAlaMetLeuPhePheIleTyrAlaIleIleGlyMetGlnValPheGly

910 930 950
. . . . . .
AATATTGCCCTGGATGATGACACCAGCATCAACCGCCACAACAACTTCCGGACGTTTTTG
AsnIleAlaLeuAspAspAspThrSerIleAsnArgHisAsnAsnPheArgThrPheLeu

970 990 1010
. . . . . .
CAAGCCCTGATGCTGCTGTTCAGGAGCGCCACGGGGGAGGCCTGGCACGAGTCATGCTGT
GlnAlaLeuMetLeuLeuPheArgSerAlaThrGlyGluAlaTrpHisGluSerCysCys

1030 1050 1070
. . . . . .
CCTGCCTGAGCAACCAGGCCTGTGATGAGCAGGCCAATGCCACCGAGTGTGGAAGTGACT
ProAlaEnd

1090 1110 1130
. . . . . .
TTGCCTACTTCTACTTCGTCTCCTTCATCTTCCTGTGCTCCTTTCTGATGTTGAACCTCT

1150 1170 1190
. . . . . .
TTGTGGCTGTGATCATGGACAATTTTGAGTACCTCACGCGGGACTCTTCCATCCTAGGTC

### Sequenzprotokoll:

- Sequenz Nr.:: 27980/5
- Int. Code:: p1247-14.1.1.1, complete sequence
- Länge:: 5438 bp
- Typ:: DNA + Aminosäure

p1247-14.1.1.1, cont.

1210 1230 1250
CTCACCACTTGGATGAGTTCATCCGGGTCTGGGCTGAATACGACCCGGCTGCGTGTGGGC

1270 1290 1310
GCATCAGTTACAATGACATGTTTGAGATGCTGAAACACATGTCCCCGCCTCTGGGGCTGG

1330 1350 1370
GGAAGAAATGCCCTGCTCGAGTTGCTTACAAGCGCCTGGTTCGCATGAACATGCCCATCT

1390 1410 1430
CCAACGAGGACATGACTGTTCACTTCACGTCCACGCTGATGGCCCTCATCCGGACGGCAC

1450 1470 1490
TGGAGATCAAGCTGGCCCCAGCTGGGACAAAGCAGCATCAGTGTGACGCGGAGTTGAGGA

1510 1530 1550
AGGAGATTTCCGTTGTGTGGGCCAATCTGCCCCAGAAGACTTTGGACTTGCTGGTACCAC

1570 1590 1610
CCCATAAGCCTGATGAGATGACAGTGGGGAAGGTTTATGCAGCTCTGATGATATTCGACT

1630 1650 1670
TCTACAAGCAGAACAAAACCACCAGAGACCAGATGCAGCAGGCTCCTGGAGGCCTCTCCC

### Sequenzprotokoll:

- Sequenz Nr.:: 27980/5
- Int. Code:: p1247-14.1.1.1, complete sequence
- Länge:: 5438 bp
- Typ:: DNA + Aminosäure

p1247-14.1.1.1, cont.

1690 1710 1730
AGATGGGTCCTGTGTCCCTGTTCCACCCTCTGAAGGCCACCCTGGAGCAGACACAGCCGG

1750 1770 1790
CTGTGCTCCGAGGAGCCCGGGTTTTCCTTCGACAGAAGAGTTCCACCTCCCTCAGCAATG

1810 1830 1850
GCGGGGCCATACAAAACCAAGAGAGTGGCATCAAAGAGTCTGTCTCCTGGGGCACTCAAA

1870 1890 1910
GGACCCAGGAtgCACCCCATGAGGCCAGGCCACCCCTGGAGCGTGGCCACTCCACAGAGA

1930 1950 1970
TCCCTGTGGGGCGGTCAGGAGCACTGGCTGTGGACGTTCAGATGCAGAGCATAACCCGGA

1990 2010 2030
GGGgcCCTGATGGGGAGCCCCAGCCTGGGCTGGAGAGCCAGGGTCGAGCGGCCTCCATGC

2050 2070 2090
CCCGCCTTGCGGCCGAGACTCAGCCCGTCACAGATGCCAGCCCCATGAAGCGCTCCATCT

2110 2130 2150
CCACGCTGGCCCAGCGGCCCCGTGGGACTCATCTTTGCAGCACCACCCCGGACCGCCCAC

### Sequenzprotokoll:

- Sequenz Nr.:: 27980/5
- Int. Code:: p1247-14.1.1.1, complete sequence
- Länge:: 5438 bp
- Typ:: DNA + Aminosäure

p1247-14.1.1.1, cont.

2170 2190 2210
CCCCTAGCCAGGCGTCGTCGCACCACCACCACCACCGCTGCCACCGCCGCAGGGACAGGA

2230 2250 2270
AGCAGAGGTCCCTGGAGAAGGGGCCCAGCCTGTCTGCCGATATGGATGGCGCACCAAGCA

2290 2310 2330
GTGCTGTGGGGCCGGGGCTGCCCCCGGGAGAGGGGCCTACAGGCTGCCGGCGGGAACGAG

2350 2370 2390
AGCGCCGGCAGGAGCGGAGCCGGTCCCAGGAGCGGAGGCAGCCCTCATCCTCCTCCTCGG

2410 2430 2450
AGAAGCAGCGCTTCTACTCCTGCGACCGCTTTGGGGGCCGTGAGCCCCCGAAGCCCAAGC

2470 2490 2510
CCTCCCTCAGCAGCCACCCAACGTCGCCAACAGCTGGCCAGGAGCCGGGACCCCACCCAC

2530 2550 2570
AGGGCAGTGGTTCCGTGAATGGGAGCCCCTTGCTGTCAACATCTGGTGCTAGCACCCCCG

2590 2610 2630
GCCGCGGTGGGCGGAGGCAGCTCCCCCAGACGCCCCTGACTCCCCGCCCCAGCATCACCT

### Sequenzprotokoll:

- Sequenz Nr.:: 27980/5
- Int. Code:: p1247-14.1.1.1, complete sequence
- Länge:: 5438 bp
- Typ:: DNA + Aminosäure

p1247-14.1.1.1, cont.

2650 2670 2690
ACAAGACGGCCAACTCCTCACCCATCCACTTCGCCGGGGCTCAGACCAGCCTCCCTGCCT

2710 2730 2750
TCTCCCCAGGCCGGCTCAGCCGTGGGCTTTCCGAACACAACGCCCTGCTGCAGAGAGACC

2770 2790 2810
CCCTCAGCCAGCCCCTGGCCCCTGGCTCTCGAATTGGCTCTGACCCTTACCTGGGGCAGC

2830 2850 2870
GTCTGGACAGTGAGGCCTCTGTCCACGCCCTGCCTGAGGACACGCTCACTTTCGAGGAGG

2890 2910 2930
CTGTGGCCACCAACTCGGGCCGCTCCTCCAGGACTTCCTACGTGTCCTCCCTGACCTCCC

2950 2970 2990
AGTCTCACCCTCTCCGCCGCGTGCCCAACGGTTACCACTGCACCCTGGGACTCAGCTCGG

3010 3030 3050
GTGGCCGAGCACGGCACAGCTACCACCACCCTGACCAAGACCACTGGTGCTAGCTGCACC

3070 3090 3110
GTGACCGCTCAGACGCCTGCATGCAGCAGGCGTGTGTTCCAGTGGATGAGTTTTATCATC

### Sequenzprotokoll:

- Sequenz Nr.:: 27980/5
- Int. Code:: p1247-14.1.1.1, complete sequence
- Länge:: 5438 bp
- Typ:: DNA + Aminosäure

p1247-14.1.1.1, cont.

3130 3150 3170
CACACGGGGCAGTCGGCCcTCGGGGAGGCCTTGCCCACCTTGGTGAGGCTCCTGTGGCC

3190 3210 3230
CCTCCCTCCCCCTCCTCCCCTCTTTACTCTAGACGACGAATAAAGCCCTGTTAGAGGAT

3250 3270 3290
GCGGCTCTCTCTGTCCCCTTCCTGTCCTGCCTTCCTGGGtCTCGTACCACACACCAGACC

3310 3330 3350
CTAAACCGCAGGCTGCTGTGTGTGGCTGAGAAGGACCCAGGAGTCCAAATCCCGTGTCCT

3370 3390 3410
GGGACTCAGCATCCAGCATGGGTGCTTGGAGCCGTTGTGAGGAGCTCTGCGTCCTGTGGG

3430 3450 3470
GAGCACCCTTCACGTGGCCGTGCGGCACAGAGAAGCAGGGCCCACCTGAAAGTGCGCCGA

3490 3510 3530
GACCTCGGGACGGAGGGGATGGGGAGGGGACACAGTCGTGGCTTGTGCAGCCCGCCAGT

3550 3570 3590
GTCAGCGAATGCTCACTCAGGCAAGCTCTGTCCTCCCTGGACACCGTCAGCCCCACAGGA

### Sequenzprotokoll:

- Sequenz Nr.:: 27980/5
- Int. Code:: p1247-14.1.1.1, complete sequence
- Länge:: 5438bp bp
- Typ:: DNA + Aminosäure

p1247-14.1.1.1, cont.

3610 3630 3650
ACCGAGCTGGGAAGTGTTCTTGCTGTGGTTGTGATTTTTAATTGCAACACCTCTCATTCT

3670 3690 3710
TGTCACTTCTATATACGTGATGTAGAAAAAATGGAAAACCAGAAAAATGGGGAAGGAAAT

3730 3750 3770
GTTCACATAACTTTAAAAAATCAAACCTGTGAAAGAAAGATGTCAGCTTTTTGCCACGTG

3790 3810 3830
TCTTTGTGGCTTATGCGAGGAgACTCCCTGTGCAGCCCTGTCCGGTCCAGGTGGACGTAG

3850 3870 3890
ATGGCCCCTGGCTCTGCTGCTCTTGACCAAGTGCCTGACCGCCAGGCCCTCACACCCAGG

3910 3930 3950
CTCCTGGGCACTGTGGTGTGAGGCGAGGCCTCGGGATCCATCACCGCAGGATGTTGTGAA

3970 3990 4010
AAGTACTCGCGATGGCAGCCAGGTAGCAAGCCCTTGCCAGTGGAGAGCACTGGATGTCAT

4030 4050 4070
GGTGGCAAACAAGGCAGCCATTTGCTGTCCTCCTCCCACGAGTGGAAGGGGTTTCCAAGG

### Sequenzprotokoll:

- Sequenz Nr.:: 27980/5
- Int. Code:: p1247-14.1.1.1, complete sequence
- Länge:: 5438 bp
- Typ:: DNA + Aminosäure

p1247-14.1.1.1, cont.

4090 4110 4130
AAGCCACAGGGCAGCTGACCACGTGCTTGTGTGAGGCATTTTCAGTCTGTTCTGCATATG

4150 4170 4190
ATTCTCAGGGCACACTCTGTGGTATGTGAAATAGGTTTCCTTCCACATACAGCAGAAGAG

4210 4230 4250
AGGCAAAGGCTGGTAGGAAGGAGGAAGACATTGGCTGGGGGCTTGGATGTGGGGCCGTCA

4270 4290 4310
GGGCAGGAGGGAGGAaGCCCCAGCTGGAATGAAACTCAGAGCAAGTGACCGAGGGAGGAC

4330 4350 4370
ACGGCTCCTGCCACTGAGGCCGGGCACCTGATGCCCAGCACTGTCCTGGCGCCAGACACA

4390 4410 4430
GGGAGCAGGCAGTCAAGTGAGGTCTGACCCCCATGGCCACgCTCAGGAGAGAAAGACCAT

4450 4470 4490
GCTCAGGACACTGTCCAAGGTGCACAAGATGCTGGGAGGTcCcTTGTTTGGTGAAGAAAG

4510 4530 4550
GGAGCATTTAGAGCAGTTGATGGTGGTGTCCTCCGTGTTCTGAAATTCCAGATGATCT

### Sequenzprotokoll:

- Sequenz Nr.:: 27980/5
- Int. Code:: p1247-14.1.1.1, complete sequence
- Länge:: 5438 bp
- Typ:: DNA + Aminosäure

p1247-14.1.1.1, cont.

4570 4590 4610
GTGTTGGATTTTTGgCTTCTACCCCATgATTCTCCTCAAAGAAAttGTGTGTGATGTGTG

4630 4650 4670
TGTGTGTGTGTGTGTGTGTCTGTGTCACAGGAGATGCAGTGCCTGTACAGGTGTGTTC

4690 4710 4730
AGTGTGTGgATGTCATTAACCCATAGGGCTATGCAACAAAAGACACATTTAATAGAAGTA

4750 4770 4790
AAACACACAAGACCGCTGCCTGGTCTCGGGGTTCAGCATGATTGTGACCAAACCTTTTTA

4810 4830 4850
TAGAATTTCCTTACCTGAAGGCACAACACTCTGAAACTTTAAAGATAACAGAGTATTTTA

4870 4890 4910
TTCCAATAGaATAAACCAGGAATCTCGgACTGTGCATGTGATCACTGTGCTCCTGTTGCA

4930 4950 4970
AAGtAGAAGGATGTGTATTTTGACACTGACGTTTTGTCTCTTGTTCCCCAGCCCCCAGCC

4990 5010 5030
CATGTTATCTTGGGTGTCGAATGTGTCCATTCCATGCAGAACCACAGCCATTTCCCCAGG

### Sequenzprotokoll:

- Sequenz Nr.:: 27980/5
- Int. Code:: p1247-14.1.1.1, complete sequence
- Länge:: 5438 bp
- Typ:: DNA + Aminosäure

p1247-14.1.1.1, cont.

5050          5070          5090
CAGTGTTGGGTCGAGAATCCACTTTTCTAAACCCACACAGCCTAGCTGGCTTGTCTAGAC

5110          5130          5150
TCTTCTAGGCATTGGAATTGATGAAAACTACAGGGAGCGGGgAAAGGAGACATTATGTCT

5170          5190          5210
TGTTTCCTGACTTTGGGTTTTGTTTCTCACTGTGTCTTCTCCGGCTATCATATATGTCCC

5230          5250          5270
CTGAATCTCATAGTGAGCTGCCAAATTTGAAGTGCATCACCCAGTTGTCTGCATCTGGAA

5290          5310          5330
CCAGTCAAGCAGTGGCTGTAGTTTGAACAAGTTATGTGTGCATGTAACATATATACATAT

5350          5370          5390
ATACATATATACAAGTATGTGCATGATAATGTATATCTTCGTACTTTTTGATACAATGTA

5410          5430
TTCATTTGTTAATTTTTAATTATATTTGATATAAATC

35

### Sequenzprotokoll:

- Sequenz Nr.:: 27980/6
- Int. Code:: pR9112-4.1.1.1 entire clone
- Länge:: 1.722 bp
- Typ:: DNA + Aminosäure

10 30 50
GAACGCAGAGGAAAACTCCCGCATCTCCATCACCTTCTTCCGCCTGTTCCGGGTCATGCGT
AsnAlaGluGluAsnSerArgIleSerIleThrPhePheArgLeuPheArgValMetArg

70 90 110
CTGGTGAAGCTGCTGAGCCGTNNNGAGGGCATCCGGACGCTGCTGTGGACCTTCATCAAG
LeuValLysLeuLeuSerArgXxxGluGlyIleArgThrLeuLeuTrpThrPheIleLys

130 150 170
TCCTTCCAGGCCCTGCCCTATgTGGCCCTCCTGATCGTGATGCTGTTCTTCATCTACgCG
SerPheGlnAlaLeuProTyrValAlaLeuLeuIleValMetLeuPhePheIleTyrAla

190 210 230
GTGATCGGGATGCAGGTGTTTGGGAAAATTGCCCTGAATGATACCACAGAGATCAACCGG
ValIleGlyMetGlnValPheGlyLysIleAlaLeuAsnAspThrThrGluIleAsnArg

250 270 290
AACAACAACTTTCAGACCTtccCCCAGGCCGTGCTGCTCCTCTTCAGGTGTGCCACCGGG
AsnAsnAsnPheGlnThrPheProGlnAlaValLeuLeuLeuPheArgCysAlaThrGly

310 330 350
GAGGCCTGGCAGGACATCATGCTGGCCTGCATGCCAGGCAAGAAGTGTgcCCCAGAGTCC
GluAlaTrpGlnAspIleMetLeuAlaCysMetProGlyLysLysCysAlaProGluSer

370 390 410
GAGCCCAGCAACAGCACGGAGGGTGAAACACCCTGTGGTAGCAGCTTTGCTGTCTTCTAC
GluProSerAsnSerThrGluGlyGluThrProCysGlySerSerPheAlaValPheTyr

### Sequenzprotokoll:

- Sequenz Nr.:: 27980/6
- Int. Code:: pR91124.1.1.1 entire clone
- Länge:: 1.722 bp
- Typ:: DNA + Aminosäure

430 450 470

TTCATCAGCTTCTACATGCTCTGTGCCTTCCTGATCATCAACCTCTTTGTAGCTGTCATC

PheIleSerPheTyrMetLeuCysAlaPheLeuIleIleAsnLeuPheValAlaValIle

490 510 530

ATGGACAACTTTGACTACCTGACAAGGGACTGGTCCATCCTTGGTCCCCACCACCTGGAT

MetAspAsnPheAspTyrLeuThrArgAspTrpSerIleLeuGlyProHisHisLeuAsp

550 570 590

GAGTTTAAAAGAATCTGGGCAGAGTATGACCCTGAAGCCAAGGGTCGTATCAAACACCTG

GluPheLysArgIleTrpAlaGluTyrAspProGluAlaLysGlyArgIleLysHisLeu

610 630 650

GATGTGGTGACCCTCCTCCgGCGAATTCAGCCGCCACTAGGTTTTGGGAAGCTGTGCCCT

AspValValThrLeuLeuArgArgIleGlnProProLeuGlyPheGlyLysLeuCysPro

670 690 710

CACCGCGTGGCTTGCAAACGCCTGGTCTCCATGAACATGCCTCTGAACAGCGACGGGACA

HisArgValAlaCysLysArgLeuValSerMetAsnMetProLeuAsnSerAspGlyThr

730 750 770

GTCATGTTCAATGCCACCCTGTTTGCCCTGGTCAGGACGGCCCTGAGGATCAAAACAGAA

ValMetPheAsnAlaThrLeuPheAlaLeuValArgThrAlaLeuArgIleLysThrGlu

790 810 830

GGGAACCTAGAACAAGCCAATGAGGAGCTGCGGGCGATCATCAAGAAGATCTGGAAGCGG

GlyAsnLeuGluGlnAlaAsnGluGluLeuArgAlaIleIleLysLysIleTrpLysArg

### Sequenzprotokoll:

- Sequenz Nr.:: 27980/6
- Int Code:: pR9112-4.1.1.1 entire clone
- Länge:: 1.722 bp
- Typ:: DNA + Aminosäure

850 870 890
ACCAGCAtgAAGCTGCTGGACCAGGTGGTGCCCCCTGCAGGTGATGATGAGGTCACCGTT
ThrSerMetLysLeuLeuAspGlnValValProProAlaGlyAspAspGluValThrVal

910 930 950
GGCAAGTTCTACGCCACGTTCCTGATCCAGGAGTACTTCCGGAAGTTCAAGAAGCGCAAA
GlyLysPheTyrAlaThrPheLeuIleGlnGluTyrPheArgLysPheLysLysArgLys

970 990 1010
GAGCAGGGCCTTGTGGgcAAGCCCTCCCAGAGGAACGCGCTGTCTCTGCAGGCTGGcTTG
GluGlnGlyLeuValGlyLysProSerGlnArgAsnAlaLeuSerLeuGlnAlaGlyLeu

1030 1050 1070
CGCACACTGCATGACATCGGGCCTGAGATCCGACGGGCCATCTCTGGAGATCTCACCGCT
ArgThrLeuHisAspIleGlyProGluIleArgArgAlaIleSerGlyAspLeuThrAla

1090 1110 1130
GAGGAGGAGCTGGACAAGGCCATGAAGGAGGCTGTGTCCGCTGCTTCTGAAGATGACATC
GluGluGluLeuAspLysAlaMetLysGluAlaValSerAlaAlaSerGluAspAspIle

1150 1170 1190
TTCAGGAGGGCCGGTggccTGTTCGGCAACCACGTCAGcTACTACCAAAGCgACGGCCGG
PheArgArgAlaGlyGlyLeuPheGlyAsnHisValSerTyrTyrGlnSerAspGlyArg

1210 1230 1250
ANNGCCTTCcCCCAGACCTTCACCACTCAGNGCCCGCTGCACATCAACAAGGCGGGCAGC
XxxAlaPheProGlnThrPheThrThrGlnXxxProLeuHisIleAsnLysAlaGlySer

### Sequenzprotokoll:

- Sequenz Nr.:: 27980/6
- Int. Code:: pR9113-4.1.1.1 entire clone
- Länge:: 1.722 bp
- Typ:: DNA + Aminosäure

1270 1290 1310
AGCCAGGGCGACACTGAGTCGCNATCCCACGAGAAGCTGGTGGACTCCACCTTCACCCCG
SerGlnGlyAspThrGluSerXxxSerHisGluLysLeuValAspSerThrPheThrPro

1330 1350 1370
AGCAGCTACTCGTCCACCGGCTCCAACGCCAACATCAACAACGCCAACAACACCGCCCTG
SerSerTyrSerSerThrGlySerAsnAlaAsnIleAsnAsnAlaAsnAsnThrAlaLeu

1390 1410 1430
GGTCGCCTCCCTCNCNCCGCCGGCTACCCCAGCACAGTCAGCACTGTGGAGcCACGGGCC
GlyArgLeuProXxxXxxAlaGlyTyrProSerThrValSerThrValGluProArgAla

1450 1470 1490
CCCCTTGTCCCCTGCCATCCGGGTGCAGGAGGTGGNGTGGAAGCTcAGCTCCAACAGGTG
ProLeuValProCysHisProGlyAlaGlyGlyGlyValGluAlaGlnLeuGlnGlnVal

1510 1530 1550
CCACTCCCGGGAGAGCCAGGCAGCCATGGCGGGTCAGGAGGAGACGTCTCAGGATGAGAC
ProLeuProGlyGluProGlySerHisGlyGlySerGlyGlyAspValSerGly *

1570 1590 1610
CTATGAAGTGAAGATGAACCATGACACGGAGGCCTGCAGTGAGCCCAGCCTGCTCTCCAC

1630 1650 1670
AGAGATGCTCTCCTACCAGGATGACGAAAATCGGCAACTGACGCTCCCAGAGGAGGACAA

### Sequenzprotokoll:

- Sequenz Nr.:: 27980/6
- Int. Code:: pR91124.1.1.1 entire clone
- Länge:: 1.722 bp
- Typ:: DNA + Aminosäure

1690          .          1710
        .         .         .         .
GAGGGACATCCGGCAATCTCCGAAGAGGGGTTTCCTCCGCT

### Sequenzprotokoll:

- Sequenz Nr.:: 27980/7
- Int. Code:: pR9112-10.1.1.1. entire clone
- Länge:: 2.049 bp
- Typ:: DNA + Aminosäure

10 30 50
GAGAACAGCAAGTTTGACTTTGACAATGTTCTGGCAGCCATGATGGCCCTCTTCACCGTC
GluAsnSerLysPheAspPheAspAsnValLeuAlaAlaMetMetAlaLeuPheThrVal

70 90 110
TCCACCTTCGAAGGGTGGCCAGAGCTGCTGTACCGCTCCATCGACTCCCACACGGAAGAC
SerThrPheGluGlyTrpProGluLeuLeuTyrArgSerIleAspSerHisThrGluAsp

130 150 170
AAGGgcCCCATCTACAACTACCGTGTGGAGATCTCCATCTTCTTCATCATCTACATCATC
LysGlyProIleTyrAsnTyrArgValGluIleSerIlePhePheIleIleTyrIleIle

190 210 230
ATCATCGCCTTCTTCATGATGAACATCTTCGTGGGCTTCGTCATCGTCACCTTTCAGGAG
IleIleAlaPhePheMetMetAsnIlePheValGlyPheValIleValThrPheGlnGlu

250 270 290
CAGGGGGAGCAGGAGTACAAGAACTGTGAGCTGGACAAGAACCAGCGACAGTGCGTGGAA
GlnGlyGluGlnGluTyrLysAsnCysGluLeuAspLysAsnGlnArgGlnCysValGlu

310 330 350
TACGCCCTCAAGGCCCGGCCCCTGCGGAGGTACATcCCCAAGAACCAGCACCAGTACAAA
TyrAlaLeuLysAlaArgProLeuArgArgTyrIleProLysAsnGlnHisGlnTyrLys

370 390 410
GTGTGGTACGTGGTCAACTCCACCTACTTCGAGTACCTGATGTTCGTCCTCATCCTGCTC
ValTrpTyrValValAsnSerThrTyrPheGluTyrLeuMetPheValLeuIleLeuLeu

### Sequenzprotokoll:

- Sequenz Nr.:: 27980/7
- Int. Code:: pR9112-10.1.1.1. entire clone
- Länge:: 2.049 bp
- Typ:: DNA + Aminosäure

430 450 470
AACACCATCTGCCTGGCCATGCAGCACTACGGCCAGAGCTGCCTGTTCAAAATCGCCATG
AsnThrIleCysLeuAlaMetGlnHisTyrGlyGlnSerCysLeuPheLysIleAlaMet

490 510 530
AACATCCTCAACATGCTCTTCACTGGCCTCTtCACCGTGGAGATGATCCTGAAGCTCATT
AsnIleLeuAsnMetLeuPheThrGlyLeuPheThrValGluMetIleLeuLysLeuIle

550 570 590
GCCTTCAAACCCAAGCACTATTTCTGTGATGCATGGAATACATTTGACGCCTTGATTGTT
AlaPheLysProLysHisTyrPheCysAspAlaTrpAsnThrPheAspAlaLeuIleVal

610 630 650
GTGGGTAGCATTGTTGATATAGCAATCACCGAGGTAAACCCAGCTGAACATACCCAATGC
ValGlySerIleValAspIleAlaIleThrGluValAsnProAlaGluHisThrGlnCys

670 690 710
TCTCCCTCTATGAAcgcAGAGGAAAACTCCCGCATCTCCATCACCTTCTTCCGCCTGTTC
SerProSerMetAsnAlaGluGluAsnSerArgIleSerIleThrPhePheArgLeuPhe

730 750 770
CGGGTCATGCGTCTGGTGAAGCTGCTGAGCCGTGGGGAGGGCATCCGGACGCTGCTGTGG
ArgValMetArgLeuValLysLeuLeuSerArgGlyGluGlyIleArgThrLeuLeuTrp

790 810 830
ACCTTCATCAAGTCCTTCCAGGCCCTGCCCTATGTGGCCCTCCTGATCGTGATGCTGTTC
ThrPheIleLysSerPheGlnAlaLeuProTyrValAlaLeuLeuIleValMetLeuPhe

### Sequenzprotokoll:

- Sequenz Nr.:: 27980/7
- Int. Code:: pR9112-10.1.1.1. entire clone
- Länge:: 2.049 bp
- Typ:: DNA + Aminosäure

850 870 890
TTCATCTACGCGGtGATCGGGATGCAGGTGTTTGGGAAAATTGCcCTGAATGatACCACA
PheIleTyrAlaValIleGlyMetGlnValPheGlyLysIleAlaLeuAsnAspThrThr

910 930 950
GAGAtcAACCGGAACAACAACTTTCAGACCTtccCCCAGGCCGTGCTGCTCCTCTTCAGG
GluIleAsnArgAsnAsnAsnPheGlnThrPheProGlnAlaValLeuLeuLeuPheArg

970 990 1010
TGTGCCACCGGGGAGGCCTGGCAGGACATCATGCTGGCCTGCATGCCAGGCAAGAAGTGT
CysAlaThrGlyGluAlaTrpGlnAspIleMetLeuAlaCysMetProGlyLysLysCys

1030 1050 1070
gcCCCAGAGTCCGAGCCCAGCAACAGCACGGAGGGTGAAACACCCTGTGGTAGCAGCTTT
AlaProGluSerGluProSerAsnSerThrGluGlyGluThrProCysGlySerSerPhe

1090 1110 1130
GCTGTCTTCTACTTCATCAGCTTCTACATGCTCTGTGCCTTCCTGATCATCAACCTCTTT
AlaValPheTyrPheIleSerPheTyrMetLeuCysAlaPheLeuIleIleAsnLeuPhe

1150 1170 1190
GTAGCTGTCATCATGGACAACTTTGACTACCTGACAAGGGACTGGTCCATCCTTGGTCCC
ValAlaValIleMetAspAsnPheAspTyrLeuThrArgAspTrpSerIleLeuGlyPro

1210 1230 1250
CACCACCTGGATGAGTTTAAAAGAATCTGGGCAGAGTATGACCCTGAAGCCAAGGGTCGT
HisHisLeuAspGluPheLysArgIleTrpAlaGluTyrAspProGluAlaLysGlyArg

### Sequenzprotokoll:

- Sequenz Nr.:: 27980/7
- Int. Code:: pR9112-10.1.1.1. entire clone
- Länge:: 2.049 bp
- Typ:: DNA + Aminosäure

1270 1290 1310
ATCAAACACCTGGATGTGGTGACCCTCCTCCGGCGGATTCAGCCGCCACTAGGTTTTgGG
IleLysHisLeuAspValValThrLeuLeuArgArgIleGlnProProLeuGlyPheGly

1330 1350 1370
AAGCTGTGCCCTCACCGCGTGGCTTGCAAACGCCTGGTCTCCATGAACATGCCTCTGAAC
LysLeuCysProHisArgValAlaCysLysArgLeuValSerMetAsnMetProLeuAsn

1390 1410 1430
AGCGACGGGACAGTCATGTTCAATGCCACCCTGTTTGCCCTGGTCAGGACGGCCCTGAGG
SerAspGlyThrValMetPheAsnAlaThrLeuPheAlaLeuValArgThrAlaLeuArg

1450 1470 1490
ATCAAAACAGAAGAGGGACCCANCCCATCAGAGGCCCACCAAGGGgCTGAGGATCCTTTC
IleLysThrGluGluGlyProXxxProSerGluAlaHisGlnGlyAlaGluAspProPhe

1510 1530 1550
CGCCCAGCAGGGAACCTAGAACAAGCCAATGAGGAGCTGCGGGCGATCATCAAGAAGATC
ArgProAlaGlyAsnLeuGluGlnAlaAsnGluGluLeuArgAlaIleIleLysLysIle

1570 1590 1610
TGGAAGCGgACCAGCATGAAGCTGCTGGACCAGGTGGTGCCCCCTGCAGGTGATGATGAG
TrpLysArgThrSerMetLysLeuLeuAspGlnValValProProAlaGlyAspAspGlu

1630 1650 1670
GTCACCGTTGGCAAGTTCTACGCCACGTTCCTGATCCAGGAGTACTTCCGGAAGTTCAAG
ValThrValGlyLysPheTyrAlaThrPheLeuIleGlnGluTyrPheArgLysPheLys

### Sequenzprotokoll:

- Sequenz Nr.:: 27980/7
- Int. Code:: pR9112-10.1.1.1. entire clone
- Länge:: 2.049 bp
- Typ:: DNA + Aminosäure

1690 1710 1730
AAGCGCAAAGAGCAGGGCCTTGTGGgCAAGCCCTCCCAGAGGAACGCGCTGTCTCTGCAG
LysArgLysGluGlnGlyLeuValGlyLysProSerGlnArgAsnAlaLeuSerLeuGln

1750 1770 1790
GCTGGCTTGCGCACACTGCATGACATCGGGCCTGAGATCCGACGGGccATCTCTGGAGAT
AlaGlyLeuArgThrLeuHisAspIleGlyProGluIleArgArgAlaIleSerGlyAsp

1810 1830 1850
CTCACCGcTGAGGAGGAGCTGGACAAGGCCATGAAGGAGGCTGTGTCCGCTGCTTCTGAA
LeuThrAlaGluGluGluLeuAspLysAlaMetLysGluAlaValSerAlaAlaSerGlu

1870 1890 1910
GATGACATCTTCAGGAGggcCGGTGGCCTGTTCGGCAACCACGTCAGCTACTACCAAAGC
AspAspIlePheArgArgAlaGlyGlyLeuPheGlyAsnHisValSerTyrTyrGlnSer

1930 1950 1970
GACGGCCGGAGCGCCTTCCNNNAGACCTTCACCACTCAGCGCCCGCTGCACATCAACAAG
AspGlyArgSerAlaPheXxxXxxThrPheThrThrGlnArgProLeuHisIleAsnLys

1990 2010 2030
GCGGGCAGCAGCCAGGGCGACACTGAGTCGCCATCCCACGAGAAGCTGGTGGACTCCACC
AlaGlySerSerGlnGlyAspThrGluSerProSerHisGluLysLeuValAspSerThr

2050
TTCACCCCGA
PheThrPro

### Sequenzprotokoll:

- Sequenz Nr.:: 27980/8
- Int. Code:: pR9112-12.1.1.1. entire clone
- Länge:: 997 bp
- Typ:: DNA + Aminosäure

10                    30                    50
GAACATCGTGTTCACATCCATGTTCTCCATGGAATGCGTGCTgAAGATCATCGCCTTTGGG
AsnIleValPheThrSerMetPheSerMetGluCysValLeuLysIleIleAlaPheGly

70                    90                    110
GTGctgaaCTATTTCAGAgATGCCTGNANTGTCtTTAACNNNGTCACtGTGTTGGGAAGT
ValLeuAsnTyrPheArgAspAlaXxxXxxValPheAsnXxxValThrValLeuGlySer

130                   150                   170
ATTANTGATATTTTAGTAACAGAGATTGCGAANAATTTCATCAACCTCAGNTTNCTCcGC
IleXxxAspIleLeuValThrGluIleAlaXxxAsnPheIleAsnLeuXxxXxxLeuArg

190                   210                   230
NTCTTTCGAcgTGNGNGGCTGATCAAGNTcgTCcGNCAGGGCTACACCATCCGCATCCTG
XxxPheArgArgXxxXxxLeuIleLysXxxValArgGlnGlyTyrThrIleArgIleLeu

250                   270                   290
CTGTGGACCTTTGTCCAGTCCTTCAAGGCCCTGCCCTACGTGTGTCTGCTCATTGCCATG
LeuTrpThrPheValGlnSerPheLysAlaLeuProTyrValCysLeuLeuIleAlaMet

310                   330                   350
CTGTTCTTCATCTACGCCATCATCGGCATGCAGGTGTTTGGGAATATTGCCCTGGATGAT
LeuPhePheIleTyrAlaIleIleGlyMetGlnValPheGlyAsnIleAlaLeuAspAsp

370                   390                   410
GACACCAgCATCAACCgCCACAACAACTTCCGGACGTTTTTGCAAGCCCTGATGCTGCTG
AspThrSerIleAsnArgHisAsnAsnPheArgThrPheLeuGlnAlaLeuMetLeuLeu

### Sequenzprotokoll:

- Sequenz Nr.:: 27980/8
- Int. Code:: pR9112-12.1.1.1. entire clone
- Länge:: 997 bp
- Typ:: DNA + Aminosäure

430 450 470
TTCAGGAGCGCCACgGGGAGGCCTGGCACGAGATCATGCTGTCCTGCCTGAGCAACCAG
PheArgSerAlaThrGlyGluAlaTrpHisGluIleMetLeuSerCysLeuSerAsnGln

490 510 530
GCCTGTGATGAGCAGGCCAATGCCACCGAGTGTGGAAGTGACTTTGCCTACTTCTACTTC
AlaCysAspGluGlnAlaAsnAlaThrGluCysGlySerAspPheAlaTyrPheTyrPhe

550 570 590
GTCTCCTTCATCTTCCTGTGCTCCTTTCTGATGTTGAACCTCTTTGTGGCTGTGATCATG
ValSerPheIlePheLeuCysSerPheLeuMetLeuAsnLeuPheValAlaValIleMet

610 630 650
GACAATTTTGAGTACCTCACGCGGGACTCTTCCATCCTAGGTCCTCACCACTTGGATGAG
AspAsnPheGluTyrLeuThrArgAspSerSerIleLeuGlyProHisHisLeuAspGlu

670 690 710
TTCATCCGGGTCTGGGCTGAATACGACCCGGCTGCGTGTGGGCGCATCAGTTACAATGAC
PheIleArgValTrpAlaGluTyrAspProAlaAlaCysGlyArgIleSerTyrAsnAsp

730 750 770
ATGTTTGAGATGCTGAAACACATGTCCCCGCCTCTGGGGCTGGGGAAGAAATGCCCTGCT
MetPheGluMetLeuLysHisMetSerProProLeuGlyLeuGlyLysLysCysProAla

790 810 830
CGAGTTGCTTACAAGCGCCTGGTTCGCATGAACATGCCCATCTCCAACGAGGACATGACT
ArgValAlaTyrLysArgLeuValArgMetAsnMetProIleSerAsnGluAspMetThr

### Sequenzprotokoll:

- Sequenz Nr.:: 27980/8
- Int. Code:: pR9112-12.1.1.1. entire clone
- Länge:: 997 bp
- Typ:: DNA + Aminosäure

850 870 890
GTTCACTTCACGTCCACGCTGATGGCCCTCATCCGGACGGCACTGGAGATCAAGCTGGCC
ValHisPheThrSerThrLeuMetAlaLeuIleArgThrAlaLeuGluIleLysLeuAla

910 930 950
CCAGCTGGgACAAAGCAGCATCAGTGTGACGCGGAGTTGAGGAAGGAGATTTCCGTTGTG
ProAlaGlyThrLysGlnHisGlnCysAspAlaGluLeuArgLysGluIleSerValVal

970 990
TGGGCCAATCTGCCCCAGAAGACTTTGGACTTGCTG
TrpAlaAsnLeuProGlnLysThrLeuAspLeuLeu

### Sequenzprotokoll:

- Sequenz Nr.:: 27980/9
- Int. Code:: pR9112-2.1.1.1. entire clone
- Länge:: 1.471 bp
- Typ:: DNA + Aminosäure

10                    30                    50
CCTCTATGAACGCAGAGGAAAACTCCCGCATCTCCATCACCTTCTTCCGCCTGTTCCGGGTC
SerMetAsnAlaGluGluAsnSerArgIleSerIleThrPhePheArgLeuPheArgVal

70                    90                    110
ATGCGTCTGGTGAAGCTGCTGAGCCGTGGGGAGGGCATCCGGACGCTGCTGTGGACCTTC
MetArgLeuValLysLeuLeuSerArgGlyGluGlyIleArgThrLeuLeuTrpThrPhe

130                   150                   170
ATCAAGTCCTTCCAGGCCCTGCCCTATGTGGCCCTCCTGATCGTGATGCTGTTCTTCATC
IleLysSerPheGlnAlaLeuProTyrValAlaLeuLeuIleValMetLeuPhePheIle

190                   210                   230
TACGCGGtGATCGGGATGCAGGTGTTTGGGAAAATTNCCCTGAATGATACCACAGAGATC
TyrAlaValIleGlyMetGlnValPheGlyLysIleXxxLeuAsnAspThrThrGluIle

250                   270                   290
AACCGGAACAACAACTTTCAGACCTtccCCCAGGCCGTGCTGCTCCTCTTCAGGTGTGCC
AsnArgAsnAsnAsnPheGlnThrPheProGlnAlaValLeuLeuLeuPheArgCysAla

310                   330                   350
ACCGGGGAGGCCTGGCAGGACATCATGCTGGCCTGCATGCCAGGCAAGAAGTGTgcCCCA
ThrGlyGluAlaTrpGlnAspIleMetLeuAlaCysMetProGlyLysLysCysAlaPro

370                   390                   410
GAGTCCGAGCCCAGCAACAGCACGGAGGGTGAAACACCCTGTGGTAGCAGCTTTGCTGTC
GluSerGluProSerAsnSerThrGluGlyGluThrProCysGlySerSerPheAlaVal

### Sequenzprotokoll:

- Sequenz Nr.:: 27980/9
- Int. Code:: pR9112-2.1.1.1. entire clone
- Länge:: 1.471 bp
- Typ:: DNA + Aminosäure

430 450 470
TTCTACTTCATCAGCTTCTACATGCTCTGTGCCTTCCTGATCATCAACCTCTTTGTAGCT
PheTyrPheIleSerPheTyrMetLeuCysAlaPheLeuIleIleAsnLeuPheValAla

490 510 530
GTCATCATGGACAACTTTGACTACCTGACAAGGGACTGGTCCATCCTTGGTCCCCACCAC
ValIleMetAspAsnPheAspTyrLeuThrArgAspTrpSerIleLeuGlyProHisHis

550 570 590
CTGGATGAGTTTAAAAGAATCTGGGCAGAGTATGACCCTGAAGCCAAGGGTCGTATCAAA
LeuAspGluPheLysArgIleTrpAlaGluTyrAspProGluAlaLysGlyArgIleLys

610 630 650
CACCTGGATGTGGTGACCCTCCTCCGGCGGATTCAGCCGCCACTAGGTTTTgGGAAGCTG
HisLeuAspValValThrLeuLeuArgArgIleGlnProProLeuGlyPheGlyLysLeu

670 690 710
TGCCCTCACCGCGTGGCTTGCAAACGCCTGGTCTCCATGAACATGCCTCTGAACAGCGAC
CysProHisArgValAlaCysLysArgLeuValSerMetAsnMetProLeuAsnSerAsp

730 750 770
GGGACAGTCATGTTCAATGCCACCCTGTTTGCCCTGGTCAGGACGGCCCTGAGGATCAAA
GlyThrValMetPheAsnAlaThrLeuPheAlaLeuValArgThrAlaLeuArgIleLys

790 810 830
ACAGAAGGGAACCTAGAACAAGCCAATGAGGAGCTGCGGGCGATCATCAAGAAGATCTGG
ThrGluGlyAsnLeuGluGlnAlaAsnGluGluLeuArgAlaIleIleLysLysIleTrp

### Sequenzprotokoll:

- Sequenz Nr.:: 27980/9
- Int. Code:: pR9112-2.1.1.1. entire clone
- Länge:: 1.471 bp
- Typ:: DNA + Aminosäure

850 870 890
AAGCGGACCAGCAtgAAGCTGCTGGACCAGGTGGTGCCCCCTGCAGGTGATGATGAGGTC
LysArgThrSerMetLysLeuLeuAspGlnValValProProAlaGlyAspAspGluVal

910 930 950
ACCGTTGGCAAGTTCTACGCCACGTTCCTGATCCAGGAGTACTTCCGGAAGTTCAAGAAG
ThrValGlyLysPheTyrAlaThrPheLeuIleGlnGluTyrPheArgLysPheLysLys

970 990 1010
CGCAAAGAGCAGGGCCTTGTGGcAAGCCCTCCCAGAGGAACGCGCTGTCTCTGCAGGCT
ArgLysGluGlnGlyLeuValGlyLysProSerGlnArgAsnAlaLeuSerLeuGlnAla

1030 1050 1070
GGcTTGCGCACACTGCATGACATCGGGCCTGAGATCCGACGGGCCATCTCTGGAGATCTC
GlyLeuArgThrLeuHisAspIleGlyProGluIleArgArgAlaIleSerGlyAspLeu

1090 1110 1130
ACCGCTGAGGAGGAGCTGGACAAGGCCATGAAGGAGGCTGTGTCCGCTGCTTCTGAAGAT
ThrAlaGluGluGluLeuAspLysAlaMetLysGluAlaValSerAlaAlaSerGluAsp

1150 1170 1190
GACATCTTCAGGAGGGCCGGTggccTGTTCGGCAACCACGTCAGCTACTACCAAAGCGAC
AspIlePheArgArgAlaGlyGlyLeuPheGlyAsnHisValSerTyrTyrGlnSerAsp

1210 1230 1250
GGCCGGANNGCCTTCcCCCAGACCTTCACCACTCAGCNCCCGCTGCACATCAACAAGGCg
GlyArgXxxAlaPheProGlnThrPheThrThrGlnXxxProLeuHisIleAsnLysAla

### Sequenzprotokoll:

- Sequenz Nr.:: 27980/9
- Int. Code:: pR9112-2.1.1.1. entire clone
- Länge:: 1.471 bp
- Typ:: DNA + Aminosäure

1270 1290 1310
GGCAGCAGCCAGGGCGACACTGAGTCGCCATCCCACGAGAAGCTGGTGGACTCCACCTTC
GlySerSerGlnGlyAspThrGluSerProSerHisGluLysLeuValAspSerThrPhe

1330 1350 1370
ACCCCGAGCAGCTACTCGTCCACCGGCTCCAACGCCAACATCAACAACGCCAACAACACC
ThrProSerSerTyrSerSerThrGlySerAsnAlaAsnIleAsnAsnAlaAsnAsnThr

1390 1410 1430
GCCCTGGGTCGCCTCCCTCNNCCCGCCGGCTACCCCAGCACAGTCAGCACTGTGGAgcCA
AlaLeuGlyArgLeuProXxxProAlaGlyTyrProSerThrValSerThrValGluPro

1450 1470
CGGGCCCCCCTTGTCCCCTGCCATCCGGG
ArgAlaProLeuValProCysHisPro

### Sequenzprotokoll:

- Sequenz Nr.:: 27980/10
- Int. Code:: pRR5-8 entire clone
- Länge:: 2.655 bp
- Typ:: DNA + Aminosäure

490 510 530
CAAGAAGCTCATCGATTGGCCGCCATTTGAGTACATGATCCTGGCCACCATCATTGCCAA
aLysLysLeuIleAspTrpProProPheGluTyrMetIleLeuAlaThrIleIleAlaAs

550 570 590
CTGCATCGTCCTGGCCCTGGAGCAGCATCTTCCTGAGGATGACAAGACCCCCATGTCCcG
nCysIleValLeuAlaLeuGluGlnHisLeuProGluAspAspLysThrProMetSerAr

610 630 650
AAGACTGGAGAAGACAGAACCTTATTTCATTGGGATCTTTTGCTTTGAAGCTGGGATCAA
gArgLeuGluLysThrGluProTyrPheIleGlyIlePheCysPheGluAlaGlyIleLy

670 690 710
AATTGTGGCCCTGGGGTTCATCTTCCATAAGGGCTCTTACCTCCGCAATGGCTGGAATGT
sIleValAlaLeuGlyPheIlePheHisLysGlySerTyrLeuArgAsnGlyTrpAsnVa

730 750 770
CATGGACTTCATCGTGGTcCTCAgTgGCATCCTGGCCACTGCAGGAACCCACTTCAATAC
lMetAspPheIleValValLeuSerGlyIleLeuAlaThrAlaGlyThrHisPheAsnTh

790 810 830
TCACGTGGACCTGAGGACCCTCCGGGCTGTGCGTGTCCTGCGGCCTTTGAAGCTCGTGTC
rHisValAspLeuArgThrLeuArgAlaValArgValLeuArgProLeuLysLeuValSe

850 870 890
AGGGATACCcTAGCCTGCAGATTGTGTTGAAGTCcATCATGAAGGCCATGGTACCTCTTCT
rGlyIleProSerLeuGlnIleValLeuLysSerIleMetLysAlaMetValProLeuLe

910 930 950
GCAGATTGGCCTTCTgCTCTTCTTTgCCATCCTGATGTTTGCTATCATTGGTTTGGAGTT
uGlnIleGlyLeuLeuLeuPhePheAlaIleLeuMetPheAlaIleIleGlyLeuGluPh

### Sequenzprotokoll:

- Sequenz Nr.:: 27980/10
- Int. Code:: pRR5-8
- Länge:: 2.655 bp
- Typ:: DNA + Aminosäure

970 990 1010
CTACAGTGGCAAGTTACATCGAGCATGCTTCATGAACAATTCAGGTATTCTAGAAGGATT
eTyrSerGlyLysLeuHisArgAlaCysPheMetAsnAsnSerGlyIleLeuGluGlyPh

1030 1050 1070
TGACCCCCCTCACCCATGTGGTGTGCAGGGCTGCCCAGCTGGTTATGAATGCAAGGACTG
eAspProProHisProCysGlyValGlnGlyCysProAlaGlyTyrGluCysLysAspTr

1090 1110 1130
GATCGGCCCCAATGATGGGATCACCCAGTTTGATAACATCCTTTTTGCTGTGCTGACTGT
pIleGlyProAsnAspGlyIleThrGlnPheAspAsnIleLeuPheAlaValLeuThrVa

1150 1170 1190
CTTCCAGTGCATCACCATGGAAGGGTGGACCACTGTGCTGTACAATACCAATGATGCCTT
lPheGlnCysIleThrMetGluGlyTrpThrThrValLeuTyrAsnThrAsnAspAlaLe

1210 1230 1250
AGGAGCCACCTGGAATTGGCTGTACTTCATCCCCCTCATCATCATTGGATCCTTCTTTGT
uGlyAlaThrTrpAsnTrpLeuTyrPheIleProLeuIleIleIleGlySerPhePheVa

1270 1290 1310
TCTCAACCTAGTCCTGGGAGTGCTTTCCGGGGAATTTGCCAAAGAGAGAGAGAGAGTGGA
lLeuAsnLeuValLeuGlyValLeuSerGlyGluPheAlaLysGluArgGluArgValGl

### Sequenzprotokoll:

- Sequenz Nr.:: 27980/10
- Int. Code:: pRR5-8
- Länge:: 2.655 bp
- Typ:: DNA + Aminosäure

1330 1350 1370
GAACCGAAGGGCTTTCATgAAGCTGCGGCGCCAGCAGCAGATTGAGCGTGAGCTGAATGG
uAsnArgArgAlaPheMetLysLeuArgArgGlnGlnGlnIleGluArgGluLeuAsnGl

1390 1410 1430
CTACCGTGCCTGGATAGACAAAGCAGAGGAAGTCATGCTCGCTGAAGAAAATAAAAATGC
yTyrArgAlaTrpIleAspLysAlaGluGluValMetLeuAlaGluGluAsnLysAsnAl

1450 1470 1490
TGGAACATCCGCCTTAGAAGTGCTTCGAAGGGCAACCATCAAGAGGAGCCGGACAGAGGC
aGlyThrSerAlaLeuGluValLeuArgArgAlaThrIleLysArgSerArgThrGluAl

1510 1530 1550
CATGACTCGAGACTCCAGTGATGAGCACTGTGTTGATATCTCCTCTGTGGGCACACCTCT
aMetThrArgAspSerSerAspGluHisCysValAspIleSerSerValGlyThrProLe

1570 1590 1610
GGCCCGAGCCAGTATCAAAAGTGCAAAGGTAGACGGGGTCTCTTATTTCCGGCACAAGGA
uAlaArgAlaSerIleLysSerAlaLysValAspGlyValSerTyrPheArgHisLysGl

1630 1650 1670
AAGGCTTCTGCGCATCTCCATTCGCCACATGGTTAAATCCCAGGTGTTTTACTGGATTGT
uArgLeuLeuArgIleSerIleArgHisMetValLysSerGlnValPheTyrTrpIleVa

1690 1710 1730
GCTGAGCCTTGTGGCACTCAACACTGCCTGTGTGGCCATTGTCCATCACAACCAGCCCCA
lLeuSerLeuValAlaLeuAsnThrAlaCysValAlaIleValHisHisAsnGlnProGl

### Sequenzprotokoll:

- Sequenz Nr.:: 27980/10
- Int. Code:: pRM-8
- Länge:: 2.655 bp
- Typ:: DNA + Aminosäure

1750 1770 1790
GTGGCTCACCCACCTCCTCTACTATGCAGAATTTCTGTTTCTGGGACTCTTCCTCTTGGA
nTrpLeuThrHisLeuLeuTyrTyrAlaGluPheLeuPheLeuGlyLeuPheLeuLeuGl

1810 1830 1850
GATGTCCCTGAAGATGTATGGCATGGGGCCTCGCCTTTATTTTCACTCTTCATTCAACTG
uMetSerLeuLysMetTyrGlyMetGlyProArgLeuTyrPheHisSerSerPheAsnCy

1870 1890 1910
CTTTGATTTTGGGGTCACAGTGGGCAGTATCTTTGAAGTGGTCTGGGCAATCTTCAGACC
sPheAspPheGlyValThrValGlySerIlePheGluValValTrpAlaIlePheArgPr

1930 1950 1970
TGGTACGTCTTTTGGAATCAGTGTCTTGCGAGCCCTCCGGCTTCTAAGAATATTTAAAAT
oGlyThrSerPheGlyIleSerValLeuArgAlaLeuArgLeuLeuArgIlePheLysIl

1990 2010 2030
AACCAAGTATTGGGCTTCCCTACGGAATTTGGTGGTCTCCTTGATGAGCTCAATGAAGTC
eThrLysTyrTrpAlaSerLeuArgAsnLeuValValSerLeuMetSerSerMetLysSe

2050 2070 2090
TATCATCAGTTTGCTTTTCCTCCTCTTCCTCTTCATCGTTGTCTTTGCTCTCCTAGGAAT
rIleIleSerLeuLeuPheLeuLeuPheLeuPheIleValValPheAlaLeuLeuGlyMe

2110 2130 2150
GCAGTTATTTGGAGGCAGGTTTAACTTTAATGATGGGACTCCTTCGGCAAATTTTGATAC
tGlnLeuPheGlyGlyArgPheAsnPheAsnAspGlyThrProSerAlaAsnPheAspTh

### Sequenzprotokoll:

- Sequenz Nr.:: 27980/10
- Int. Code:: pRR3-8
- Länge:: 2.655 bp
- Typ:: DNA + Aminosäure

2170                    2190                    2210
CTTCCCTGCAGCCATCATGACTGTGTTCCAGATCCTGACGGGTGAGGACTGGAATGAGGT
rPheProAlaAlaIleMetThrValPheGlnIleLeuThrGlyGluAspTrpAsnGluVa

2230                    2250                    2270
GATGTACAATGGGATCCGCTCCCAGGGTGGGGTCAGCTCAGGCATGTGGTCTGCCATCTA
lMetTyrAsnGlyIleArgSerGlnGlyGlyValSerSerGlyMetTrpSerAlaIleTy

2290                    2310                    2330
CTTCATTGTGCTCACCTTGTTTGGCAACTACACGCTACTGAATGTGTTCTTGGCTATCGC
rPheIleValLeuThrLeuPheGlyAsnTyrThrLeuLeuAsnValPheLeuAlaIleAl

2350                    2370                    2390
TGTGGATAATCTCGCCAACGCCCAGGAACTGACCAAGGATGAACAGGAGGAAGAAGAGGC
aValAspAsnLeuAlaAsnAlaGlnGluLeuThrLysAspGluGlnGluGluGluGluAl

2410                    2430                    2450
CTTCAACCAGAAACATGCACTGCAGAAGGCCAAGGAGGTCAGCCCGATGTCTGCACCCAA
aPheAsnGlnLysHisAlaLeuGlnLysAlaLysGluValSerProMetSerAlaProAs

2470                    2490                    2510
CATGCCTTCGATCGAAAGAGACAGAAGGAGAAGACACCACATGTCGATGTGGGAGCCACG
nMetProSerIleGluArgAspArgArgArgArgHisHisMetSerMetTrpGluProAr

### Sequenzprotokoll:

- Sequenz Nr.:: 27980/10
- Int. Code:: pRR5-8
- Länge:: 2.655 bp
- Typ:: DNA + Aminosäure

2530                2550                2570
CAGCAGCCACCTGAGGGAGCGGAGGCGCCGGCACCACATGTCCGTGTGGGAGCAGCGTAC
gSerSerHisLeuArgGluArgArgArgArgHisHisMetSerValTrpGluGlnArgTh

2590                2610                2630
CAGCCAGCTGAGGAAGCACATGCAGATGTCCAGCCAGGAGGCCCTCAACAGAGAGGAGGC
rSerGlnLeuArgLysHisMetGlnMetSerSerGlnGluAlaLeuAsnArgGluGluAl

2650
GCCGACCATGAGCCC
aProThrMetSer

### Sequenzprotokoll:

- Sequenz Nr.:: 27980/11
- Int. Code:: pR14-5.3.3.1, entire clone
- Länge:: 6232 bp
- Typ:: DNA + Aminosäure

10                    30                    50
TCCGCCTCTCGGGTTCAGGCAATTCTCCTGCCTCAGCCTCCCGAGTAGCTGGGACTATAG

70                    90                    110
GTGCTCACCACCATGCCTGGCTGATTTTGTATTTTAGTAGAGACAGGGTCTCCTCATG

130                   150                   170
TTGGCCAGGTTGGTCTGAAATTCCTGACCTCAGGTGCTCCACCCACCTTGGCcTCCCAAA

190                   210                   230
GTCCTGGGATTACAGGCGTGAGTCACTGCACTGGCTTTTTTTTTTTTTAAGACAGAGT

250                   270                   290
TTTGCTTATGCGGAGGAGAAGTCCCCTTTGGACGTGCTGAAGAGAGCGGCCACCAAGAAG
GluGluLysSerProLeuAspValLeuLysArgAlaAlaThrLysLys

310                   330                   350
AGCAGAAATGACCTGATCCACGCAGAGGAGGGAGAGGACCGGTTTGCAGATCTCTGTGCT
SerArgAsnAspLeuIleHisAlaGluGluGlyGluAspArgPheAlaAspLeuCysAla

370                   390                   410
GTTGGATCCCCCTTCGCCcGCGCCAGCCTCAAGAGCGGGAAGACAGAGAGCTCGTCATAC
ValGlySerProPheAlaArgAlaSerLeuLysSerGlyLysThrGluSerSerSerTyr

### Sequenzprotokoll:

- Sequenz Nr.:: 27980/11
- Int. Code:: pR14-5.3.3.1, entire clone
- Länge:: 6232 bp
- Typ:: DNA + Aminosäure

pR14-5.3.3.1, cont.

430 450 470
TTCCGGAGGAAGGAGAAGATGTTCCGGTTTTTATCCGGCGCATGGTGAAGGCTCAGAGC
PheArgArgLysGluLysMetPheArgPhePheIleArgArgMetValLysAlaGlnSer

490 510 530
TTCTACTGGGTGGTGCTGTGCGTGGTGGCCCTGAACACACTGTGTGTGGCCATGGTGCAT
PheTyrTrpValValLeuCysValValAlaLeuAsnThrLeuCysValAlaMetValHis

550 570 590
TACAACCAGCCGCGGCGGCTTACCACGACCCTGTATTTTGCAGAGTTTGTTTTCCTGGGT
TyrAsnGlnProArgArgLeuThrThrThrLeuTyrPheAlaGluPheValPheLeuGly

610 630 650
CTCTTCCTCACAGAGATGTCCCTGAAGATGTATGGCCTGGGGCCCAGAAGCTACTTCCGG
LeuPheLeuThrGluMetSerLeuLysMetTyrGlyLeuGlyProArgSerTyrPheArg

670 690 710
TCCTCCTTCAACTGCTTCGACTTTGGGGTCATCGTGGGGAGCGTCTTTGAAGTGGTCTGG
SerSerPheAsnCysPheAspPheGlyValIleValGlySerValPheGluValValTrp

730 750 770
GCGGCCATCAAGCCGGGAAGCTCCTTTGGGATCAGTGTGCTgCgGGCCCTCCGCCTGCTG
AlaAlaIleLysProGlySerSerPheGlyIleSerValLeuArgAlaLeuArgLeuLeu

790 810 830
AGGATCTTCAAAGTCACGAAGTACTGGAGCTCCCTGCGGAACCTGGTGGTGTCCCTGCTG
ArgIlePheLysValThrLysTyrTrpSerSerLeuArgAsnLeuValValSerLeuLeu

### Sequenzprotokoll:

- Sequenz Nr.:: 27980/11
- Int. Code:: pR14-5.3.3.1, entire clone
- Länge:: 6232 bp
- Typ:: DNA + Aminosäure

pR14-5.3.3.1, cont.

850 870 890
AACTCCATGAAGTCCATCATCAGCCTGCTCTTCTTGCTCTTCCTGTTCATTGTGGTCTTC
AsnSerMetLysSerIleIleSerLeuLeuPheLeuLeuPheLeuPheIleValValPhe

910 930 950
GCCCTGCTGGGGATGCAGCTGTTTGGGGGACAGTTCAACTTCCAGGATGAGACTCCCACA
AlaLeuLeuGlyMetGlnLeuPheGlyGlyGlnPheAsnPheGlnAspGluThrProThr

970 990 1010
ACCAACTTCGACACCTTCCCTGCCGCCATCCTCACTGTCTTCCAGATCCTGACGGGAGAG
ThrAsnPheAspThrPheProAlaAlaIleLeuThrValPheGlnIleLeuThrGlyGlu

1030 1050 1070
GACTGGAATGCAGTGATGTATCACGGGATCGAATCGCAAGGCGGCGTCAGCAAAGGCATG
AspTrpAsnAlaValMetTyrHisGlyIleGluSerGlnGlyGlyValSerLysGlyMet

1090 1110 1130
TTCTCGTCCTTtTACTTCATTGTCCTGACACTGTTCGGAAACTACACTCTGCTGAAtGTC
PheSerSerPheTyrPheIleValLeuThrLeuPheGlyAsnTyrThrLeuLeuAsnVal

1150 1170 1190
TTTCTGGCCATCGCTGTGGACAACCTGGCCAACGCCCAAGAGCTGACCAAGGATGAAGAG
PheLeuAlaIleAlaValAspAsnLeuAlaAsnAlaGlnGluLeuThrLysAspGluGlu

1210 1230 1250
GAGATGGAAGAAGCAGCCAATCAGAAGCTTGCTCTGCAAAAGGCCAAAGAAGTGGCTGAA
GluMetGluGluAlaAlaAsnGlnLysLeuAlaLeuGlnLysAlaLysGluValAlaGlu

### Sequenzprotokoll:

- Sequenz Nr.:: 27980/11
- Int. Code:: pR14-5.3.3.1, entire clone
- Länge:: 6232 bp
- Typ:: DNA + Aminosäure

pR14-5.3.3.1, cont.

1270 1290 1310
GTCAGCCCCATGTCTGCCGCGAACATCTCCATCGCCGCGCAGCAGAACTCGGCCAAGGCG
ValSerProMetSerAlaAlaAsnIleSerIleAlaAlaGlnGlnAsnSerAlaLysAla

1330 1350 1370
CGCTCGGTGTGGGAGCAGCGGGCCAGCCAGCTACGGCTGCAGAACCTGCGGGCCAGCTGC
ArgSerValTrpGluGlnArgAlaSerGlnLeuArgLeuGlnAsnLeuArgAlaSerCys

1390 1410 1430
GAGGCGCTGTACAGCGAGATGGACCCCGAGgAGCGGCTGCGCTTCGCCACTACGCGCCAC
GluAlaLeuTyrSerGluMetAspProGluGluArgLeuArgPheAlaThrThrArgHis

1450 1470 1490
CTGCGGCCCGACATGAAGACGCACCTGGACCGGCCGCTGGTGGTGGAGCTGGGCCGCGAC
LeuArgProAspMetLysThrHisLeuAspArgProLeuValValGluLeuGlyArgAsp

1510 1530 1550
GGcGCGCGGGGGCCCGTGGGAGGCAAAGCCCGACCTGAGGCTGCGGAGGCCCCCGAGGGC
GlyAlaArgGlyProValGlyGlyLysAlaArgProGluAlaAlaGluAlaProGluGly

1570 1590 1610
GTCGACCCTCCGCGCAGGCACCACCGGCACCGCGACAAGGACAAGACCCCCGCGGCGGGG
ValAspProProArgArgHisHisArgHisArgAspLysAspLysThrProAlaAlaGly

1630 1650 1670
GACCAGGACCGAGCAGAGGCCCCGAAGGCGGAGAGCGGGGAGCCCGGTGCCCGGGAGGAG
AspGlnAspArgAlaGluAlaProLysAlaGluSerGlyGluProGlyAlaArgGluGlu

### Sequenzprotokoll:

- Sequenz Nr.:: 27980/11
- Int. Code:: pR14-5.3.3.1, entire clone
- Länge:: 6232 bp
- Typ:: DNA + Aminosäure

pR14.5.3.3.1, cont.

1690 1710 1730
CGGCCGCGGCCCGACCGCAGCCACAGCAAGGAGGCCGCGGGCCCCGGAGGCGCGGAGC
ArgProArgProAspArgSerHisSerLysGluAlaAlaGlyProProGluAlaArgSer

1750 1770 1790
GAGCGCGGCCGAGGCCCAGGCCCCGAGGGCGGCCGGCGGCACCACCGGCGCGGCTCCCCG
GluArgGlyArgGlyProGlyProGluGlyGlyArgArgHisHisArgArgGlySerPro

1810 1830 1850
GAGGAGGCGGCCGAGCGGGAGCCCCGACGCCACCGCGCGCACCGGCACCAGGATCCGAGC
GluGluAlaAlaGluArgGluProArgArgHisArgAlaHisArgHisGlnAspProSer

1870 1890 1910
AAGGAGTGCGCCGGCGCCAAGGGCGAGCGGcGcGCGCGGCACCGCGGCGGCCCCCGAGCG
LysGluCysAlaGlyAlaLysGlyGluArgArgAlaArgHisArgGlyGlyProArgAla

1930 1950 1970
GGGCCCCGGGAGGCGGAGAGCGGGGAGGAGCCGGCGCGGCGGCACCGGGCCCGGCACAAG
GlyProArgGluAlaGluSerGlyGluGluProAlaArgArgHisArgAlaArgHisLys

1990 2010 2030
GCGCAGCCTGCTCACGAGGCTGTGGAGAAGGAGACCACgGAGAAGGAGGCCACGGAGAAG
AlaGlnProAlaHisGluAlaValGluLysGluThrThrGluLysGluAlaThrGluLys

2050 2070 2090
GAGGCTGAGATAGTGGAAGCCGACAAGGAAAAGGAGCTCCGGAACCACCAGCCCCGGGAG
GluAlaGluIleValGluAlaAspLysGluLysGluLeuArgAsnHisGlnProArgGlu

### Sequenzprotokoll:

- Sequenz Nr.:: 27980/11
- Int. Code:: pR14-5.3.3.1, entire clone
- Länge:: 6232 bp
- Typ:: DNA + Ardnosäure

pR14-.5.3.3.1, cont.

2110 2130 2150
CCACACTGTGACCTGGAGACCAGTGGACTGTGACTGTGGGTCCCATGCACACACTGCCC
ProHisCysAspLeuGluThrSerGlyThrValThrValGlyProMetHisThrLeuPro

2170 2190 2210
AGCACCTGTCTCCAGAAGGTGGAGGAACAGCCAGAGGATGCAGACAATCAGCGGAACGTC
SerThrCysLeuGlnLysValGluGluGlnProGluAspAlaAspAsnGlnArgAsnVal

2230 2250 2270
ACTCGCATGGGCAGTCAGCCCCCAGACCCGAACACTATTGTACATATCCCAGTGATGCTG
ThrArgMetGlySerGlnProProAspProAsnThrIleValHisIleProValMetLeu

2290 2310 2330
ACGGGCCCTCTTGGGGAAGCCACGGTCGTTCCCAGTGGTAACGTGGACCTGGAAAGCCAA
ThrGlyProLeuGlyGluAlaThrValValProSerGlyAsnValAspLeuGluSerGln

2350 2370 2390
GCAGAGGGGAAGAAGGAGGTGGAAGCGGATGACGTGATGAGGAGCGGCCCCCGGCCTATC
AlaGluGlyLysLysGluValGluAlaAspAspValMetArgSerGlyProArgProIle

2410 2430 2450
GTCCCATACAGCTCCATGTTCTGTTTAAGCCCCACCAACCTGcTCCGCCGCTTCTGCCAC
ValProTyrSerSerMetPheCysLeuSerProThrAsnLeuLeuArgArgPheCysHis

2470 2490 2510
TACATCGTGACCATGAGGTACTTCGAGGTGGTCATTCTCGTGGTCATCGCCTTGAGCAGC
TyrIleValThrMetArgTyrPheGluValValIleLeuValValIleAlaLeuSerSer

### Sequenzprotokoll:

- Sequenz Nr.:: 27980/11
- Int. Code:: pR14-5.3.3.1, entire clone
- Länge:: 6232 bp
- Typ:: DNA + Aminosäure

pR14-5.3.3.1, cont.

2530 2550 2570
ATCGCCCTGGCTGCTGAGGAcCCAGTGCGCACAGACTCGCCCAGGAACAAcGCTCTGAaA
IleAlaLeuAlaAlaGluAspProValArgThrAspSerProArgAsnAsnAlaLeuLys

2590 2610 2630
TACCTGGAtTACATTTTCACTGGTGTCTTTACCTTTGAGATGGTGATAAAGATGATCGAC
TyrLeuAspTyrIlePheThrGlyValPheThrPheGluMetValIleLysMetIleAsp

2650 2670 2690
TTGGGACTGCTGCTTCACCCTGGAGCCTATTTCCGGGACTTGTGGAACATTCTGGACTTC
LeuGlyLeuLeuLeuHisProGlyAlaTyrPheArgAspLeuTrpAsnIleLeuAspPhe

2710 2730 2750
ATTGTGGTCAGTGGCGcCCTGGTGGCGTTTGCtTTCTCAGGATCCAAAGGGaAAGACATC
IleValValSerGlyAlaLeuValAlaPheAlaPheSerGlySerLysGlyLysAspIle

2770 2790 2810
AATACCATCAAGTCTCTGAGAGTCCTTCGTGTcCTGCGGCCCCTCAAGACCATCAAACGG
AsnThrIleLysSerLeuArgValLeuArgValLeuArgProLeuLysThrIleLysArg

2830 2850 2870
CTGCCCAAGCTCAAGGCTGTGTTTGACTGTGTGGTGAACTCCCTGAAGAATGTCCTCAAC
LeuProLysLeuLysAlaValPheAspCysValValAsnSerLeuLysAsnValLeuAsn

2890 2910 2930
ATCTTGATTGTCTACATGCTCTTCATGTTCATATTTGCCGTCATTGCGGTGCAGCTCTTC
IleLeuIleValTyrMetLeuPheMetPheIlePheAlaValIleAlaValGlnLeuPhe

### Sequenzprotokoll:

- Sequenz Nr.:: 27980/11
- Int. Code:: pR14-5.3.3.1, entire clone
- Länge:: 6232 bp
- Typ:: DNA + Aminosäure

pR14-5.3.3.1, cont.

2950 2970 2990
AAAGGGAAGTTTTTCTACTGCACAGATGAATCCAAGGAGCTGGAGAGGGACTGCAGGGGT
LysGlyLysPhePheTyrCysThrAspGluSerLysGluLeuGluArgAspCysArgGly

3010 3030 3050
CAGTATTTGGATTATGAGAAGGAGGAAGTGGAAGCTCAGCCCAGGCAGTGGAAGAAATAC
GlnTyrLeuAspTyrGluLysGluGluValGluAlaGlnProArgGlnTrpLysLysTyr

3070 3090 3110
GACTTTCACTACGACAATGTGCTCTGGGCTCTGCTGACGCTGTTCACAGTGTCCACGGGA
AspPheHisTyrAspAsnValLeuTrpAlaLeuLeuThrLeuPheThrValSerThrGly

3130 3150 3170
GAAGGCTGGCCCATGGTGCTGAAACACTCCGTGGATGCCACCTATGAGGAGCAGGGTCCA
GluGlyTrpProMetValLeuLysHisSerValAspAlaThrTyrGluGluGlnGlyPro

3190 3210 3230
AGCCCTGGGTACCGCATGGAGCTGTCCATCTTCTACGTGGTCTACTTTGTGGTCTTTCCC
SerProGlyTyrArgMetGluLeuSerIlePheTyrValValTyrPheValValPhePro

3250 3270 3290
TTCTTCTTCGTCAACATCTTTGTGGCTTTGATCATCATCACCTTCCAGGAGCAGGGGGAC
PhePhePheValAsnIlePheValAlaLeuIleIleIleThrPheGlnGluGlnGlyAsp

3310 3330 3350
AAGGTGATGTCTGAATGCAGCCTGGAGAAGAACGAGAGGGCTTGCATTGACTTCGCCATC
LysValMetSerGluCysSerLeuGluLysAsnGluArgAlaCysIleAspPheAlaIle

### Sequenzprotokoll:

- Sequenz Nr.:: 27980/11
- Int. Code:: pR14-5.3.3.1, entire clone
- Länge:: 6232 bp
- Typ:: DNA + Aminosäure

pR14-5.3.3.1, cont.

3370 3390 3410
AGCGCCAAACCCCTGACACGGTACATGCCCCAAAACCGGCAGTCGTTCCAGTATAAGACg
SerAlaLysProLeuThrArgTyrMetProGlnAsnArgGlnSerPheGlnTyrLysThr

3430 3450 3470
TGGACATTTGTGGTCTCCCCGCCCTTTGAATACTTCATCATGGCCATGATaGCCCTCAAC
TrpThrPheValValSerProProPheGluTyrPheIleMetAlaMetIleAlaLeuAsn

3490 3510 3530
ACTGTGGTgCTgATgATGAAGTTCTATGATGCACCCTATGAGTACgAGCTGATGCTGAAA
ThrValValLeuMetMetLysPheTyrAspAlaProTyrGluTyrGluLeuMetLeuLys

3550 3570 3590
TGCCTGAACATCGTGTTCACATCCATGTTCTCCATGGAATGCGTGCTGAAGATCATCGCC
CysLeuAsnIleValPheThrSerMetPheSerMetGluCysValLeuLysIleIleAla

3610 3630 3650
TTTgGGGTGCTGAACTATTTCAGAGATGCCTGGAATGTCTTTGACTTTGTCACTGTGTTg
PheGlyValLeuAsnTyrPheArgAspAlaTrpAsnValPheAspPheValThrValLeu

3670 3690 3710
GGAAGTATTACTGATATTTTAGTAACAGAGATtGCGGAAACGAACAATTTCATCAACCTC
GlySerIleThrAspIleLeuValThrGluIleAlaGluThrAsnAsnPheIleAsnLeu

3730 3750 3770
AGCTTCCTCCGcCTCTTTCGAGCTGNGCGGCTGATCAAGCTGCTCcGGCAGGGCTACACc
SerPheLeuArgLeuPheArgAlaXxxArgLeuIleLysLeuLeuArgGlnGlyTyrThr

### Sequenzprotokoll:

- Sequenz Nr.:: 27980/11
- Int. Code:: pR14-5.3.3.1, entire clone
- Länge:: 6232 bp
- Typ:: DNA + Aminosäure

pR14-5.3.3.1, cont.

3790 3810 3830
ATCCGCATCCTGCTGTGGACCTTTGTCCAGTCcTTCAAGGCCCTGCCCTACGTGTGTCTG
IleArgIleLeuLeuTrpThrPheValGlnSerPheLysAlaLeuProTyrValCysLeu

3850 3870 3890
CTCATTGCCATGCTGTTCTTCATCTACGCCATCATCGGCATGCAGGTGTTTGGGAATATT
LeuIleAlaMetLeuPhePheIleTyrAlaIleIleGlyMetGlnValPheGlyAsnIle

3910 3930 3950
GCCCTGGATGATGACACCAGCATCAACCGCCACAACAACTTCCGGACGTTTTTGCAAGCC
AlaLeuAspAspAspThrSerIleAsnArgHisAsnAsnPheArgThrPheLeuGlnAla

3970 3990 4010
CTGATgCTGCTGTTCAGGAGCGCCACGGGGGAGGCCTGGCACGAGATCATGCTGTCCTGC
LeuMetLeuLeuPheArgSerAlaThrGlyGluAlaTrpHisGluIleMetLeuSerCys

4030 4050 4070
CTGAGCAACCAGGCCTGTGATGAGCAGGCCAATGCCACCGAGTGTGGAAGTGACTTTGCC
LeuSerAsnGlnAlaCysAspGluGlnAlaAsnAlaThrGluCysGlySerAspPheAla

4090 4110 4130
TACTTCTACTTCGTCTCCTTCATCTTCCTGTGCTCCTTtCTGATGTTGAACCTCTTTGTG
TyrPheTyrPheValSerPheIlePheLeuCysSerPheLeuMetLeuAsnLeuPheVal

4150 4170 4190
GCtGTGATCATGGACAATTTTGAGTACCTCACGCGGGACTCTTCCATCCTAGGTcCTCAC
AlaValIleMetAspAsnPheGluTyrLeuThrArgAspSerSerIleLeuGlyProHis

### Sequenzprotokoll:

- Sequenz Nr.:: 27980/11
- Int. Code:: pR14-5.3.3.1, entire clone
- Länge :: 6232 bp
- Typ:: DNA + Aminosäure

pR14-5.3.3.1, cont.

4210 4230 4250
CACTTGGATGAGTTCATCCGGGTCTgGGCTGAATACGACCCGGctGcGTGTGGGCGCATC
HisLeuAspGluPheIleArgValTrpAlaGluTyrAspProAlaAlaCysGlyArgIle

4270 4290 4310
AGTTACAATGACATGTTTGAGATGCTGAAACACATGTCCCCGCCTCTGGGGCTGGGGAAG
SerTyrAsnAspMetPheGluMetLeuLysHisMetSerProProLeuGlyLeuGlyLys

4330 4350 4370
AAATGCCCTGCTCGAGTTGCTTACAAGCGCCTGGTTCGCATGAACATGCCCATCTCCAAC
LysCysProAlaArgValAlaTyrLysArgLeuValArgMetAsnMetProIleSerAsn

4390 4410 4430
GAGGACATGACTGTTCACTTCACGTCCACGCTGATGGCCCTCATCCGGACGGCACTGGAG
GluAspMetThrValHisPheThrSerThrLeuMetAlaLeuIleArgThrAlaLeuGlu

4450 4470 4490
ATCAAGCTGGCCCCAGCTGGGACAAAGCAGCATCAGTGTGACGCGGAGTTGAGGAAGGAG
IleLysLeuAlaProAlaGlyThrLysGlnHisGlnCysAspAlaGluLeuArgLysGlu

4510 4530 4550
ATTTCCGTTGTGTGGGCCAATCTGCCCCAGAAGACTTTGGACTTGCTGGTACCACCCCAT
IleSerValValTrpAlaAsnLeuProGlnLysThrLeuAspLeuLeuValProProHis

4570 4590 4610
AAGCCTGATGAGATGACAGTGGGGAAGGTTTATGCAGCTCTGATGATATTTGACTTCTAC
LysProAspGluMetThrValGlyLysValTyrAlaAlaLeuMetIlePheAspPheTyr

### Sequenzprotokoll:

- Sequenz Nr.:: 27980/11
- Int Code:: pR14-5.3.3.1, entire clone
- Länge:: 6232 bp
- Typ:: DNA + Aminosäure

pR14-5.3.3.1, cont.

4630 4650 4670
AAGCAGAACAAAACCACCAGAGACCAGATGCAGCAGGCTCCTGGAGGCCTCTCCCAGATg
LysGlnAsnLysThrThrArgAspGlnMetGlnGlnAlaProGlyGlyLeuSerGlnMet

4690 4710 4730
GGTCCTGTGTCCCTGTTCCACCCTCTGAAGGCCACCCTGGAGCAGACACAGCCGGCTGTg
GlyProValSerLeuPheHisProLeuLysAlaThrLeuGluGlnThrGlnProAlaVal

4750 4770 4790
CTCCGAGGAGCCCGGGTTTTcCTTCGACAGAAGAGTTCCACCTCCCTCAGCAATGGCGGG
LeuArgGlyAlaArgValPheLeuArgGlnLysSerSerThrSerLeuSerAsnGlyGly

4810 4830 4850
GCCATACAAAACCAAGAGAGTGGCATCAaAGAGTCTGTCTCCTGGGGCACTCAAAGGACC
AlaIleGlnAsnGlnGluSerGlyIleLysGluSerValSerTrpGlyThrGlnArgThr

4870 4890 4910
CAGGATGCACCCCATGAGGCCAGGCCACCCCTGGAGCGTGGCCACTCCACAGAGATCCCT
GlnAspAlaProHisGluAlaArgProProLeuGluArgGlyHisSerThrGluIlePro

4930 4950 4970
GTGGGGCGGTCAGGAGCACTGGCTGTGGACGTTCAGATGCAGAGCATAACCCGGAGGGGC
ValGlyArgSerGlyAlaLeuAlaValAspValGlnMetGlnSerIleThrArgArgGly

4990 5010 5030
CCTGATGGGGAGCCCCAGCCTGGGCTGGAGAGCCAGGGTCGAGCGGCCTCCATGCCCCGC
ProAspGlyGluProGlnProGlyLeuGluSerGlnGlyArgAlaAlaSerMetProArg

### Sequenzprotokoll:

- Sequenz Nr. :: 27980/11
- Int. Code:: pR14-5.3.3.1, entire clone
- Länge:: 6232 bp
- Typ:: DNA + Aminosäure

pR14-5.3.3.1, cont.

5050 5070 5090
CTTGCGGCCGAGACTCAGCCCGTCACAGATGCCAGCCCCATGAAGCGCTCCATCTCCACG
LeuAlaAlaGluThrGlnProValThrAspAlaSerProMetLysArgSerIleSerThr

5110 5130 5150
CTGGCCCAGCGGCCCCGTGGGACTCATCTTTGCAGCACCACCCCGGACCGCCCACCCCCT
LeuAlaGlnArgProArgGlyThrHisLeuCysSerThrThrProAspArgProProPro

5170 5190 5210
AGCCAGGCGTCGTCGCACCACCACCACCACCGCTGCCACCGCCGCAGGGACAGGAAGCAG
SerGlnAlaSerSerHisHisHisHisHisArgCysHisArgArgArgAspArgLysGln

5230 5250 5270
AGGTCCCTGGAGAAGGGGCCCAGCCTGTCTGCCGATATGGATGGCGCACCAAGCAGTGCT
ArgSerLeuGluLysGlyProSerLeuSerAlaAspMetAspGlyAlaProSerSerAla

5290 5310 5330
GTGGGGCCGGGGCTGCCCCCGGGAGAGGGGCCTACAGGCTGCCGGCGGGAACGAGAGCGC
ValGlyProGlyLeuProProGlyGluGlyProThrGlyCysArgArgGluArgGluArg

5350 5370 5390
CGGCAGGAGCGGGGCCGGTCCCAGGAGCGGAGGCAGCCCTCATCCTCCTCCTCGGAGAAG
ArgGlnGluArgGlyArgSerGlnGluArgArgGlnProSerSerSerSerSerGluLys

5410 5430 5450
CAGCGCTTCTACTCCTGCGACCGCTTTGGGGGCCGTGAGCCCCCGAAGCCCAAGCCCTCC
GlnArgPheTyrSerCysAspArgPheGlyGlyArgGluProProLysProLysProSer

### Sequenzprotokoll:

- Sequenz Nr.:: 27980/11
- Int. Code:: pR14-5.3.3.1, entire clone
- Länge:: 6232 bp
- Typ:: DNA + Aminosäure

pR14-5.3.3.1, cont.

5470 5490 5510
CTCAGCAGCCACCCAACGTCGCCAACAGCTGGCCAGGAGCCGGGACCCCACCCACAGGGC
LeuSerSerHisProThrSerProThrAlaGlyGlnGluProGlyProHisProGlnGly

5530 5550 5570
AGTGGTTCCGTGAATGGGAGCCCCTTGCTGTCAACATCTGGTGCTAGCACCCCCGGCcGC
SerGlySerValAsnGlySerProLeuLeuSerThrSerGlyAlaSerThrProGlyArg

5590 5610 5630
GGTGGGCGGAGGCAGCTCCCCCAGACGCCCCTGACTCCCCGCCCCAGCATCACCTACAAG
GlyGlyArgArgGlnLeuProGlnThrProLeuThrProArgProSerIleThrTyrLys

5650 5670 5690
ACGGCCAACTCCTCACCCATCCACTTCGCCGGGGCTCAGACCAGCCTCCCTGCCTTCTCC
ThrAlaAsnSerSerProIleHisPheAlaGlyAlaGlnThrSerLeuProAlaPheSer

5710 5730 5750
CCAGGCCGGCTCAGCCGTGGGCTTTCCGAACACAACGCCCTGCTGCAGAGAGACCCCCTC
ProGlyArgLeuSerArgGlyLeuSerGluHisAsnAlaLeuLeuGlnArgAspProLeu

5770 5790 5810
AGCCAGCCCCTGGCCCCTGGCTCTCGAATTGGCTCTGACCCTTACCTGGGGCAGcGTCTG
SerGlnProLeuAlaProGlySerArgIleGlySerAspProTyrLeuGlyGlnArgLeu

5830 5850 5870
GACAGTGAGGCCTCTGTCCACGCCCTGCCTGAGGACACGCTCACTTTCGAGGAGGCTGTG
AspSerGluAlaSerValHisAlaLeuProGluAspThrLeuThrPheGluGluAlaVal

### Sequenzprotokoll:

- Sequenz Nr.:: 27980/11
- Int. Code:: pR14-5.3.3.1, entire clone
- Länge:: 6232 bp
- Typ:: DNA + Aminosäure

5

pR14-5.3.3.1, cont.

5890 5910 5930
GCCACCAACTCGGGCCGCTCCTCCAGGACTTCCTACGTGTCCTCCCTGACCTCCCAGTCT
AlaThrAsnSerGlyArgSerSerArgThrSerTyrValSerSerLeuThrSerGlnSer

5950 5970 5990
CACCCTCTCCGCCGCGTGCCCAACGGTTACCACTGCACCCTGGGACTCAGCTCGGGTGGC
HisProLeuArgArgValProAsnGlyTyrHisCysThrLeuGlyLeuSerSerGlyGly

6010 6030 6050
CGAGCACGGCACAGCTACCACCACCCTGACCAAGACCACTGGTGCT[?]AGCTGCACCGTGA[?]
ArgAlaArgHisSerTyrHisHisProAspGlnAspHisTrpCysEnd

6070 6090 6110
CGCTCAGACGCCTGCATGCAGCAGGCGTGTGTTCCAGTGGATGAGTTTTATCATCCACAC

6130 6150 6170
GGGGCAGTCGGCCCTCGGGGGAGGCCTTGCCCACCTTGGTGAGGCTCCTGTGGCC[?]CCTCC

6190 6210 6230
CTCCCCCTCCTCCCCTCTTTTACTCTAGACGACGAATAAAGCCCTGTTAGAG

35

### Sequenzprotokoll:

- Sequenz Nr.:: 27980/12
- Int. Code:: pCA3
- Länge:: 2.980 bp
- Typ:: DNA + Aminosäure

10                    30                    50

GAATTCTCAATCTCTCATCTTATGCAAAAATCAACTCAAGATGGATCAAAAGACCTAAAT
GluPheSerIleSerHisLeuMetGlnLysSerThrGlnAspGlySerLysAspLeuAsn

70                    90                    110

CCAAGACCTGAAACCATAAAGATACTAGAAGGTAACATCAGAATAATCTTCAAGACATTG
ProArgProGluThrIleLysIleLeuGluGlyAsnIleArgIleIlePheLysThrLeu

130                   150                   170

GCTTAGGCAAAGACTTTTTGACCAAAAACCCAAAAGAACAACAAACCAGAAGTCAACCAG
AlaEndAlaLysThrPheEndProLysThrGlnLysAsnAsnLysProGluValAsnGln

190                   210                   230

ATAGCCAACAGTGACAACAAGGTTACAATTGATGACTATAGAGAAGAGGATGAAGACAAG
IleAlaAsnSerAspAsnLysValThrIleAspAspTyrArgGluGluAspGluAspLys

250                   270                   290

GACCCCTATCCGCCTTGCGATGTGCCAGTAGGGGAAGAGGAAGAGGAAGAGGAGGAGGAT
AspProTyrProProCysAspValProValGlyGluGluGluGluGluGluGluGluAsp

310                   330                   350

GAACCTGAGGTTCCTGCCGGACCCCGTCCTCGAAGGATCTCGGAGTTGAACATGAAGGAA
GluProGluValProAlaGlyProArgProArgArgIleSerGluLeuAsnMetLysGlu

370                   390                   410

AAAATTGCCCCCATCCCTGAAGGGAGCGCTTTCTTCATTCTTAGCAAGACCAACCCGATC
LysIleAlaProIleProGluGlySerAlaPhePheIleLeuSerLysThrAsnProIle

430                   450                   470

CGCGTAGGCTGCCACAAGCTCATCAACCACCACATCTTCACCAACCTCATCCTTGTCTTC
ArgValGlyCysHisLysLeuIleAsnHisHisIlePheThrAsnLeuIleLeuValPhe

490                   510                   530

ATCATGCTGAGCAGCGCTGCCCTGGCCGCAGAGGACCCCATCCGCAGCCACTCCTTCCGG
IleMetLeuSerSerAlaAlaLeuAlaAlaGluAspProIleArgSerHisSerPheArg

550                   570                   590

AACACGATACTGGGTTACTTTGACTATGCCTTCACAGCCATCTTTACTGTTGAGATCCTG
AsnThrIleLeuGlyTyrPheAspTyrAlaPheThrAlaIlePheThrValGluIleLeu

610                   630                   650

TTGAAGATGACAACTTTTGGAGCTTTCCTCCACAAAGGGGCCTTCTGCAGGAACTACTTC
LeuLysMetThrThrPheGlyAlaPheLeuHisLysGlyAlaPheCysArgAsnTyrPhe

### Sequenzprotokoll:

- Sequenz Nr.:: 27980/12
- Int. Code:: pCA3
- Länge:: 2.980 bp
- Typ:: DNA + Aminosäure

670 690 710
AATTTGCTGGATATGCTGGTGGTTGGGGTGTCTCTGGTGTCATTTGGGATTCAATCCAGT
AsnLeuLeuAspMetLeuValValGlyValSerLeuValSerPheGlyIleGlnSerSer

730 750 770
GCCATCTCCGTTGTGAAGATTCTGAGGGTCTTAAGGGTCCTGCGTCCCCTCAGGGCCATC
AlaIleSerValValLysIleLeuArgValLeuArgValLeuArgProLeuArgAlaIle

790 810 830
AACAGAGCAAAAGGACTTAAGCACGTGGTCCAGTGCGTCTTCGTGGCCATCCGGACCATC
AsnArgAlaLysGlyLeuLysHisValValGlnCysValPheValAlaIleArgThrIle

850 870 890
GGCAACATCATGATCGTCACCACCCTCCTGCAGTTCATGTTTGCCTGTATCGGGGTCCAG
GlyAsnIleMetIleValThrThrLeuLeuGlnPheMetPheAlaCysIleGlyValGln

910 930 950
TTGTTCAAGGGGAAGTTCTATCGCTGTACGGATGAAGCCAAAAGTAACCCTGTTGAATGC
LeuPheLysGlyLysPheTyrArgCysThrAspGluAlaLysSerAsnProValGluCys

970 990 1010
AGGGGACTTTTCATCCTCTACAAGGATGGGGATGTTGACAGTGGTGTGGTCCGTGAACGG
ArgGlyLeuPheIleLeuTyrLysAspGlyAspValAspSerGlyValValArgGluArg

1030 1050 1070
ACTCGGCAAAACAGTGATTTCAACTTCGACAACGTCCTCTCTGCTATGATGGCGCTCTTC
ThrArgGlnAsnSerAspPheAsnPheAspAsnValLeuSerAlaMetMetAlaLeuPhe

1090 1110 1130
ACAGTCTCCACGTTTGAGGGCTGGCCTCGGTTGCTGTATAAAGCCATCGACTCGAATGGA
ThrValSerThrPheGluGlyTrpProArgLeuLeuTyrLysAlaIleAspSerAsnGly

1150 1170 1190
GAGAACATCGGCCCAATCTACAACCACCGCGTGGAGATCTCCATCTTCTTCATCATCTAC
GluAsnIleGlyProIleTyrAsnHisArgValGluIleSerIlePhePheIleIleTyr

1210 1230 1250
ATCATCATTGTAGCTTTCTTCATGATGAACATCTTTGTGGGCTTTGTCATCGTTACATTT
IleIleIleValAlaPhePheMetMetAsnIlePheValGlyPheValIleValThrPhe

1270 1290 1310
CAGGAACAAGGAGAAAAAGAGTATAAGAACTGTGAGCTGGACAAAAATCAGCGTCAGTGT
GlnGluGlnGlyGluLysGluTyrLysAsnCysGluLeuAspLysAsnGlnArgGlnCys

### Sequenzprotokoll:

- Sequenz Nr.:: 27980/12
- Int. Code:: pCA3
- Länge:: 2.980 bp
- Typ:: DNA + Aminosäure

1330 1350 1370

GTTGAATACGCCTTGAAAGCACGTCCTTTGCGGAGATACATCCCCAAAAACCCCTACCAG
ValGluTyrAlaLeuLysAlaArgProLeuArgArgTyrIleProLysAsnProTyrGln

1390 1410 1430

TACAAGTTCTGGTACGCGGCGAACTCTTCGCCTTTCGAATACATGATGTTTGTCCTCATC
TyrLysPheTrpTyrAlaAlaAsnSerSerProPheGluTyrMetMetPheValLeuIle

1450 1470 1490

ATGCTCAACACACTCTGCTTGGCCATGCAGCACTACGAGCAGTCCAAGATGTTCAATGAT
MetLeuAsnThrLeuCysLeuAlaMetGlnHisTyrGluGlnSerLysMetPheAsnAsp

1510 1530 1550

GCCATGGACATTCTGAACATGGTCTTCACCGGGGTGTTCACCGTCGAGATGGTTTTGAAA
AlaMetAspIleLeuAsnMetValPheThrGlyValPheThrValGluMetValLeuLys

1570 1590 1610

GTCATCGCATTTAAGCCTAAGGGGTATTTTAGTGACGCCTGGAACACGTTTAGCTCCCTC
ValIleAlaPheLysProLysGlyTyrPheSerAspAlaTrpAsnThrPheSerSerLeu

1630 1650 1670

ATCGTAATCGGCAGCATTATAGACGTCGCCCTCAGCGAAGCAGACCACTATTTCACTGAT
IleValIleGlySerIleIleAspValAlaLeuSerGluAlaAspHisTyrPheThrAsp

1690 1710 1730

GCATGGAACACTTTTGATGCCTTAATTGTTGTTGGTAGCGTCGTTGATATTGCTATAACT
AlaTrpAsnThrPheAspAlaLeuIleValValGlySerValValAspIleAlaIleThr

1750 1770 1790

GAAGTGAATCCAACTGAAAGTGAAAATGTCCCTGTCCCAACTGCTACACCTGGGAACTCT
GluValAsnProThrGluSerGluAsnValProValProThrAlaThrProGlyAsnSer

1810 1830 1850

GAAGAGAGCAATAGAATCTCCATCACCTTTTTCCGTCTTTTCCGAGTGATGCGATTGGTG
GluGluSerAsnArgIleSerIleThrPhePheArgLeuPheArgValMetArgLeuVal

1870 1890 1910

AAGCTTCTCAGCAGGGGGGAAGGCATCCGGACATTGCTGTGGACTTTTATTAAGTCCTTT
LysLeuLeuSerArgGlyGluGlyIleArgThrLeuLeuTrpThrPheIleLysSerPhe

1930 1950 1970

CAGGCGCTCCCGTATGTGGCCCTCCTCATAGCCATGCTGTTCTTCATCTATGCGGTCATT
GlnAlaLeuProTyrValAlaLeuLeuIleAlaMetLeuPhePheIleTyrAlaValIle

### Sequenzprotokoll:

- Sequenz Nr.:: 27980/12
- Int. Code:: pCA3
- Länge:: 2.980 bp
- Typ:: DNA + Aminosäure

1990 2010 2030
GGCATGCAGATGTTTGGGAAAGTTGCCATGAGAGATAACAACCAGATCAATAGGAACAAT
GlyMetGlnMetPheGlyLysValAlaMetArgAspAsnAsnGlnIleAsnArgAsnAsn

2050 2070 2090
AACTTCCAGACGTTTCCCCAGGCGGTGCTGCTGCTCTTCAGGTGTGCAACAGGTGAGGCC
AsnPheGlnThrPheProGlnAlaValLeuLeuLeuPheArgCysAlaThrGlyGluAla

2110 2130 2150
TGGCAGGAGATCATGCTGGCCTGTCTCCCAGGGAAGCTCTGTGACCCTGAGTCAGATTAC
TrpGlnGluIleMetLeuAlaCysLeuProGlyLysLeuCysAspProGluSerAspTyr

2170 2190 2210
AACCCCGGGGAGGAGTATACATGTGGGAGCAACTTTGCCATTGTCTATTTCATCAGTTTT
AsnProGlyGluGluTyrThrCysGlySerAsnPheAlaIleValTyrPheIleSerPhe

2230 2250 2270
TACATGCTCTGTGCATTTCTGATCATCAATCTGTTTGTGGCTGTCATCATGGATAATTTC
TyrMetLeuCysAlaPheLeuIleIleAsnLeuPheValAlaValIleMetAspAsnPhe

2290 2310 2330
GACTATCTGACCCGGGACTGGTCTATTTTGGGGCCTCACCATTTAGATGAATTCAAAAGA
AspTyrLeuThrArgAspTrpSerIleLeuGlyProHisHisLeuAspGluPheLysArg

2350 2370 2390
ATATGGTCAGAATATGACCCTGAGGCAAAGGGAAGGATAAAACACCTTGATGTGGTCACT
IleTrpSerGluTyrAspProGluAlaLysGlyArgIleLysHisLeuAspValValThr

2410 2430 2450
CTGCTTCGACGCATCCAGCCTCCCCTGGGGTTTGGGAAGTTATGTCCACACAGGGTAGCG
LeuLeuArgArgIleGlnProProLeuGlyPheGlyLysLeuCysProHisArgValAla

2470 2490 2510
TGCAAGAGATTAGTTGCCATGAACATGCCTCTCAACAGTGACGGGACAGTCATGTTTAAT
CysLysArgLeuValAlaMetAsnMetProLeuAsnSerAspGlyThrValMetPheAsn

2530 2550 2570
GCAACCCTGTTTGCTTTGGTTCGAACGGCTCTTAAGATCAAGACCGAAGGGAACCTGGAG
AlaThrLeuPheAlaLeuValArgThrAlaLeuLysIleLysThrGluGlyAsnLeuGlu

2590 2610 2630
CAAGCTAATGAAGAACTTCGGGCTGTGATAAAGAAAATTTGGAAGAAAACCAGCATGAAA
GlnAlaAsnGluGluLeuArgAlaValIleLysLysIleTrpLysLysThrSerMetLys

### Sequenzprotokoll:

- Sequenz Nr.:: 27980/12
- Int. Code:: pCA3
- Länge:: 2.980 bp
- Typ:: DNA + Aminosäure

2650 2670 2690
TTACTTGACCAAGTTGTCCCTCCAGCTGGTGATGATGAGGTAACCGTGGGGAAGTTCTAT
LeuLeuAspGlnValValProProAlaGlyAspAspGluValThrValGlyLysPheTyr

2710 2730 2750
GCCACTTTCCTGATACAGGACTACTTTAGGAAATTCAAGAAACGGAAAGAACAAGGACTG
AlaThrPheLeuIleGlnAspTyrPheArgLysPheLysLysArgLysGluGlnGlyLeu

2770 2790 2810
GTGGGAAAGATCCCTGCGAAGAACACCACAATTGCCCTACAGGCGGGATTAAGGACACTG
ValGlyLysIleProAlaLysAsnThrThrIleAlaLeuGlnAlaGlyLeuArgThrLeu

2830 2850 2870
CATGACATTGGGCCAGAAATCCGGCGTGCTATATCGTGTGATTTGCAAGATGACGAGCCT
HisAspIleGlyProGluIleArgArgAlaIleSerCysAspLeuGlnAspAspGluPro

2890 2910 2930
GAGGAAACAAAACGAGAAGAAGAAGATGATGTGTTCAAAGTAATTATTCCACGCCTAGCT
GluGluThrLysArgGluGluGluAspAspValPheLysValIleIleProArgLeuAla

2950 2970
ACACACTGGCCATCTGGAAATAGCAGGGCAGGCCGAATTC
ThrHisTrpProSerGlyAsnSerArgAlaGlyArgIle

### Sequenzprotokoll:

- Sequenz Nr.:: 27980/13
- Int. Code:: pCA9.3
- Länge:: 1.857 bp
- Typ:: DNA + Aminosäure

10                    30                    50

TCAAAGGAAAGAGAGAAGGCAAAAGCACGGGGAGATTTCCAGAAGCTCCGGGAGAAGCAG
SerLysGluArgGluLysAlaLysAlaArgGlyAspPheGlnLysLeuArgGluLysGln

70                    90                    110

CAGCTGGAGGAGGATCTAAAGGGCTACTTGGATTGGATCACCCAAGCTGAGGACATCGAT
GlnLeuGluGluAspLeuLysGlyTyrLeuAspTrpIleThrGlnAlaGluAspIleAsp

130                   150                   170

CCGGAGAATGAGGAAGAAGGAGGAGAGGAAGGCAAACGAAATACTAGCATGCCCACCAGC
ProGluAsnGluGluGluGlyGlyGluGluGlyLysArgAsnThrSerMetProThrSer

190                   210                   230

GAGACTGAGTCTGTGAACACAGAGAACGTCAGCGGTGAAGGCGAGAACCGAGGCTGCTGT
GluThrGluSerValAsnThrGluAsnValSerGlyGluGlyGluAsnArgGlyCysCys

250                   270                   290

GGAAGTCTCTGGTGCTGGTGGAGACGGAGAGGCGCGGCAAGGCGGGGCCTCTGGTGTCGG
GlySerLeuTrpCysTrpTrpArgArgArgGlyAlaAlaArgArgGlyLeuTrpCysArg

310                   330                   350

CGGTGGGGTCAAGCCATCTCAAAATCCAAACTCAGCCGACGCTGGCGCTGGAACCGATTC
ArgTrpGlyGlnAlaIleSerLysSerLysLeuSerArgArgTrpArgTrpAsnArgPhe

370                   390                   410

AATCGCAGAAGATGTAGGGCCGCCGTGAAGTCTGTCACGTTTTACTGGCTGGTTATCGTC
AsnArgArgArgCysArgAlaAlaValLysSerValThrPheTyrTrpLeuValIleVal

430                   450                   470

CTGGTGTTTCTGAACACCTTAACCATTTCCTCTGAGCACTACAATCAGCCGAGTTGGTTG
LeuValPheLeuAsnThrLeuThrIleSerSerGluHisTyrAsnGlnProSerTrpLeu

490                   510                   530

ACACAGATTCAAGATATTGCCAACAAAGTCCTCTTGGCTCTGTTCACCTGCGAGATGCTG
ThrGlnIleGlnAspIleAlaAsnLysValLeuLeuAlaLeuPheThrCysGluMetLeu

550                   570                   590

GTAAAAATGTACAGCTTGGGCCTCCAAGCATATTTCATCTCTCTTTTCAACCGGTTTGAT
ValLysMetTyrSerLeuGlyLeuGlnAlaTyrPheIleSerLeuPheAsnArgPheAsp

610                   630                   650

TGCTTCGTGGTGTGTGGTGGAATCACTGAGACGATCTTGGTGGAACTGGAAATCATGTCT
CysPheValValCysGlyGlyIleThrGluThrIleLeuValGluLeuGluIleMetSer

### Sequenzprotokoll:

- Sequenz Nr.:: 27980/13
- Int. Code:: pCA9.3
- Länge:: 1.857 bp
- Typ:: DNA + Aminosäure

670 690 710
CCCCTGGGGATCTCTGTGTTTCGGTGTGTGCGCCTCTTAAGAATCTTCAAAGTGACCAGG
ProLeuGlyIleSerValPheArgCysValArgLeuLeuArgIlePheLysValThrArg

730 750 770
CACTGGACTTCCCTGAGCAACTTAGTGGCATCCTTATTAAACTCCATGAAGTCCATCGCT
HisTrpThrSerLeuSerAsnLeuValAlaSerLeuLeuAsnSerMetLysSerIleAla

790 810 830
TCGCTGTTGCTTCTGCTTTTTCTCTTCATTATCATCTTTTCCTTGCTTGGGATGCAGCTG
SerLeuLeuLeuLeuLeuPheLeuPheIleIleIlePheSerLeuLeuGlyMetGlnLeu

850 870 890
TTTGGCGGCAAGTTTAATTTTGATGAAACGCAAACCAAGCAAGCAGCCTTTGACAATTTC
PheGlyGlyLysPheAsnPheAspGluThrGlnThrLysGlnAlaAlaPheAspAsnPhe

910 930 950
CCTCAAGCACTTCTCACAGTGTTCCAGATCCTGACAGGCGAAGACTGGAATGCTGTGATG
ProGlnAlaLeuLeuThrValPheGlnIleLeuThrGlyGluAspTrpAsnAlaValMet

970 990 1010
TACGATGGCATCATGGCTTACGGGGGCCCATCCTCTTCAGGAATGATCGTCTGCATCTAC
TyrAspGlyIleMetAlaTyrGlyGlyProSerSerSerGlyMetIleValCysIleTyr

1030 1050 1070
TTCATCATCCTCTTCATTTGTGGTAACTATATTCTACTGAATGTCTTCTTGGCCATCGCT
PheIleIleLeuPheIleCysGlyAsnTyrIleLeuLeuAsnValPheLeuAlaIleAla

1090 1110 1130
GTAGACAATTTGGCTGATGCTGAAAGTCTGAACACTGCTCAGAAAGAAGAAGCGGAAGAA
ValAspAsnLeuAlaAspAlaGluSerLeuAsnThrAlaGlnLysGluGluAlaGluGlu

1150 1170 1190
AAGGAGAGGAAAAAGATTGCCAGAAAAGAGAGCCTAGAAAATAAAAAGAACAACAAACCA
LysGluArgLysLysIleAlaArgLysGluSerLeuGluAsnLysLysAsnAsnLysPro

1210 1230 1250
GAAGTCAACCAGATAGCCAACAGTGACAACAAGGTTACAATTGATGACTATAGAGAAGAG
GluValAsnGlnIleAlaAsnSerAspAsnLysValThrIleAspAspTyrArgGluGlu

### Sequenzprotokoll:

- Sequenz Nr.:: 27980/13
- Int. Code:: pCA9.3
- Länge:: 1.857 bp
- Typ:: DNA + Aminosäure

1270 1290 1310
GATGAAGACAAGGACCCCTATCCGCCTTGCGATGTGCCAGTAGGGGAAGAGGAAGAGGAA
AspGluAspLysAspProTyrProProCysAspValProValGlyGluGluGluGluGlu

1330 1350 1370
GAGGAGGAGGATGAACCTGAGGTTCCTGCCGGACCCCGTCCTCGAAGGATCTCGGAGTTG
GluGluGluAspGluProGluValProAlaGlyProArgProArgArgIleSerGluLeu

1390 1410 1430
AACATGAAGGAAAAAATTGCCCCCATCCCTGAAGGGAGCGCTTTCTTCATTCTTAGCAAG
AsnMetLysGluLysIleAlaProIleProGluGlySerAlaPhePheIleLeuSerLys

1450 1470 1490
ACCAACCCGATCCGCGTAGGCTGCCACAAGCTCATCAACCACCACATCTTCACCAACCTC
ThrAsnProIleArgValGlyCysHisLysLeuIleAsnHisHisIlePheThrAsnLeu

1510 1530 1550
ATCCTTGTCTTCATCATGCTGAGCAGCGCTGCCCTGGCCGCAGAGGACCCCATCCGCAGC
IleLeuValPheIleMetLeuSerSerAlaAlaLeuAlaAlaGluAspProIleArgSer

1570 1590 1610
CACTCCTTCCGGAACACGATACTGGGTTACTTTGACTATGCCTTCACAGCCATCTTTACT
HisSerPheArgAsnThrIleLeuGlyTyrPheAspTyrAlaPheThrAlaIlePheThr

1630 1650 1670
GTTGAGATCCTGTTGAAGATGACAACTTTTGGAGCTTTCCTCCACAAAGGGGCCTTCTGC
ValGluIleLeuLeuLysMetThrThrPheGlyAlaPheLeuHisLysGlyAlaPheCys

1690 1710 1730
AGGAACTACTTCAATTTGCTGGATATGCTGGTGGTTGGGGTGTCTCTGGTGTCATTTGGG
ArgAsnTyrPheAsnLeuLeuAspMetLeuValValGlyValSerLeuValSerPheGly

1750 1770 1790
ATTCAATCCAGTGCCATCTCCGTTGTGAAGATTCTGAGGGTCTTAAGGGTCCTGCGTCCC
IleGlnSerSerAlaIleSerValValLysIleLeuArgValLeuArgValLeuArgPro

1810 1830 1850
CTCAGGGCCATCAACAGAGCAAAAGGACTTAAGCACGTGGTCCAGTGCGTCTTCGTG
LeuArgAlaIleAsnArgAlaLysGlyLeuLysHisValValGlnCysValPheVal

### Sequenzprotokoll:

- Sequenz Nr.:: 27980/14
- Int. Code:: p1247-4.2.1.1 entire clone
- Länge:: 920 bp
- Typ:: DNA + Aminosäure

10                    30                    50
ATTTCCGCNATGCCTGGAACATCTTCGACTTTGTGACTGTTCTGGGCAGCATCACCGATATC
PheArgXxxAlaTrpAsnIlePheAspPheValThrValLeuGlySerIleThrAspIle

70                    90                    110
CTCGTGACTGAGTTTGGGAATCCGAATAACTTCATCAACCTGAGCTTTCTCCGnnnnTTC
LeuValThrGluPheGlyAsnProAsnAsnPheIleAsnLeuSerPheLeuArgXxxPhe

130                   150                   170
CGAGCTGCCCGGctcATCAAACTTCTCCGTCAGGGTTACACCATCCGcATTCTTCTCTGG
ArgAlaAlaArgLeuIleLysLeuLeuArgGlnGlyTyrThrIleArgIleLeuLeuTrp

190                   210                   230
ACCTTTGTGCAGTCCTTCAAGGCCCTGCCTTATGTCTGTCTGCTGATCGCCATGCTCTTC
ThrPheValGlnSerPheLysAlaLeuProTyrValCysLeuLeuIleAlaMetLeuPhe

250                   270                   290
TTCATCTATGCCATCATTGGGATGCAGGTGTTTGGTAACATTGGCATCGACGTGGAGGAC
PheIleTyrAlaIleIleGlyMetGlnValPheGlyAsnIleGlyIleAspValGluAsp

310                   330                   350
GAGGACAGTGATGAAGATGAGTTCCAAATCACTGAGCACAATAACTTCCGGACCTTCTTC
GluAspSerAspGluAspGluPheGlnIleThrGluHisAsnAsnPheArgThrPhePhe

370                   390                   410
CAGGCCCTCATGCTTCTCTTCCGGAGTGCCACCGGGGAAGCTTGGCACAACATCATGCTT
GlnAlaLeuMetLeuLeuPheArgSerAlaThrGlyGluAlaTrpHisAsnIleMetLeu

### Sequenzprotokoll:

- Sequenz Nr.:: 27980/14
- Int. Code:: p1247-4.2.1.1 entire clone
- Länge:: 920 bp
- Typ:: DNA + Aminosäure

430 450 470
TCCTGCCTCAGCGGGAAACCGTGTGATAAGAACTCTNgCATCCTGACTCGAGAGTGTGGC
SerCysLeuSerGlyLysProCysAspLysAsnSerXxxIleLeuThrArgGluCysGly

490 510 530
AATGAATTTGCTTATTTTacTTTGTTTCCTTCATCTTCCTCTGCTCGTTTCTGATGCTG
AsnGluPheAlaTyrPheTyrPheValSerPheIlePheLeuCysSerPheLeuMetLeu

550 570 590
AATCTCTTTGTCGCCGTCATCATGGACAACTTTGAGTACCTCACCCGAGACTCCTCCATC
AsnLeuPheValAlaValIleMetAspAsnPheGluTyrLeuThrArgAspSerSerIle

610 630 650
CTGGGCCCCCACCACCTGGATGAGTACGTGCGTGTCTGGGCCGAGTATGACCCCGCAGCT
LeuGlyProHisHisLeuAspGluTyrValArgValTrpAlaGluTyrAspProAlaAla

670 690 710
TGGGGCCGCATGCCTTACCTGGACATGTATCAGATGcTGAGACACATGTCTCCGNcCCTG
TrpGlyArgMetProTyrLeuAspMetTyrGlnMetLeuArgHisMetSerProXxxLeu

730 750 770
GGTCTGGGGAAGAAGTGTCCGGcCAGAGTGGCTTACAAGCGGCTTCTGCGGATGGACCTG
GlyLeuGlyLysLysCysProAlaArgValAlaTyrLysArgLeuLeuArgMetAspLeu

790 810 830
CCCGTCGCAGATGACAACACCGTCCACTTCAATTCCACCCTCATGGCTCTGATCCGCACA
ProValAlaAspAspAsnThrValHisPheAsnSerThrLeuMetAlaLeuIleArgThr

### Sequenzprotokoll:

- Sequenz Nr.:: 27980/14
- Int. Code:: p1247-4.2.1.1 entire clone
- Länge:: 920 bp
- Typ:: DNA + Aminosäure

850 870 890
GCCCTGGACATCAAGATTGCCAAGGGAGGAGCCGACAAACAGCAGATGGACGCTGAGCTG
AlaLeuAspIleLysIleAlaLysGlyGlyAlaAspLysGlnGlnMetAspAlaGluLeu

910
CGGAAGGAGATGATGGCG
ArgLysGluMetMetAla

### Sequenzprotokoll:

- Sequenz Nr.:: 27980/15
- Int. Code:: pR5-6cort, entire clone
- Länge:: 1424 bp
- Typ:: DNA + Aminosäure

10 30 50
GGGCGAAGCTACCATCTGTGGATTATGACTGAACGCCTCTAAGTCAGAATCCCGCCCAGGC
LeuAsnAlaSerLysSerGluSerArgProGly

70 90 110
GGAACGATACGGCAGCGCCGCGGAGCCTCGGTTGGCCTCACGGTTAACCGGTCTCTCTTC
GlyThrIleArgGlnArgArgGlyAlaSerValGlyLeuThrValAsnArgSerLeuPhe

130 150 170
CTCTTCAGCGAAGACAACGTGGTGAGAAAATACGCCAAAAAGATCACCGAATGGCCTCCC
LeuPheSerGluAspAsnValValArgLysTyrAlaLysLysIleThrGluTrpProPro

190 210 230
TTTGAATATATGATTTTAGCCACCATCATAGCGAATTGCATCGTCCTcGCACTGGAGCAG
PheGluTyrMetIleLeuAlaThrIleIleAlaAsnCysIleValLeuAlaLeuGluGln

250 270 290
CATCTGCCTGATGATGACAAGACCCCGATGTCTGAACGGCTGGATGACACAGAACCATAC
HisLeuProAspAspAspLysThrProMetSerGluArgLeuAspAspThrGluProTyr

310 330 350
TTCATTGGAATTTTTTGTTTCGAGGCTGGAATTAAAATCATTGCCCTTGGGTTTGCCTTC
PheIleGlyIlePheCysPheGluAlaGlyIleLysIleIleAlaLeuGlyPheAlaPhe

### Sequenzprotokoll:

- Sequenz Nr.:: 27980/15
- Int. Code:: pR5-6cort, entire clone
- Länge:: 1424 bp
- Typ:: DNA + Aminosäure

pR5-6cort, cont.

370 390 410
CACAAAGGCTCCTACTTGAGGAATGGCTGGAATGTCATGGACTTTGTGGTGGTGCTAACG
HisLysGlySerTyrLeuArgAsnGlyTrpAsnValMetAspPheValValValLeuThr

430 450 470
GGCATCTTGGCGACAGTTGGGACGGAGTTTGACCTACGGACGCTGAGGGCAGTTCGAGTG
GlyIleLeuAlaThrValGlyThrGluPheAspLeuArgThrLeuArgAlaValArgVal

490 510 530
CTGCNNCCGCTCAAGCTGGTGTCTGGAATCCCAAgTTTACAAGTCGTCCTGAAGTCGATC
LeuXxxProLeuLysLeuValSerGlyIleProSerLeuGlnValValLeuLysSerIle

550 570 590
ATGAAGGCGATGATCCCTTTNCTGCAGATCGgCCTCCTCCTATTTTTNCAATCCTTATT
MetLysAlaMetIleProXxxLeuGlnIleGlyLeuLeuLeuPhePheXxxIleLeuIle

610 630 650
TTTgCAATCATAGGGTTAGAATTTTATATGGgAAAATTTCATACCACCTGCTTTGAAGAG
PheAlaIleIleGlyLeuGluPheTyrMetGlyLysPheHisThrThrCysPheGluGlu

670 690 710
GGGACAGATGACATTCAGGGTGAGTCTCCGGCTCCATGTGGGACAGAAGAGCCCGCCCGC
GlyThrAspAspIleGlnGlyGluSerProAlaProCysGlyThrGluGluProAlaArg

730 750 770
ACCTGCCCCAATGGGACCAAATGTCAGCCCTACTGGGAAGGGCCCAACAACGGGATCACT
ThrCysProAsnGlyThrLysCysGlnProTyrTrpGluGlyProAsnAsnGlyIleThr

### Sequenzprotokoll:

- Sequenz Nr.:: 27980/15
- Int. Code:: pR5-6cort, entire clone
- Länge:: 1424 bp
- Typ:: DNA + Aminosäure

pR5-6cort, cont.

790 810 830
. . . . . .
CAGTTCGACAACATCCTGTTTGCAGTGCTGACTGTTTTCCAGTGCATAACCATGGAAGGG
GlnPheAspAsnIleLeuPheAlaValLeuThrValPheGlnCysIleThrMetGluGly

850 870 890
. . . . . .
TGGACTGATCTCCTCTACAATAGCAACGATgCCTCAGGGAACACTTGGAACTGGTTGTAC
TrpThrAspLeuLeuTyrAsnSerAsnAspAlaSerGlyAsnThrTrpAsnTrpLeuTyr

910 930 950
. . . . . .
TTCATCCCCCTCATCATCATCGGCTCCTTTTTTATGCTGAACCTTGTGCTGGGTGTGCTG
PheIleProLeuIleIleIleGlySerPhePheMetLeuAsnLeuValLeuGlyValLeu

970 990 1010
. . . . . .
TCAGGGGaGTTTGCCAAAGAAAGGNACGGGTGGAGNACCGGcGGGcTTTTCTGAAGCTG
SerGlyGluPheAlaLysGluArgXxxArgValGluXxxArgArgAlaPheLeuLysLeu

1030 1050 1070
. . . . . .
AGGCGGNaANAACAGATTGAACGTGAGCTCAATGGGTACATGGAATGGATCTCAAAAGCA
ArgArgXxxXxxGlnIleGluArgGluLeuAsnGlyTyrMetGluTrpIleSerLysAla

1090 1110 1130
. . . . . .
GAAGAGGTGATCCTCGCCgAGGATGAAACTGACGGGGAGCAGAGGCATCCCTTTGATGGA
GluGluValIleLeuAlaGluAspGluThrAspGlyGluGlnArgHisProPheAspGly

1150 1170 1190
. . . . . .
GCTCTGCGGAGAACCACCATAAAGAAAAGCAAGACAGATTTGCTCAACCCCGAAGAGGNT
AlaLeuArgArgThrThrIleLysLysSerLysThrAspLeuLeuAsnProGluGluXxx

### Sequenzprotokoll:

- Sequenz Nr.:: 27980/15
- Int. Code:: pR5-6cort, entire clone
- Länge:: 1424 bp
- Typ:: DNA + Aminosäure

pR5-6cort, cont.

1210 1230 1250
. . . . . .
GAGGATCAGCTGGCTGATATAGCCTCTGTGGGTTCTCCCTTCGCCCGAGCCAGCATTAAA
GluAspGlnLeuAlaAspIleAlaSerValGlySerProPheAlaArgAlaSerIleLys

1270 1290 1310
. . . . . .
AGTGCCAAGCTGGAGAACTCGACCTTTTTCACAAAAGGAGAGGAGGATGCGTTTCTAC
SerAlaLysLeuGluAsnSerThrPhePheHisLysLysGluArgArgMetArgPheTyr

1330 1350 1370
. . . . . .
ATCCGCCGCATGGTCAAAACTCAGGCCTTCTACTGGACTGTACTCAGTTTGGTAGCTCTC
IleArgArgMetValLysThrGlnAlaPheTyrTrpThrValLeuSerLeuValAlaLeu

1390 1410
. . . .
AACACGCTGTGTGTTGCTATTGTTCACTACAACCAGCCCGAG
AsnThrLeuCysValAlaIleValHisTyrAsnGlnProGlu

### Sequenzprotokoll:

- Sequenz Nr.:: 27980/16
- Int. Code:: pR5Acort, entire clone
- Länge:: 910 bp
- Typ:: DNA + Aminosäure

10                    30                    50
GGAGAACTCGACCTTTTTCACAAAAAGGAGAGGAGGATGCGTTTCTACATCCGCCGCATG
GluAsnSerThrPhePheHisLysLysGluArgArgMetArgPheTyrIleArgArgMet

70                    90                    110
GTCAAAACTCAGGCCTTCTACTGGACTGTACTCAGTTTGGTAGCTCTCAACACGCTGTGT
ValLysThrGlnAlaPheTyrTrpThrValLeuSerLeuValAlaLeuAsnThrLeuCys

130                   150                   170
GTTGCTATTGTTCACTACAACCAGCCCGAGTGGCTCTCCGACTTCCTTTACTATgCAGAA
ValAlaIleValHisTyrAsnGlnProGluTrpLeuSerAspPheLeuTyrTyrAlaGlu

190                   210                   230
TTCATTTTCTTAGGACTCTTTATGTCCGAAATGTTTATAAAAATGTACGGCTTGGGACG
PheIlePheLeuGlyLeuPheMetSerGluMetPheIleLysMetTyrGlyLeuGlyThr

250                   270                   290
CGGCCTTACTTCCACTCTTCCTTCAACtGCTTTGACTGTGGGGTTATCATTGGGAGCATC
ArgProTyrPheHisSerSerPheAsnCysPheAspCysGlyValIleIleGlySerIle

310                   330                   350
TtCGAGGTCATCTGGGCTGTCATAAaACCTGGCACATCCTTtGGAATCAGCGTGTTACGA
PheGluValIleTrpAlaValIleLysProGlyThrSerPheGlyIleSerValLeuArg

### Sequenzprotokoll:

- Sequenz Nr.:: 27980/16
- Int. Code:: pR5-4cort, entire clone
- Länge:: 910 bp
- Typ:: DNA + Aminosäure

pR5-4cort, cont.

370 390 410
GCCCTCAGGTTATTGCGTATTTTCAAAGTCACAAAGTACtGGGCATCTCTCAGAAACCTG
AlaLeuArgLeuLeuArgIlePheLysValThrLysTyrTrpAlaSerLeuArgAsnLeu

430 450 470
GTCGTCTCTCTCCTCAACTCCATGAAGTCCATCATCAGCCTGTTGTTTCTCCTTTTCCTG
ValValSerLeuLeuAsnSerMetLysSerIleIleSerLeuLeuPheLeuLeuPheLeu

490 510 530
TTCATTGTCGTCTTCGCCCTTTTGGGAATGCAACTCTTCGGCGGCCAGTTTAATTTCGAT
PheIleValValPheAlaLeuLeuGlyMetGlnLeuPheGlyGlyGlnPheAsnPheAsp

550 570 590
gAAGGGACTCCTCCCACCAACTTCGATACTTTTCCAGCAGCAATAATGACGGTGTTTCAG
GluGlyThrProProThrAsnPheAspThrPheProAlaAlaIleMetThrValPheGln

610 630 650
ATCCTGACGGGCGAAGACTGgAACGAGGTCATGTACGACGGGATCAAGTCTCAGGGGGGC
IleLeuThrGlyGluAspTrpAsnGluValMetTyrAspGlyIleLysSerGlnGlyGly

670 690 710
GTGCAGGGCGGCATGGTGTTCTCCATCTATTTCATTGTACTGACGNTCTTTGGGAACTAC
ValGlnGlyGlyMetValPheSerIleTyrPheIleValLeuThrXxxPheGlyAsnTyr

730 750 770
ACCCTCCTGAATGTGTTCTTGGCCATcGCTGTGGACAATCTGGCCAACGCCCAGGAGCTC
ThrLeuLeuAsnValPheLeuAlaIleAlaValAspAsnLeuAlaAsnAlaGlnGluLeu

### Sequenzprotokoll:

- Sequenz Nr.:: 27980/16
- Int. Code:: pR5-4cort, entire clone
- Länge:: 910 bp
- Typ:: DNA + Aminosäure

pR5-4cort, cont.

790 810 830
. . . . . .
ACNAAGgACGAGCAAGAGGAAGAAGCAGCGaACCAGAAACTTGCCCTACAGaAAGCC
ThrLysAspGluGlnGluGluGluAlaAlaAsnGlnLysLeuAlaLeuGlnLysAla

850 870 890
. . . . . .
AAGGAGGTGGcAGAAGTGAGTCCTCTGTCCGCGGCCAACATGTCTATAGCTGTGAAAGAG
LysGluValAlaGluValSerProLeuSerAlaAlaAsnMetSerIleAlaValLysGlu

910
.
CAGAAGAAT
GlnLysAsn

### Sequenzprotokoll:

- Sequenz Nr.:: 27980/17
- Int. Code:: pRR14-35, 5' end
- Länge:: 1100 bp
- Typ:: DNA + Aminosäure

10 30 50
. . . . . .
GGGCGACACTGAGTGCCGGGAGTACTGGCCAGGACCCAACTTTGGCATCACCAACTTTGA[?]
GlyAspThrGluCysArgGluTyrTrpProGlyProAsnPheGlyIleThrAsnPheAs[?]

70 90 110
. . . . . .
AATATCCTGTTTGCCATCTTGACGGTGTTCCAGTGCATCACCATGGAGGGCTGGACTGA[?]
AsnIleLeuPheAlaIleLeuThrValPheGlnCysIleThrMetGluGlyTrpThrAs[?]

130 150 170
. . . . . .
ATCCTCTATAATACAAACGATGCGGCCGGCAACACCTGGAACTGGCTCTACTTCATCCC[?]
IleLeuTyrAsnThrAsnAspAlaAlaGlyAsnThrTrpAsnTrpLeuTyrPheIlePr[?]

190 210 230
. . . . . .
CTCATCATCATCGGCTCCTTCTTCATGCTCAACCTGGTGCTGGGCGTGCTCTCGGGGA[?]
LeuIleIleIleGlySerPhePheMetLeuAsnLeuValLeuGlyValLeuSerGlyGl[?]

250 270 290
. . . . . .
TTTGCCAAGGAGCGAGAGAGGGTGGAGAACCGCCGCGCCTTCCTGAAGCTGCGCCGGCA[?]
PheAlaLysGluArgGluArgValGluAsnArgArgAlaPheLeuLysLeuArgArgGl[?]

310 330 350
. . . . . .
CAGCAGATCGAGCGAGAGCTCAACGGGTACCTGGAGTGGATCTTCAAGGCGGAGGAAGT[?]
GlnGlnIleGluArgGluLeuAsnGlyTyrLeuGluTrpIlePheLysAlaGluGluVa[?]

### Sequenzprotokoll:

- Sequenz Nr.:: 27980/17
- Int. Code:: pRR14-35, 5' end
- Länge:: 1100 bp
- Typ:: DNA + Aminosäure

pRR14-35, cont.

370 390 410
ATGCTGGCCGAGGAGGACAGGAATGCAGAGGAGAAGTCCCCTTTGGACGTGCTGAAGAGA
MetLeuAlaGluGluAspArgAsnAlaGluGluLysSerProLeuAspValLeuLysArg

430 450 470
GCGGCCACCAAGAAGAGCAGAAATGACCTGATCCACGCAGAGGAGGGAGAGGACCGGTTT
AlaAlaThrLysLysSerArgAsnAspLeuIleHisAlaGluGluGlyGluAspArgPhe

490 510 530
GCAGATCTCTGTGCTGTTGGATCCCCCTTCGCCCGCGCCAGCCTcAAGAGCGGGAAGACA
AlaAspLeuCysAlaValGlySerProPheAlaArgAlaSerLeuLysSerGlyLysThr

550 570 590
GAGAGCTCGTCATACTTCCGGAGGAAGGAGAAGATGTTCCGGTTTTTATCCGGCGCATG
GluSerSerSerTyrPheArgArgLysGluLysMetPheArgPhePheIleArgArgMet

610 630 650
GTGAAGGCTCAGAgCTTCTACTGGGTGGTGCTGTGCGTGGTGGCCCTGAACACACTGTGT
ValLysAlaGlnSerPheTyrTrpValValLeuCysValValAlaLeuAsnThrLeuCys

670 690 710
GTGGCCATGGTGCATTACAACCAGCCGCGGCGGCTTACCACGACCCTGTATTTTGCAGAG
ValAlaMetValHisTyrAsnGlnProArgArgLeuThrThrThrLeuTyrPheAlaGlu

730 750 770
TTTGTTTTCCTGGGTCTCTTCCTCACAGAGATGTCCCTGAAGATGTATgGCCTGGGGCCC
PheValPheLeuGlyLeuPheLeuThrGluMetSerLeuLysMetTyrGlyLeuGlyPro

### Sequenzprotokoll:

- Sequenz Nr.:: 27980/17
- Int Code:: pRR14-35, 5' end
- Länge:: 1100 bp
- Typ:: DNA + Aminosäure

pRR14-35, cont.

790 810 830
AGAAGCTACTTCCGGTCCTCCTTCAACTGCTTCGACTTTGGGGTCATCGTGGGGAGCGTC
ArgSerTyrPheArgSerSerPheAsnCysPheAspPheGlyValIleValGlySerVal

850 870 890
TTTGAAGTGGTCTGGGCGGCCATCAAGCCGGgAAGCTCCTTTGGGATCAGTGTGCTGCgG
PheGluValValTrpAlaAlaIleLysProGlySerSerPheGlyIleSerValLeuArg

910 930 950
GCCCTCCGCCTGCTGAGGATCTTCAAAGTCACGAAGTACTGGAGCTCCCTGCGGAACCTG
AlaLeuArgLeuLeuArgIlePheLysValThrLysTyrTrpSerSerLeuArgAsnLeu

970 990 1010
GTGGTGTCCCTGCTGAACTCCATGAAGTCCATCATCAGCCTGCTCTTCTTNCTCTTCCTG
ValValSerLeuLeuAsnSerMetLysSerIleIleSerLeuLeuPheXxxLeuPheLeu

1030 1050 1070
TTCATTGTGGTCTTCNNNCTGCTGGGGATNCAGCTGTTTNNGGACAGTTCAACTTCCAG
PheIleValValPheXxxLeuLeuGlyXxxGlnLeuPheXxxGlyGlnPheAsnPheGln

1090
GATGAGACTCCCACAACCA
AspGluThrProThrThr

## Patentansprüche

1. Klonierte humane neuronale Calcium-Kanäle der Sequenzen Nr. 27980/1 bis 27980/17.

2. Verwendung der Calcium-Kanäle gemäß Anspruch 1 in einem Test für Ca-Antagonisten.

## Claims

1. Cloned human neuronal calcium channels of sequences Nos. 27980/1 to 27980/17.

2. Use of the calcium channels according to Claim 1 in a calcium antagonist test.

## Revendications

1. Canaux du calcium neuronaux humains clonés des séquences n° 27980/1 à 27980/17.

2. Utilisation des canaux du calcium selon la revendication 1 dans un essai pour des antagonistes du calcium.
